# EUROPEAN PATENT SPECIFICATION

(11) **EP 1 633 354 B1**
(45) Date of publication and mention of the grant of the patent: **23.01.2008**
(21) Application number: 04750170.5
(22) Date of filing: 14.04.2004
(51) Int. Cl.: A61K 31/426, A61K 31/415, A61K 31/4406, C07D 277/46, C07D 231/12, C07D 213/55, C07C 311/21, A61P 3/10

(54) **N-(((((1,3-THIAZOL-2-YL)AMINO)CARBONYL)PHENYL)SULFONYL)PHENYLALANINE DERIVATIVES AND RELATED COMPOUNDS FOR THE TREATMENT OF DIABETES**
N-(((((1,3-THIAZOL-2-YL)AMINO)CARBONYL)PHENYL)SULPHONYL)PHENYLALANIN DERIVATE UND VERWANDTE VERBINDUNGEN ZUR BEHANDLUNG VON DIABETES
ACIDES PHENYLALCANOIQUES SUBSTITUES

(30) Priority: 14.04.2003 US 463102 P
(43) Date of publication of application: 15.03.2006
(73) Proprietor: The Institutes for Pharmaceutical Discovery, LLC, Branford, CT 06405 (US)
(72) Inventor: VAN ZANDT, Michael, C., Guilford, CT 06437 (US); FANG, Haiquan, Madison, CT 06443 (US); HU, Shaojing, Hamden, CT 06514 (US); WHITEHOUSE, Darren, Westbrook, CT 06498 (US)
(74) Representative: Adam, Holger
(86) International application number: PCT/US2004/011650
(87) International publication number: WO 2004/092146

(56) References cited:
- EP-A- 0 950 656
- EP-A- 1 288 199
- WO-A-01/83461
- WO-A-01/83464
- WO-A-02/08188
- WO-A-02/28844
- WO-A-97/38986
- WO-A-99/32466
- WO-A-99/58514
- WO-A-02/064094
- WO-A-03/035610
- WO-A-03/048140
- WO-A-20/04010992
- WO-A-20/04020409
- DE-A- 10 150 172
- US-A1- 2002 077 347

### SEQUENCE LISTING

<110> THE INSTITUTES FOR PHARMACEUTICAL DISCOVERY, LLC
<120> N- (((((1,3-THIAZOL-2-YL) AMINO) CARBONYL) PHENYL) SULFONYL) PHENYLALANINE DERIVATIVES AND RELATED COMPOUNDS FOR THE TREATMENT OF DIABETES
<130> 15876EP
<140> 04 750 170.5
   <141> 2004-04-14
<150> US 60/463,102
   <151> 2003-04-14
<160> 1
<170> PatentIn Ver. 2.1
<210> 1
   <211> 12
   <212> PRT
   <213> Artificial Sequence
<220>
   <223> Description of Artificial Sequence:phosphotyrosyl dodecapeptide, corresponding to the 1142-1153 insulin receptor kinase regulatory domain, phosphorylated on the 1146, 1150 and 1151 tyrosine residues
<220>
   <221> MOD_RES
   <222> (5)
   <223> PHOSPHORYLATION
<220>
   <221> MOD_RES
   <222> (9)..(10)
   <223> PHOSPHORYLATION
<400> 1
   Thr Arg Asp Ile Tyr Glu Thr Asp Tyr Tyr Arg Lys
   1 5 10

### SEQUENCE LISTING

<110> THE INSTITUTES FOR PHARMACEUTICAL DISCOVERY, LLC
<120> N- (((((1,3-THIAZOL-2-YL) AMINO) CARBONYL) PHENYL) SULFONYL) PHENYLALANINE DERIVATIVES AND RELATED COMPOUNDS FOR THE TREATMENT OF DIABETES
<130> 15876EP
<140> 04 750 170.5
   <141> 2004-04-14
<150> US 60/463,102
   <151> 2003-04-14
<160> 1
<170> Patent In Ver. 2.1
<210> 1
   <211> 12
   <212> PRT
   <213> Artificial Sequence
<220>
   <223> Description of Artificial Sequence:phosphotyrosyl dodecapeptide, corresponding to the 1142-1153 insulin receptor kinase regulatory domain, phosphorylated on the 1146, 1150 and 1151 tyrosine residues
<220>
   <221> MOD_RES
   <222> (5)
   <223> PHOSPHORYLATION
<220>
   <221> MOD_RES
   <222> (9)..(10)
   <223> PHOSPHORYLATION
<400> 1
   Thr Arg Asp Ile Tyr Glu Thr Asp Tyr Tyr Arg Lys
   1 5 10

## Description

### BACKGROUND OF THE INVENTION

### Field of the Invention

The invention relates to substituted phenylalkanoic acids that are useful in the treatment of diabetes. More specifically, it relates to such compounds that are capable of inhibiting Protein tyrosine phosphatase-1B (PTP-1B), which is a negative regulator of the insulin signaling pathway, and improves insulin-sensitivity.

### Description of the Related Art

Protein tyrosine phosphatases are a large family of transmembrane or intracellular enzymes that dephosphorylate substrates involved in a variety of regulatory processes (Fischer et al., 1991, Science 253:401-406). Protein tyrosine phosphatase-1B (PTP-1B) is an approximately 50 kd intracellular protein, which is present in abundant amounts in various human tissues (Charbonneau et al., 1989, Proc. Natl. Acad. Sci. USA 86:5252-5256; Goldstein, 1993, Receptor 3:1-15).

Determining which proteins are substrates of PTP-1B has been of considerable interest. One substrate which has aroused especial interest is the insulin receptor. The binding of insulin to its receptor results in autophosphorylation of the domain. This causes activation of the insulin receptor tyrosine kinase, which phosphorylates the various insulin receptor substrate (IRS) proteins that propagate the insulin signaling event further downstream to mediate insulin's various biological effects.

Seely et al., 1996, Diabetes 45:1379-1385 ("Seely") studied the relationship of PTP-1B and the insulin receptor in vitro. Seely constructed a glutathione S-transferase (GST) fusion protein of PTP-1B that had a point mutation in the PTP-1B catalytic domain. Although catalytically inactive, this fusion protein was able to bind to the insulin receptor, as demonstrated by its ability to precipitate the insulin receptor from purified receptor preparations and from whole cell lysates derived from cells expressing the insulin receptor.

Ahmad et al., 1995, J. Biol. Chem. 270:20503-20508 used osmotic loading to introduce PTP-1B neutralizing antibodies into rat KRC-7 hepatoma cells. The presence of the antibody in the cells resulted in an increase of 42% and 38%, respectively, in insulin stimulated DNA synthesis and phosphatidyinositol 3' kinase activity. Insulin receptor autophosphorylation and insulin receptor substrate-1 tyrosine phosphorylation were increased 2.2 and 2.0-fold, respectively, in the antibody-loaded cells. The antibody-loaded cells also showed a 57% increase in insulin stimulated insulin receptor kinase activity toward exogenous peptide substrates.

Kennedy et al., 1999, Science 283: 1544-1548 showed that protein tyrosine phosphatase PTP-1B is a negative regulator of the insulin signaling pathway, indicating that inhibitors of this enzyme are beneficial in the treatment of Type 2 diabetes, which appears to involve a defect in an early process in insulin signal transduction rather than a structural defect in the insulin receptor itself. (J. M. Olefsky, W. T. Garvey, R. R. Henry, D. Brillon, S. Matthai and G. R. Freidenberg, G. R. (1988).) Cellular mechanisms of insulin resistance in non-insulin-dependent (Type II) diabetes. (Am. J. Med. 85: Suppl. 5A, 86-105.) A drug that improved insulin sensitivity would have several advantages over traditional therapy of NIDDM using sulfonylureas, which do not alleviate insulin resistance but instead compensate by increasing insulin secretion.

Therefore, inhibitors of PTP-1B are useful in controlling or treating Type 2 diabetes, in improving glucose tolerance, and in improving insulin sensitivity in patients in need thereof. The compounds are also useful in treating or controlling other PTP-1B mediated diseases, such as the treatment of cancer, neurodegenerative diseases and the like.

US Patent Application 2002/077347 A1 relates to PTP-1B inhibitors having the formula 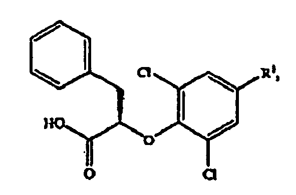 or therapeutically acceptable salts thereof, wherein R¹ is selected from the group consisting of optionally substituted benzodioxolyl, dibenzofuranyl, indolyl, phenyl and thianthrenyl as well as to the preparation of these compounds, compositions containing the compounds and treatment of diseases using the compounds.

International Patent Application WO 02/08188A is concerned with N-substituted indoles having aryloxyacetic acid substituents of the general formula 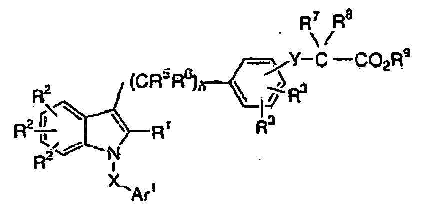 and pharmaceutically acceptable salts and prodrugs thereof, which are useful as therapeutic compounds in the treatment of Type II diabetes mellitus of conditions that are frequently associated with this disease and of lipid disorders.

Similarly, International Patent Application WO 02/064094A relates to 2-aryloxy-2-aryl alcanoic acids as potent agonists of PPAR α and/or γ and to their use in the treatment, control or prevention of inter alia non-insulin dependent diabetes mellitus.

European Patent Application EP 1 288 199 A1, International Patent Applications WO 02/28844A, WO 01/83461A and WO 01/83464A relate to MMP-inhibitors of the general formula 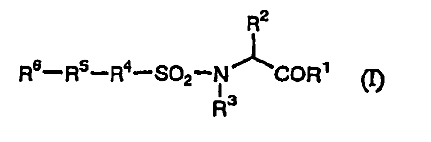 optically active substances thereof, prodrugs, pharmaceutically acceptable salts or solvates thereof as well as to their use for the treatment of osteoarthritis, rheumatoid arthritis, corneal ulceration, periodontitis, metastasis, invasion of tumor, virus infection, pulmonary emphysema, chronic obstructive pulmonary disease, respiratory obstruction and chronic bronchitis. Similarly, European Patent Application EP 0950656A1 relates to MMP-inhibitors having the similar formula 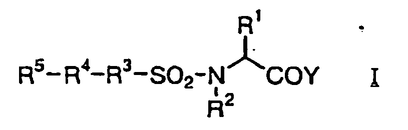 and their possible use for the treatment of such diseases.

International Patent Application WO 99/32466A relates to substituted benzenesulfonamide derivatives of the formula 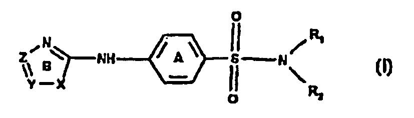 having Ring B as an unsubstituted heteroaromatic substituent and to salts thereof as well as to their pharmaceutical use for the treatment of diseases such as obesity, bulimia nervosa, diabetes, dyslipidaemia and hypertonia.

Furthermore, International Patent Application WO 97/38986A pertains to a class of substituted sulfonamide compounds having the general formula 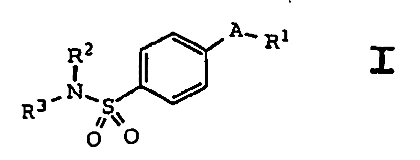 and to their use for the treatment of inflammation and other cyclooxygenase-2 mediated disorders.

German Patent Application DE 10 150 172 A1 discloses PTP-1B inhibitor compounds of the general formula 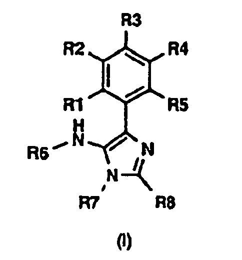 as well as to prodrugs, pharmaceutically acceptable salts thereof and to pharmaceutical preparations comprising these compounds as active ingredient as well as to their use in the prevention and treatment of diseases involving PTP-1B such as diabetes or obesity. This application does not disclose sulfonamide compounds.

Similarly, International Patent Application WO 03/48140A discloses PTP-1B inhibiting compounds of the general formula 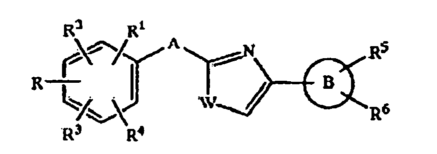 as well as their use for the treatment of Type I or II diabetes by inhibiting PTP-1B.

Furthermore, International Patent Application WO 04/20409A relates to indole compounds having aryloxylalcanoic acid substituents of the formula 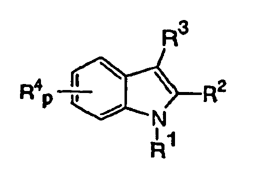 which exhibit a PPAR-γ agonistic activity and are, thus, useful in the treatment and control of diseases such as Type II diabetes, dyslipidemia, hyperlipidemia, hypercholesterolemia, hypertriglyceridemia and obesity.

International Patent Application WO 03/35610A relates to MMP inhibiting sulfonamide derivatives having the formula

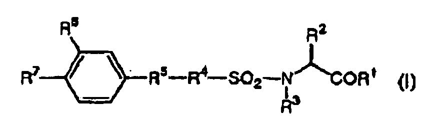

International Patent Application WO 04/10992A relates to a class of chromane and chromene compounds having the formula 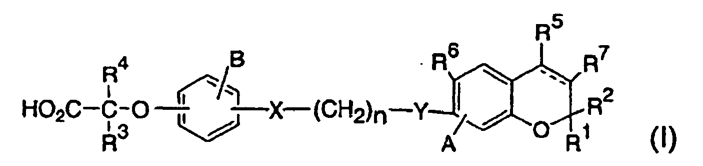 and pharmaceutically acceptable salts thereof and their use as therapeutic compounds in the treatment and control of hyperlipidema, hypercholesterolemia, dyslipidaemia and other lipid disorders.

### SUMMARY OF THE INVENTION

In a broad aspect, the invention encompasses the compounds of formula (I) shown below, pharmaceutical compositions containing the compounds and methods employing such compounds or compositions in the treatment of diabetes.

In one aspect, the invention encompasses compounds formula: 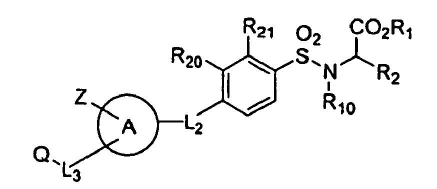 wherein,
- L₂: is a bond or -C(O)NR₉-, -N(R₉)C(O)-, -(C₁-C₄)alkyl-C(O)NR₉-,-(C₁-C₄)alkyl-N(R₉)C(O)-, -C(O)N(R₉)-(C₁-C₄)alkyl-, -N(R₉)C(O) -(C₁-C₉)alkyl-,-N(R₉)SO₂-, -SO₂N(R₉)-, -N(R9)-, -N(R₉)-(C₁-C₄)alkyl-, -O-(C₁-C₄)alkyl-, -(C₁-C₉)alkyl-O-, or -(C₁-C₄)alkyl-N(R₉)-,
R₉ is H, C₁-C₆ alkyl, -SO₂phenyl, phenyl(C₁-C₄)alkyl, wherein the phenyl group is optionally substituted with 1, 2, 3, or 4 groups that are independently C₁-C₄ alkyl, C₁-C₄ alkoxy, halogen, OH, NO₂, NH₂, NH(C₁-C₆)alkyl, N(C₁-C₆)alkyl(C₁-C₆)alkyl, C₁-C₂ haloalkyl, or C₁-C₂ haloalkoxy;
- L₃: is a bond, -(C₁-C₄)alkyl-O-, -O-(C₁-C₄)alkyl, -(C₁-C₄)alkyl-, -C(O)-;
- the A: ring is thiazolyl, pyrazolyl, dihydropyrazolyl, benzofuranyl, imidazolyl, isothiazolyl, pyrrolyl, oxazolyl, pyrimidyl, or triazolyl, each of which is optionally substituted with 1, or 2 groups that are independently, halogen, C₁-C₆ alkyl, C₁-C₄ alkoxy, haloalkyl, haloalkoxy, NO₂, NH₂, NH(C₁-C₆)alkyl, N(C₁-C₆)alkyl(C₁-C₆)alkyl;
- Q is: phenyl, naphthyl, -phenyl-carbonyl-phenyl, -phenyl-(C₁-C₄)alkyl- phenyl, -phenyl-pyridyl, -phenyl-pyrimidyl, -phenyl-pyrrolyl, -phenyl-piperidinyl, -phenyl-pyrrolidinyl, -phenyl-piperazinyl, -phenyl-, pyridyl, pyrimidyl, furanyl, thienyl, pyrrolyl, imidazolyl,-pyridyl-(C₁-C₄)alkyl-phenyl, imidazolidinyl, dibenzofuranyl, tetrahydrofuranyl, tetrahydrothienyl, piperidinyl, pyrrolidinyl, piperazinyl, C₁-C₆ alkyl, halogen, C₁-C₄ haloalkoxy, C₁-C₄ haloalkyl, or C₁-C₆ alkoxycarbonyl, wherein the aforementioned cyclic groups are optionally substituted with 1, 2, 3, 4, or 5 groups that are independently alkoxycarbonyl, C₁-C₆ alkyl, C₁-C₆ alkoxy, halogen, C₁-C₄ haloalkyl, C₁-C₄ haloalkoxy, NR₆R₇, or phenyl; wherein
R₆ and R₇ are independently H, C₁-C₆ alkyl, phenyl(C₁-C₆)alkyl, C₂-C₆ alkanoyl, phenyl(C₁-C₆)alkanoyl, C₁-C₆ alkoxycarbonyl, phenyl(C₁-C₆)alkoxycarbonyl, pyridylcarbonyl, or -SO₂-phenyl, wherein the cyclic groups are optionally substituted with 1, 2, 3, or 4 groups that are independently halogen, C₁-C₄ alkyl, C₁-C₄ alkoxy, NO₂, OH, NH₂, NH (C₁-C₆) alkyl, N(C₁-C₆)alkyl(C₁-C₆)alkyl, C₁-C₂ haloalkyl or C₁-C₂ haloalkoxy, and
- Z is: -NHC(O)phenyl, -NHC(O)naphthyl, -N(C₁-C₄ alkyl)C(O)phenyl,-N(C₁-C₄ alkyl)C(O)naphthyl, naphthyl, or phenyl, wherein the phenyl groups are optionally substituted with 1, 2, 3, 4, or 5 groups that are independently C₁-C₆ alkyl, C₁-C₆ alkoxy, halogen, C₁-C₂ haloalkyl, C₁-C₂ haloalkoxy, or NO₂, or
- Z is: -NHC(O)-(C₁-C₄)alkyl-(C₃-C₇)cycloalkyl, or -N(C₁-C₄)alkylC(O)- (C₁-C₄)alkyl- (C₃-C₇) cycloalkyl;
- R₁ is: H, C₁-C₄ alkyl, or benzyl;
- R₂ is: phenyl, phenyl(C₁-C₄) alkyl, -(C₁-C₄) alkyl-piperidinyl,-(C₁-C₄) alkyl-pyrrolidinyl, wherein the heterocycloalkyl group is optionally fused to a phenyl ring and wherein the heterocycloalkyl portion, the phenyl portion, or both are optionally substituted with a total of 1, 2, 3, or 4 groups that are independently halogen, C₁-C₄ alkyl, C₁-C₄ alkoxy, -SO₂-(C₁-C₄) alkyl, C₁-C₂ haloalkyl, or C₁-C₂ haloalkoxy;
- R₁₀ is: H, C₁-C₆ alkyl, wherein the alkyl group is optionally substituted with phenyl, which is optionally substituted with 1, 2, 3, or 4 groups that are independently C₁-C₆ alkyl, C₁-C₆ alkoxy, halogen, OH, NO₂, C₁-C₂ haloalkyl, or C₁-C₂ haloalkoxy; and
- R₂₀, and R₂₁,: are independently selected from H, benzyloxy, benzyl, halogen, C₁-C₄ alkyl, OH, C₁-C₄ alkoxy, NO₂, NH₂, NH(C₁-C₆)alkyl, N(C₁-C₆)alkyl(C₁-C₆)alkyl, NH-phenyl, N(C₁-C₄)alkyl-phenyl, NHbenzyl, or -N(C₁-C₆)alkylbenzyl, wherein the phenyl groups are optionally substituted with 1, 2, 3, or 4 groups that are independently C₁-C₆ alkyl, C₁-C₆ alkoxy, halogen, OH, NO₂, C₁-C₂ haloalkyl, or C₁-C₂ haloalkoxy.

Compounds according to the present invention bind to PTP-1B.

Preferably that interaction results in inhibition of the enzyme.

The invention also provides pharmaceutical compositions comprising a compound or salt of formula II and at least one pharmaceutically acceptable carrier, solvent, adjuvant or diluent.

The invention further provides the use of a compound according to the present invention in the preparation of a medicament for the treatment of diabetes.

The compounds according to the invention may also be used for methods and compositions for combination therapy of Type I and Type II diabetes. In these embodiments, the compounds according to the invention may be used in formulations and pharmaceutical compositions, as well as methods for treating Type I and Type II diabetes with the PTPase inhibitors of formula I plus additional compounds and medicaments as disclosed in more detail below. These methods can comprise treatment methods for Type I and Type II diabetes where the PTPase inhibitors of formula I are formulated with a therapeutically-effective amount of said additional compounds and medicaments. Alternatively, the treatment methods for Type I and Type II diabetes comprise administration PTPase inhibitors of formula I as disclosed herein concomitantly, simultaneously or together with a therapeutically-effective amount of said additional compounds and medicaments.

### DETAILED DESCRIPTION OF THE INVENTION

The present invention is directed to compounds of formula II: 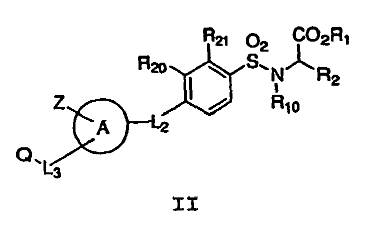 wherein,
- L₂ is: a bond or -C(O)NR₉-, -N(R₉)C(O)-, -(C₁-C₄)alkyl-C(O)NR₉-,-(C₁-C₄)alkyl-N(R₉)C(O)-, -C(O)N(R₉)-(C₁-C₄)alkyl-, -N(R₉)C(O) -(C₁-C₄)alkyl-, -N(R₉)SO₂-, -SO₂N(R₉)-, -N(R9)-, -N(R₉)-(C₁-C₄)alkyl-, -O-(C₁-C₄)alkyl-, -(C₁-C₄)alkyl-O-, or -(C₁-C₄)alkyl-N(R₉)-,
R₉ is H, C₁-C₆ alkyl, -SO₂phenyl, phenyl(C₁-C₄)alkyl, wherein the phenyl group is optionally substituted with 1, 2, 3, or 4 groups that are independently C₁-C₄ alkyl, C₁-C₄ alkoxy, halogen, OH, NO₂, NH₂, NH(C₁-C₆)alkyl, N(C₁-C₆)alkyl(C₁-C₆)alkyl, C₁-C₂ haloalkyl, or C₁-C₂ haloalkoxy;
- L₃ is: a bond, -(C₁-C₄)alkyl-O-, -O-(C₁-C₄)alkyl, - (C₁-C₄)alkyl-, -C(O)-;
- the A: ring is thiazolyl, pyrazolyl, dihydropyrazolyl, benzofuranyl, imidazolyl, isothiazolyl, pyrrolyl, oxazolyl, pyrimidyl, or triazolyl, each of which is optionally substituted with 1, or 2 groups that are independently, halogen, C₁-C₆ alkyl, C₁-C₄ alkoxy, haloalkyl, haloalkoxy, NO₂, NH₂, NH(C₁-C₆)alkyl, N(C₁-C₆)alkyl(C₁-C₆)alkyl;
- Q is: phenyl, naphthyl, -phenyl-carbonyl-phenyl, -phenyl -(C₁-C₄)alkyl- phenyl, -phenyl-pyridyl, -phenyl-pyrimidyl, -phenyl-pyrrolyl, -phenyl-piperidinyl, -phenyl-pyrrolidinyl, -phenyl-piperazinyl, -phenyl-, pyridyl, pyrimidyl, furanyl, thienyl, pyrrolyl, imidazolyl,-pyridyl-(C₁-C₄)alkyl-phenyl, imidazolidinyl, dibenzofuranyl, tetrahydrofuranyl, tetrahydrothienyl, piperidinyl, pyrrolidinyl, piperazinyl, C₁-C₆ alkyl, halogen, C₁-C₄ haloalkoxy, C₁-C₄ haloalkyl, or C₁-C₆ alkoxycarbonyl, wherein the aforementioned cyclic groups are optionally substituted with 1, 2, 3, 4, or 5 groups that are independently alkoxycarbonyl, C₁-C₆ alkyl, C₁-C₆ alkoxy, halogen, C₁-C₄ haloalkyl, C₁-C₄ haloalkoxy, NR₆R₇, or phenyl; wherein
R₆ and R₇ are independently H, C₁-C₆ alkyl, phenyl(C₁-C₆)alkyl, C₂-C₆ alkanoyl, phenyl(C₁-C₆)alkanoyl, C₁-C₆ alkoxycarbonyl, phenyl(C₁-C₆)alkoxycarbonyl, pyridylcarbonyl, or -SO₂-phenyl, wherein the cyclic groups are optionally substituted with 1, 2, 3, or 4 groups that are independently halogen, C₁-C₄ alkyl, C₁-C₄ alkoxy, NO₂, OH, NH₂, NH(C₁-C₆)alkyl, N(C₁-C₆)alkyl(C₁-C₆)alkyl, C₁-C₂ haloalkyl or C₁-C₂ haloalkoxy, and
- Z is: -NHC(O)phenyl, -NHC(O)naphthyl, -N(C₁-C₄ alkyl)C(O)phenyl,-N(C₁-C₄ alkyl)C(O)naphthyl, naphthyl, or phenyl, wherein the phenyl groups are optionally substituted with 1, 2, 3, 4, or 5 groups that are independently C₁-C₆ alkyl, C₁-C₆ alkoxy, halogen, C₁-C₂ haloalkyl, C₁-C₂ haloalkoxy, or NO₂, or
- Z is: -NHC(O)-(C₁-C₄)alkyl-(C₃-C₇)cycloalkyl, or -N(C₁-C₄)alkylC(O)-(C₁-C₄)alkyl-(C₃-C₇)cycloalkyl;
- R₁ is: H, C₁-C₄ alkyl, or benzyl;
- R₂ is: phenyl, phenyl(C₁-C₄)alkyl, -(C₁-C₄) alkyl-piperidinyl,-(C₁-C₄) alkyl-pyrrolidinyl, wherein the heterocycloalkyl group is optionally fused to a phenyl ring and wherein the heterocycloalkyl portion, the phenyl portion, or both are optionally substituted with a total of 1, 2, 3, or 4 groups that are independently halogen, C₁-C₄ alkyl, C₁-C₄ alkoxy, -SO₂-(C₁-C₄) alkyl, C₁-C₂ haloalkyl, or C₁-C₂ haloalkoxy;
- R₁₀ is: H, C₁-C₆ alkyl, wherein the alkyl group is optionally substituted with phenyl, which is optionally substituted with 1, 2, 3, or 4 groups that are independently C₁-C₆ alkyl, C₁-C₆ alkoxy, halogen, OH, NO₂, C₁-C₂ haloalkyl, or C₁-C₂ haloalkoxy; and
- R₂₀, and R₂₁,: are independently selected from H, benzyloxy, benzyl, halogen, C₁-C₄ alkyl, OH, C₁-C₄ alkoxy, NO₂, NH₂, NH (C₁-C₆)alkyl, N(C₁-C₆)alkyl(C₁-C₆)alkyl, NH-phenyl, N(C₁-C₄)alkyl-phenyl, NHbenzyl, or -N(C₁-C₆)alkylbenzyl, wherein the phenyl groups are optionally substituted with 1, 2, 3, or 4 groups that are independently C₁-C₆ alkyl, C₁-C₆ alkoxy, halogen, OH, NO₂, C₁-C₂ haloalkyl, or C₁-C₂ haloalkoxy.

Preferred compounds of formula II include compounds of formula II-1, i.e., compounds wherein
- L₂ is: a bond or -C(O)NR₉-, -N(R₉)C(O)-, -(C₁-C₄)alkyl-C(O)NR₉-, -(C₁-C₄)alkyl-N(R₉)C(O)-, -N(R₉)SO₂-, -SO₂N(R₉)-,-N(R₉)-, -N(R₉)-(C₁-C₄)alkyl-, or -(C₁-C₄)alkyl-N(R₉)-,
- R₉ is: H, C₁-C₆ alkyl, -SO₂phenyl, benzyl, phenethyl, naphthyl-CH₂-, anthracenyl-CH₂-, wherein the phenyl group is optionally substituted with 1, 2, 3, or 4 groups that are independently C₁-C₄ alkyl, C₁-C₉ alkoxy, halogen, OH, NO₂, NH₂, NH(C₁-C₆)alkyl, N(C₁-C₆)alkyl(C₁-C₆)alkyl, C₁-C₂ haloalkyl, or C₁-C₂ haloalkoxy;
- the A: ring is thiazolyl, pyrazolyl, dihydropyrazolyl, benzofuranyl, imidazolyl, isothiazolyl, pyrrolyl, pyrimidyl, or oxazolyl, each of which is optionally substituted with 1, or 2 groups that are independently, halogen, C₁-C₆ alkyl, C₁-C₄ alkoxy, haloalkyl, haloalkoxy, NO₂, NH₂, NH(C₁-C₆)alkyl, N(C₁-C₆)alkyl(C₁-C₆)alkyl; and
- Q is: phenyl, naphthyl, -phenyl-carbonyl-phenyl, -phenyl-pyridyl, -phenyl-piperidinyl, -phenyl-pyrrolidinyl, pyridyl, pyrimidyl, furanyl, thienyl, piperidinyl, dibenzofuranyl, pyrrolidinyl, piperazinyl, C₁-C₆ alkyl, halogen, C₁-C₄ haloalkoxy, C₁-C₄ haloalkyl, or C₁-C₆ alkoxycarbonyl, wherein the aforementioned cyclic groups are optionally substituted with 1, 2, 3, 4, or 5 groups that are independently alkoxycarbonyl, C₁-C₆ alkyl, C₁-C₆ alkoxy, halogen, C₁-C₄ haloalkyl, C₁-C₄ haloalkoxy, or NR₆R₇; wherein
R₆ and R₇ are independently H, C₁-C₆ alkyl, phenyl(C₁-C₄)alkyl, C₂-C₆ alkanoyl, phenyl(C₁-C₄)alkanoyl, C₁-C₆ alkoxycarbonyl, phenyl(C₁-C₉)alkoxycarbonyl, pyridylcarbonyl, or -SO₂-phenyl, wherein the cyclic groups are optionally substituted with 1, 2, 3, or 4 groups that are independently halogen, C₁-C₄ alkyl, C₁-C₄ alkoxy, NO₂, OH, NH₂, NH(C₁-C₆)alkyl, N(C₁-C₆)alkyl(C₁-C₆)alkyl, CF₃, or OCF₃.

Other compounds of formula II-1 include compounds of formula II-2, i.e., compounds wherein
- R₂ is: phenyl or phenyl(C₁-C₄) alkyl wherein the phenyl portion, or both are optionally substituted with a total of 1, 2, 3, or 4 groups that are independently halogen, C₁-C₄ alkyl, C₁-C₄ alkoxy, -SO₂-(C₁-C₄) alkyl, CF₃, or OCF₃;
- R₁₀ is: H, C₁-C₄ alkyl, wherein the alkyl group is optionally substituted with phenyl, which is optionally substituted with 1, 2, 3, or 4 groups that are independently C₁-C₆ alkyl, C₁-C₆ alkoxy, halogen, OH, NO₂, C₁-C₂ haloalkyl, or C₁-C₂ haloalkoxy; and
- R₂₀, and R₂₁,: are independently selected from H, halogen, C₁-C₄ alkyl, OH, C₁-C₄ alkoxy, NO₂, NH₂, NH(C₁-C₆)alkyl, or N(C₁-C₆)alkyl(C₁-C₆)alkyl,
- L₂ is: a bond or -C(O)NR₉-, -N(R₉)C(O)-, -(C₁-C₄)alkyl-C(O)NR₉-,-(C₁-C₄)alkyl-N(R₉)C(O)-, -N(R₉)SO₂-, -SO₂N(R₉)-, -N(R₉)-, -N(R₉)-(C₁-C₄) alkyl-, or -(C₁-C₄)alkyl-N(R₉)-,
R₉ is H, C₁-C₆ alkyl, -SO₂phenyl, benzyl, phenethyl, wherein the phenyl group is optionally substituted with 1, 2, 3, or 4 groups that are independently C₁-C₄ alkyl, C₁-C₄ alkoxy, halogen, OH, NO₂, NH₂, NH(C₁-C₆)alkyl, N(C₁-C₆)alkyl(C₁-C₆)alkyl, CF₃, or OCF₃;
- Q is: phenyl, naphthyl, pyridyl, pyrimidyl, furanyl, thienyl, piperidinyl, pyrrolidinyl, piperazinyl, C₁-C₆ alkyl, halogen, C₁-C₂ haloalkoxy, C₁-C₂ haloalkyl, or C₁-C₆ alkoxycarbonyl, wherein the aforementioned cyclic groups are optionally substituted with 1, 2, 3, 4, or 5 groups that are independently alkoxycarbonyl, C₁-C₆ alkyl, C₁-C₆ alkoxy, halogen, C₁-C₄ haloalkyl, C₁-C₄ haloalkoxy, or NR₆R₇; wherein
R₆ and R₇ are independently H, C₁-C₆ alkyl, phenyl(C₁-C₄)alkyl, C₂-C₆ alkanoyl, phenyl(C₁-C₄)alkanoyl, wherein the phenyl groups are optionally substituted with 1, 2, 3, or 4 groups that are independently halogen, C₁-C₄ alkyl, C₁-C₄ alkoxy, NO₂, OH, NH₂, NH(C₁-C₆)alkyl, N(C₁-C₆)alkyl(C₁-C₆)alkyl, CF₃, or OCF₃, and
- Z is: -NHC(O)phenyl, -N(C₁-C₄ alkyl)C(O)phenyl, or phenyl, wherein the phenyl groups are optionally substituted with 1, 2, 3, 4, or 5 groups that are independently C₁-C₆ alkyl, C₁-C₆ alkoxy, halogen, C₁-C₂ haloalkyl, C₁-C₂ haloalkoxy, or NO₂, or
- Z is: -NHC(O)-(C₁-C₄)alkyl-(C₃-C₇) cycloalkyl, or -N(C₁-C₄)alkylC(O)-(C₁-C₄)alkyl-(C₃-C₇)cycloalkyl.

Preferred compounds of formula II-2 include compounds of formula II-3, i.e., compounds wherein
- R₁ is: H, or C₁-C₄ alkyl;
- R₂ is: phenyl, phenyl(C₁-C₄) alkyl, wherein the phenyl portion, or both are optionally substituted with a total of 1, 2, 3, or 4 groups that are independently halogen, C₁-C₄ alkyl, C₁-C₄ alkoxy, or -SO₂-(C₁-C₄) alkyl;
- R₁₀ is: H, C₁-C₄ alkyl, wherein the alkyl group is optionally substituted with phenyl, which is optionally substituted with 1, 2, 3, or 4 groups that are independently C₁-C₆ alkyl, C₁-C₆ alkoxy, halogen, OH, NO₂, CF₃, or OCF₃; and
- at: least one of R₂₀ and R₂₁, is H, while the other is H, halogen, C₁-C₄ alkyl, OH, C₁-C₄ alkoxy, NO₂, NH₂, NH(C₁-C₆)alkyl, or N(C₁-C₆)alkyl(C₁-C₆)alkyl;
- L₂ is: a bond or -C(O)NR₉-, -N(R₉)C(O)-, -N(R₉)SO₂-, -SO₂N(R₉)-,-N(R₉)-, -N(R₉)-(C₁-C₄)alkyl-, or -(C₁-C₄)alkyl-N(R₉)-; and
- Q is: phenyl, naphthyl, pyridyl, pyrimidyl, furanyl, thienyl, piperidinyl, pyrrolidinyl, or piperazinyl each of which is optionally substituted with 1, 2, 3, 4, or 5 groups that are independently alkoxycarbonyl, C₁-C₆ alkyl, C₁-C₆ alkoxy, halogen, CF₃, OCF₃, or NR₆R₇.

Preferred compounds of formula II-3 include compounds of formula II-4, i.e., compounds wherein
- L₂ is: a bond or -NR₉-; wherein R₉ is H, C₁-C₆ alkyl, or benzyl;
- R₂ is: phenyl, benzyl, or phenethyl, wherein the phenyl portion is optionally substituted with a total of 1, 2, 3, or 4 groups that are independently halogen, C₁-C₄ alkyl, C₁-C₄ alkoxy, or -SO₂-(C₁-C₄) alkyl;
- Q is: phenyl, or pyridyl, each of which is optionally substituted with 1, 2, 3, 4, or 5 groups that are independently C₁-C₆ alkoxycarbonyl, C₁-C₆ alkyl, C₁-C₆ alkoxy, halogen, CF₃, OCF₃, or NR₆R₇; and
- Z is: phenyl, which is optionally substituted with 1, 2, 3, 4, or 5 groups that are independently C₁-C₆ alkyl, C₁-C₆ alkoxy, halogen, C₁-C₂ haloalkyl, C₁-C₂ haloalkoxy, or NO₂.

Preferred compounds of formula II-4 include compounds of formula II-5, i.e., compounds wherein the A ring is pyrazolyl, dihydropyrazolyl, thiazolyl, or pyrimidyl each of which is optionally substituted with 1, or 2 groups that are independently, halogen, C₁-C₆ alkyl, C₁-C₄ alkoxy, haloalkyl, haloalkoxy, NO₂, NH₂, NH(C₁-C₆)alkyl, N(C₁-C₆)alkyl(C₁-C₆)alkyl. In a preferred embodiment, the A ring is unsubstituted or substituted with at least one halogen.

Preferred compounds of formula II-5 include compounds of formula II-6, i.e., compounds wherein R₁₀ is H or C₁-C₄ alkyl; and L₃ is a bond or -(C₁-C₄) alkyl-. More preferably, R₁₀ is H or methyl.

In another aspect, the invention provides compounds of formula II-6-a, i.e., compounds of formula II-5 or II-6 wherein the A ring is pyrazolyl, dihydropyrazolyl, thiazolyl, or pyrimidyl each of which is unsubstituted.

In yet another aspect, the invention provides compounds of formula II-6-b, i.e., compounds of formula II-5. II-6, or II-6-a wherein R₁ is H.

In still another aspect, the invention provides compounds of formula II-6-c, i.e., compounds of formula II-5, II-6, II-6-a, or II-6-b wherein L₃ is a bond, and L₂ is a bond.

In yet another aspect, the invention provides compounds of formula II-6-d, i.e., compounds of formula II-6-c or II-6-b wherein the A ring is pyrazolyl or thiazolyl.

In another aspect, the invention provides compounds of formula II-6-f, i.e., compounds of formula II-4, II-5, II-6, II-6-a, II-6-b, II-6-c or II-6-d, wherein Z is phenyl, which is optionally substituted with 1, 2, or 3 groups that are independently C₁-C₆ alkyl (in another aspect, C₁-C₄ alkyl), C₁-C₆ alkoxy (in another aspect, C₁-C₄ alkoxy), halogen, C₁-C₂ haloalkyl (in one aspect, CF₃), C₁-C₂ haloalkoxy (in one aspect, OCF₃), or NO₂.

In yet another aspect, the phenyl is monosubstituted. In still another aspect, the phenyl ring is unsubstituted.

In still another aspect, the invention provides compounds of formula II-6-h i.e., compounds of formula II-4, II-5, II-6, II-6-a, II-6-b, II-6-c or II-6-d, II-6-e, II-6-f, or II-6-g, wherein Q is phenyl, which is optionally substituted with 1, 2, or 3, groups that are independently C₁-C₆ alkoxycarbonyl (in another aspect, C₁-C₄ alkoxycarbonyl), C₁-C₆ alkyl (in another aspect, C₁-C₄ alkyl), C₁-C₆ alkoxy (in another aspect, C₁-C₄ alkoxy), halogen, CF₃, or OCF₃.

In still another aspect, the invention provides compounds of formula II-6-i i.e., compounds of formula II-4, II-5, II-6, II-6-a, II-6-b, II-6-c or II-6-d, II-6-e, II-6-f, or II-6-g wherein Q is phenyl, which is optionally substituted with 1, 2, or 3, groups that are independently C₁-C₆ alkoxycarbonyl (in another aspect, C₁-C₄ alkoxycarbonyl), C₁-C₆ alkyl (in another aspect, C₁-C₄ alkyl), C₁-C₆ alkoxy (in another aspect, C₁-C₄ alkoxy), halogen, CF₃, OCF₃ or NR₆R₇; wherein
- R₆ and R₇: are independently H, C₁-C₆ alkyl (in another aspect, C₁-C₄ alkyl), phenyl(C₁-C₂)alkyl, C₂-C₆ alkanoyl, or phenyl(C₁-C₂)alkanoyl, wherein the phenyl groups are optionally substituted with 1, 2, or 3 groups that are independently halogen, C₁-C₄ alkyl, C₁-C₄ alkoxy, NO₂, OH, NH₂, NH(C₁-C₆)alkyl, N(C₁-C₆)alkyl(C₁-C₆)alkyl, CF₃, or OCF₃.

In yet another aspect, the invention provides compounds of formula II-6-j, i.e., compounds of formula II-4, II-5, II-6, II-6-a, II-6-b, II-6-c or II-6-d, II-6-e, or II-6-f, wherein Q is pyridyl, which is optionally substituted with 1, 2, 3, 4, or 5 groups that are independently C₁-C₆ alkoxycarbonyl, C₁-C₆ alkyl, C₁-C₆ alkoxy, halogen, CF₃, OCF₃, or NR₆R₇; wherein
- R₆ and R₇: are independently H, C₁-C₆ alkyl, phenyl (C₁-C₄) alkyl, C₂-C₆ alkanoyl, or phenyl(C₁-C₄)alkanoyl, wherein the phenyl groups are optionally substituted with 1, 2, 3, or 4 groups that are independently halogen, C₁-C₄ alkyl, C₁-C₄ alkoxy, NO₂, OH, NH₂, NH(C₁-C₆)alkyl, N(C₁-C₆)alkyl(C₁-C₆)alkyl, CF₃, or OCF₃.

In still another aspect, the invention provides compounds of formula II-6-k i.e., compounds of formula II-4, II-5, II-6, II-6-a, II-6-b, II-6-c or II-6-d, II-6-e, II-6-f, or II-6-j, wherein Q is pyridyl, which is optionally substituted with 1, 2, or 3, groups that are independently C₁-C₆ alkoxycarbonyl (in another aspect, C₁-C₄ alkoxycarbonyl), C₁-C₆ alkyl (in another aspect, C₁-C₄ alkyl), C₁-C₆ alkoxy (in another aspect, C₁-C₄ alkoxy), halogen, CF₃, or OCF₃.

In still another aspect, the invention provides compounds of formula II-6-1 i.e., compounds of formula II-4, II-5, II-6, II-6-a, II-6-b, II-6-c or II-6-d, II-6-e, II-6-f, or II-6-j wherein Q is pyridyl, which is optionally substituted with 1, 2, or 3, groups that are independently C₁-C₆ alkoxycarbonyl (in another aspect, C₁-C₄ alkoxycarbonyl), C₁-C₆ alkyl (in another aspect, C₁-C₄ alkyl), C₁-C₆ alkoxy (in another aspect, C₁-C₄ alkoxy), halogen, CF₃, OCF₃ or NR₆R₇; wherein
- R₆ and R₇: are independently H, C₁-C₆ alkyl (in another aspect, C₁-C₄ alkyl), phenyl(C₁-C₂)alkyl, C₂-C₆ alkanoyl, or phenyl(C₁-C₂)alkanoyl, wherein the phenyl groups are optionally substituted with 1, 2, or 3 groups that are independently halogen, C₁-C₄ alkyl, C₁-C₄ alkoxy, NO₂, OH, NH₂, NH(C₁-C₆)alkyl, N(C₁-C₆)alkyl(C₁-C₆)alkyl, CF₃, or OCF₃.

In another aspect, the invention provides the use of a compound of any preceding claim in the preparation of a medicament for the treatment of diabetes.

In another aspect, the invention encompasses a pharmaceutical composition comprising a compound according to claim 1 and at least one pharmaceutically acceptable carrier, solvent, adjuvant or excipient.

Illustrative compounds of the invention include the following, which were named using ChemDraw v. 6.02, which is sold by Cambridgesoft.com in Cambridge, MA.

| | |
|---|---|
| N-({4-[3-(4-methoxyphenyl)-5-(4-pentylphenyl)-4,5-dihydro-1H-pyrazol-l-yl]phenyl}sulfonyl)-N-methylphenylalanine | 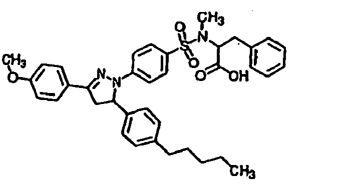 |
| N-methyl-N-[(4-{5-(4-pentylphenyl)-3-[4-(trifluoromethoxy)phenyl]-4,5-dihydro-1H-pyrazol-1-yl}phenyl)sulfonyl]phenylalanine | 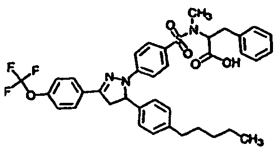 |

As noted above, compounds of the invention bind to and preferably, inhibit PTP-1B. As a result, compounds of the invention are useful in the treatment of various diseases, including controlling or treating Type 2 diabetes, improving glucose tolerance, and in improving insulin sensitivity in patients in need thereof. Compounds or their pharmaceutically acceptable salts are also useful in treating or controlling other PTP-1B mediated diseases, such as the treatment of cancer, neurodegenerative diseases and the like.

The term "alkoxy" represents an alkyl group of indicated number of carbon atoms attached to the parent molecular moiety through an oxygen bridge. Examples of alkoxy groups include, for example, methoxy, ethoxy, propoxy, isopropoxy and hexyloxy.

As used herein, the term "alkyl" includes those alkyl groups of a designed number of carbon atoms. Alkyl groups may be straight, or branched. Examples of "alkyl" include methyl, ethyl, propyl, isopropyl, butyl, iso-, sec- and tert-butyl, pentyl, hexyl, heptyl, 3-ethylbutyl, and the like.

The term "aryl" refers to an aromatic hydrocarbon ring system containing at least one aromatic ring. The aromatic ring may optionally be fused or otherwise attached to other aromatic hydrocarbon rings or non-aromatic hydrocarbon rings. Examples of aryl groups include, for example, phenyl, naphthyl, 1,2,3,4-tetrahydronaphthalene and biphenyl. Preferred examples of aryl groups include phenyl, naphthyl, and anthracenyl. More preferred aryl groups are phenyl and naphthyl. Most preferred is phenyl.

The term "cycloalkyl" refers to a C₃-C₈ cyclic hydrocarbon. Examples of cycloalkyl include cyclopropyl, cyclobutyl, cyclopentyl, cyclohexyl, cycloheptyl and cyclooctyl.

The terms "halogen" or "halo" indicate fluorine, chlorine, bromine, and/or iodine.

The term "heterocycloalkyl," refers to a ring or ring system containing at least one heteroatom selected from nitrogen, oxygen, and sulfur, wherein said heteroatom is in a non-aromatic ring. The heterocycloalkyl ring is optionally fused to or otherwise attached to other heterocycloalkyl rings and/or non-aromatic hydrocarbon rings and/or phenyl rings. Preferred heterocycloalkyl groups have from 3 to 7 members. Examples of heterocycloalkyl groups include, for example, 1,2,3,4-tetrahydroisoquinoline, piperazine, morpholine, piperidine, tetrahydrofuran, pyrrolidine, pyridinonyl, and pyrazole. Preferred heterocycloalkyl groups include piperidinyl, piperazinyl, morpholinyl, pyrrolidinyl, pyridinonyl, dihydropyrrolidinyl, and pyrrolidinonyl.

The term "heteroaryl" refers to an aromatic ring containing at least one heteroatom selected from nitrogen, oxygen, and sulfur. The heteroaryl ring may be fused or otherwise attached to one or more heteroaryl rings, aromatic or non-aromatic hydrocarbon rings or heterocycloalkyl rings. Examples of heteroaryl groups include, for example, pyridine, furan, thienyl, 5,6,7,8-tetrahydroisoquinoline and pyrimidine. Preferred examples of heteroaryl groups include thienyl, benzothienyl, pyridyl, quinolyl, pyrazolyl, pyrimidyl, imidazolyl, benzimidazolyl, furanyl, benzofuranyl, dibenzofuranyl, thiazolyl, benzothiazolyl, isoxazolyl, oxadiazolyl, isothiazolyl, benzisothiazolyl, triazolyl, pyrrolyl, indolyl, pyrazolyl, and benzopyrazolyl.

The compounds of this invention may contain one or more asymmetric carbon atoms, so that the compounds can exist in different stereoisomeric forms. These compounds can be, for example, racemates, chiral non-racemic or diastereomers. In these situations, the single enantiomers, i.e., optically active forms, can be obtained by asymmetric synthesis or by resolution of the racemates. Resolution of the racemates can be accomplished, for example, by conventional methods such as crystallization in the presence of a resolving agent; chromatography, using, for example a chiral HPLC column; or derivatizing the racemic mixture with a resolving reagent to generate diastereomers, separating the diastereomers via chromatography, and removing the resolving agent to generate the original compound in enantiomerically enriched form. Any of the above procedures can be repeated to increase the enantiomeric purity of a compound.

When the compounds described herein contain olefinic double bonds or other centers of geometric asymmetry, and unless otherwise specified, it is intended that the compounds include the cis, trans, Z- and E- configurations. Likewise, all tautomeric forms are also intended to be included.

The compounds of general Formula II may be administered orally, topically, parenterally, by inhalation or spray or rectally in dosage unit formulations containing conventional non-toxic pharmaceutically acceptable carriers, adjuvants and vehicles. The term parenteral as used herein includes percutaneous, subcutaneous, intravascular (e.g., intravenous), intramuscular, or intrathecal injection or infusion techniques and the like. In addition, there is provided a pharmaceutical formulation comprising a compound of general Formula II and a pharmaceutically acceptable carrier. One or more compounds of general Formula II may be present in association with one or more non-toxic pharmaceutically acceptable carriers and/or diluents and/or adjuvants, and if desired other active ingredients. The pharmaceutical compositions containing compounds of general Formula I may be in a form suitable for oral use, for example, as tablets, troches, lozenges, aqueous or oily suspensions, dispersible powders or granules, emulsion, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preservative agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques. In some cases such coatings may be prepared by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatin capsules, wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Formulations for oral use may also be presented as lozenges.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the active ingredients in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, may also be present.

Pharmaceutical compositions of the invention may also be in the form of oil-in-water emulsions. The oily phase may be a vegetable oil or a mineral oil or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol, glucose or sucrose. Such formulations may also contain a demulcent, a preservative and flavoring and coloring agents. The pharmaceutical compositions may be in the form of a sterile injectable aqueous or oleaginous suspension. This suspension may be formulated according to the known art using those suitable dispersing or wetting agents and suspending agents that have been mentioned above. The sterile injectable preparation may also be a sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The compounds of general Formula II may also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Compounds of general Formula II may be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

For disorders of the eye or other external tissues, e.g., mouth and skin, the formulations are preferably applied as a topical gel, spray, ointment or cream, or as a suppository, containing the active ingredients in a total amount of, for example, 0.075 to 30% w/w, preferably 0.2 to 20% w/w and most preferably 0.4 to 15% w/w. When formulated in an ointment, the active ingredients may be employed with either paraffinic or a water-miscible ointment base.

Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas. Examples of such dermal penetration enhancers include dimethylsulfoxide and related analogs. The compounds of this invention can also be administered by a transdermal device. Preferably topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane. The transdermal patch may include the compound in a suitable solvent system with an adhesive system, such as an acrylic emulsion, and a polyester patch. The oily phase of the emulsions of this invention may be constituted from known ingredients in a known manner. While the phase may comprise merely an emulsifier, it may comprise a mixture of at least one emulsifier with a fat, an oil, or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. Emulsifiers and emulsion stabilizers suitable for use in the formulation of the present invention include Tween 60, Span 80, cetostearyl alcohol, myristyl alcohol, glyceryl monostearate, and sodium lauryl sulfate, among others. The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for the active ingredients. The antiinflammatory active ingredients are preferably present in such formulations in a concentration of 0.5 to 20%, advantageously 0.5 to 10% and particularly about 1.5% w/w. For therapeutic purposes, the active compounds of this combination invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or nonaqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Dosage levels of the order of from about 0.1 mg to about 140 mg per kilogram of body weight per day are useful in the treatment of the above-indicated conditions (about 0.5 mg to about 7 g per patient per day). The amount of active ingredient that may be combined with the carrier materials to produce a single dosage form will vary depending upon the host treated and the particular mode of administration. Dosage unit forms will generally contain between from about 1 mg to about 500 mg of an active ingredient. The daily dose can be administered in one to four doses per day. In the case of skin conditions, it may be preferable to apply a topical preparation of compounds of this invention to the affected area two to four times a day.

It will be understood, however, that the specific dose level for any particular patient will depend upon a variety of factors including the activity of the specific compound employed, the age, body weight, general health, sex, diet, time of administration, route of administration, and rate of excretion, drug combination and' the severity of the particular disease undergoing therapy.

For administration to non-human animals, the composition may also be added to the animal feed or drinking water. It may be convenient to formulate the animal feed and drinking water compositions so that the animal takes in a therapeutically appropriate quantity of the composition along with its diet. It may also be convenient to present the composition as a premix for addition to the feed or drinking water. Preferred non-human animals include domesticated animals.

As noted above, the described are also methods and compositions for combination therapy of Type I and Type II diabetes. In one such aspect, methods of using compounds of formula II in combination with one or more angiotensin converting enzyme (ACE) inhibitors for improving the cardiovascular risk profile in patients experiencing or subject to Syndrome X or type II diabetes (non-insulin-dependent diabetes mellitus), preferably in human type II diabetics are described. These methods may also be characterized as the reduction of risk factors for heart disease, stroke or heart attack in a type II diabetic.

These methods include the reduction of hyperlipidemia in a patients experiencing or subject to Syndrome X or type II diabetes. These methods include methods lowering low density lipoprotein (LDL) blood levels and to increase high density lipoprotein (HDL) blood levels. The methods herein may further be characterized as useful for inhibiting, preventing or reducing atherosclerosis in a type II diabetics, or for reducing the risk factors thereof.

These methods also include the lowering of free fatty acid blood levels and triglyceride levels in type II diabetics.

Among the ACE inhibitors which may be utilized with the invention described herein are quinapril, ramipril, verapamil, captopril, diltiazem, clonidine, hydrochlorthiazide, benazepril, prazosin, fosinopril, lisinopril, atenolol, enalapril, perindropril, perindropril tert-butylamine, trandolapril and moexipril, or a pharmaceutically acceptable salt form of one or more of these compounds.

Described are also methods of using PTPase inhibitors of formula II for improving the cardiovascular or cerebrovascular risk profile in patients experiencing or subject to type II diabetes (non-insulin-dependent diabetes mellitus), preferably in human type II diabetics or a patient experiencing or subject to Syndrome X. These methods may also be characterized as the reduction of risk factors for heart disease, stroke or heart attack in a type II diabetic or a patient experiencing or subject to Syndrome X.

Described are also methods of using a pharmacological combination of one or more PTPase inhibiting agents, one or more biguanide agents, and, optionally one or more sulfonlylurea agents for treatment of type II diabetes or Syndrome X in a patient in need of such treatment. Described are also methods of using these agents to treat or inhibit metabolic disorders mediated by insulin resistance or hyperglycemia in a patient in need thereof. Described are also a method of modulating blood glucose levels in a patient in need thereof.

Each of these methods comprises administering to a patient in need thereof pharmaceutically effective amounts of:
a) a PTPase inhibiting agent of formula II; and
b) a biguanide agent; and
c) optionally, a sulfonylurea agent.

Biguanide agents useful with this invention include metformin and its pharmaceutically acceptable salt forms. Sulfonylurea agents useful for the methods and combinations described may be selected from the group of glyburide, glyburide, glipizide, glimepiride, chlorpropamide, tolbutamide, or tolazamide, or a pharmaceutically acceptable salt form of these agents.

This invention also provides pharmaceutical compositions and methods of using PTPase inhibitors of formula II in combination with one or more alpha-glucosidase inhibitors, such as miglitol or acarbose, for improving the cardiovascular risk profile in patients experiencing or subject to Syndrome X or type II diabetes (non-insulin-dependent diabetes mellitus), preferably in human type II diabetics. These methods may also be characterized as the reduction of risk factors for heart disease, stroke or heart attack in a patient in such need.

These methods include the reduction of hyperlipidemia in type II diabetics, including methods in type II diabetics for lowering low density lipoprotein (LDL) blood levels and to increase high density lipoprotein (HDL) blood levels. The methods herein may further be characterized as useful for inhibiting, preventing or reducing atherosclerosis in a type II diabetic or a patient experiencing or subject to Syndrome X, or the risk factors of either.

These methods also include the lowering free fatty acid blood levels and triglyceride levels in type II diabetics, or a patient experiencing or subject to Syndrome X.

Among the alpha-glucosidase inhibitors which may be utilized with the invention described herein are miglitol or acarbose, or a pharmaceutically acceptable salt form of one or more of these compounds.

Described are also methods for using a PTPase inhibitor of the invention and a sulfonylurea agent for the management of Syndrome X or type 2 diabetes and for improving the cardiovascular risk profile in patients experiencing or subject to those maladies. These methods may also be characterized as the reduction of risk factors in such patients for heart disease, stroke or heart attack in a type II diabetic. Such methods include the reduction of hyperlipidemia in a patients experiencing or subject to Syndrome X or type II diabetes and include methods for lowering low density lipoprotein (LDL) blood levels, high density lipoprotein (HDL) blood levels, and overall blood lipoprotein levels. The methods herein may further be characterized as inhibiting, preventing or reducing atherosclerosis in patients subject to or experiencing Syndrome X or type II diabetes, or the risk factors thereof. Such methods further include the lowering of free fatty acid blood levels and triglyceride levels in such patients.

Representative sulfonylurea agents include glipizide, glyburide (glibenclamide), chlorpropamide, tolbutamide, tolazamide and glimepriride, or the pharmaceutically acceptable salt forms thereof.

In addition, the invention provides combinations of a PTPase inhibitor of the invention and at least one thiazolidinedione agents. Such combinations are useful for treatment, inhibition or maintenance of Syndrome X or type II diabetes in patients in need of such treatment. Accordingly, methods of using such combinations are described. Thus, the compounds of the invention are useful for the preparation of these agents to treat or inhibit metabolic disorders mediated by insulin resistance or hyperglycemia in patients in need thereof. This invention may be further used to modulate blood glucose levels in a patient in need thereof.

Each of these methods comprises administering to a patient in need thereof pharmaceutically effective amounts of:
a) a thiazolidinedione agent, such as selected from the group of pioglitizone and rosiglitazone, or a pharmaceutically acceptable salt form of these agents; and
b) a compound of formula II.

The invention also provides pharmaceutical compositions and methods of using PTPase inhibitors in combination with one or more antilipemic agents. Such methods and compositions are useful for improving the cardiovascular risk profile in patients experiencing or subject to type II diabetes (non-insulin-dependent diabetes mellitus), preferably in type II diabetics or Syndrome X. These methods also include reducing the risk factors for heart disease, stroke or heart attack in a type II diabetic or a patient experiencing or subject to Syndrome X. Such methods further include the reduction of hyperlipidemia in type II diabetics, including such methods in type II diabetics for lowering low density lipoprotein (LDL) blood levels and to increase high density lipoprotein (HDL) blood levels. These compositions and methods are also useful for inhibiting, preventing or reducing atherosclerosis in a type II diabetic or a patient experiencing or subject to Syndrome X, or the risk factors thereof. In this aspect, the compositions and methods are useful for lowering of free fatty acid blood levels and triglyceride levels in type II diabetics, or patients experiencing or subject to Syndrome X.

Representative antilipemic or agents, also known as antihyperlipidemic agents, suitable for use in the invention are bile acid sequestrants, fibric acid derivatives, HMG-CoA reductase inhibitors and nicotinic acid compounds. Bile acid sequestrant agents useful with this invention include colestipol and colesevelam, and their pharmaceutically acceptable salt forms. Fibric acid derivatives which may be used with the present invention include clifofibrate, gemfibrozil and fenofibrate. HMG-CoA reductase inhibitors, also known as statins, useful with this invention include cerivastatin, fluvastatin, atorvastatin, lovastatin, pravastatin and simvastatin, or the pharmaceutically acceptable salt forms thereof. Niacin is an example of a nicotinic acid compound which may be used with the methods described. Also useful are lipase inhibiting agents, such as orlistat.

This invention also provides pharmaceutical compositions that are a combination of a compound of Formula 11 and an aldose reductase inhibitor (ARI). Such combinations are useful in methods for treating, inhibiting or preventing type II diabetes, or its related and associated symptoms, disorders and maladies. These methods comprise administering to a patient in need of such therapy a pharmaceutically effective amount of a composition comprising a combination of pharmaceutically effective amounts of a compound of formula II and an ARI. These compositions and methods are useful for the treatment, prevention or inhibition of diabetic neuropathy, diabetic nephropathy, retinopathy, keratopathy, diabetic uveitis, cataracts.

Representative suitable ARIs are disclosed in U.S. Patent Nos. 6,420,426 and 6,214,991.

Combinations of the compounds of Formula II and an ARI are also useful for inhibition or reduction of risk factors for heart disease, stroke or heart attack in a type II diabetic. Therefore, in this aspect the invention is useful for reducing hyperlipidemia and/or low density lipoprotein (LDL) blood levels in type II diabetics. Also included in this aspect are methods for inhibiting, preventing or reducing atherosclerosis or the risk factors thereof in type II diabetics. This aspect includes lowering of free fatty acid blood levels and triglyceride levels.

Described are also methods of using a compound of formula I and insulin(s) for the management of type I or type II diabetes. Accordingly, the invention provides for combination therapy, i.e., where a compound of Formula II is administered in combination with insulin. Such combination therapy encompasses simultaneous or sequential administration of the compound of Formula II and insulin. The insulins useful in this aspect include both naturally occurring and synthetic insulins.

Insulins useful with the methods and combinations disclosed herein include rapid acting insulins, intermediate acting insulins, long acting insulins and combinations of intermediate and rapid acting insulins.

Rapid acting commercially available insulin products include HUMALOG^{®} Brand Lispro Injection (rDNA origin); HUMULIN® Regular Human' Injection, USP [rDNA origin]; HUMULIN® Regular U-500 Concentrated Human Injection, USP [rDNA origin]; REGULAR ILETIN^{®} II (insulin injection, USP, purified pork) available from Eli Lilly and Co.; and the NOVALIN^{®} Human Insulin Injection and VENOSULIN^{®} BR Buffered Regular Human Injection, each available from Novo Nordisk Pharmaceuticals.

Commercially available intermediate acting insulins useful with this invention include, but are not limited to, the HUMULIN^{®} L brand LENTE^{®} human insulin [rDNA origin] zinc suspension, HUMULIN® N NPH human insulin [rDNA origin] isophane suspension, LENTE^{®} ILETIN.RTM. II insulin zinc suspension, USP, purified pork, and NPH ILETIN^{®} II isophane insulin suspension, USP, purified pork, available from Eli Lilly and Company, LANTUS^{®} insulin glargine [rDNA origin] injection, available from Aventis Pharmaceuticals, and the NOVOLIN L Lente^{®} human insulin zinc suspension (recombinant DNA origin), and NOVOLIN^{®} N NPH human insulin isophane suspension (recombinant DNA origin) products available from Novo Nordisk Pharmaceuticals, Inc, Princeton N.J.

Also useful with the methods and formulations disclosed herein are intermediate and rapid acting insulin combinations, such as the HUMALOG^{®} Mix 75/25 (75% Insulin Lispro Protamine Suspension and 25% Insulin Lispro Injection), HUMULIN^{®} 50/50 (50% Human Insulin Isophane Suspension and 50% Human Insulin Injection) and HUMULIN^{®} 70/30 (70% Human Insulin Isophane Suspension and 30% Human Insulin Injection), each available from Eli Lilly and Company. Also useful are the NOVALIN^{®} 70/30 (70% NPH, Human Insulin Isophane Suspension and 30% Regular, Human Insulin Injection) line of combination products available from Novo Nordisk Pharmaceuticals.

A commercially available long acting insulin for the use disclosed herein is the HUMULIN^{®} U Ultralente^{®} human insulin [rDNA origin] extended zinc suspension, available from Eli Lilly and Company.
Also useful in the methods disclosed in the present application are inhaled insulin products, such as the EXUBERA^{®} inhaled insulin product developed by Pfizer Inc. and Aventis SA.

Each of these insulin products can be administered as directed by a medical professional using administrations, dosages and regimens known in the art, such as those published for each product in the Physicians' Desk Reference, 55 Edition, 2001, published by Medical Economics Company, Inc. at Montvale, N. J.. In this aspect, for example, methods for improving the cardiovascular and cerebrovascular risk profiles in patients experiencing or subject to type I or type II diabetes (non-insulin-dependent diabetes mellitus), preferably in human type II diabetics are described. These methods may also be characterized as the inhibition or reduction of risk factors for heart disease, stroke or heart attack in a type II diabetic.

The compounds of the present invention may be prepared by use of known chemical reactions and procedures. Representative methods for synthesizing compounds of the invention are presented below. It is understood that the nature of the substituents required for the desired target compound often determines the preferred method of synthesis. All variable groups of these methods are as described in the generic description if they are not specifically defined below.

### Methods of Preparation

Compounds of the invention where A in Formula II is a thiazole, and L₂ is -C(O)N(R₉)- can be conveniently prepared from a substituted 2-bromoacetophenone using general Scheme A set forth below. In this method, the desired 2-bromoacetophenone A-1 is cyclized with thiourea A-2 in an alcoholic solvent to form the thiazole product A-3. Subsequent bromination with NBS followed by coupling with a Q-boronic acid provides intermediate A-6. If the boronic acid is not readily available, other metallic intermediates such as the tin or zinc reagent may be used. The 2-aminothiazole can then be coupled to the desired acid chloride A-7 using standard conditions. The resulting sulfonyl chloride A-8 can subsequently be coupled to the amino acid ester A-9 by treatment with a base like triethylamine or pyridine in DMF or dichloromethane. The coupled product A-10 can then be hydrolyzed to give the target compound, A-11, by treatment with aqueous NaOH in a solvent like THF or ethanol.

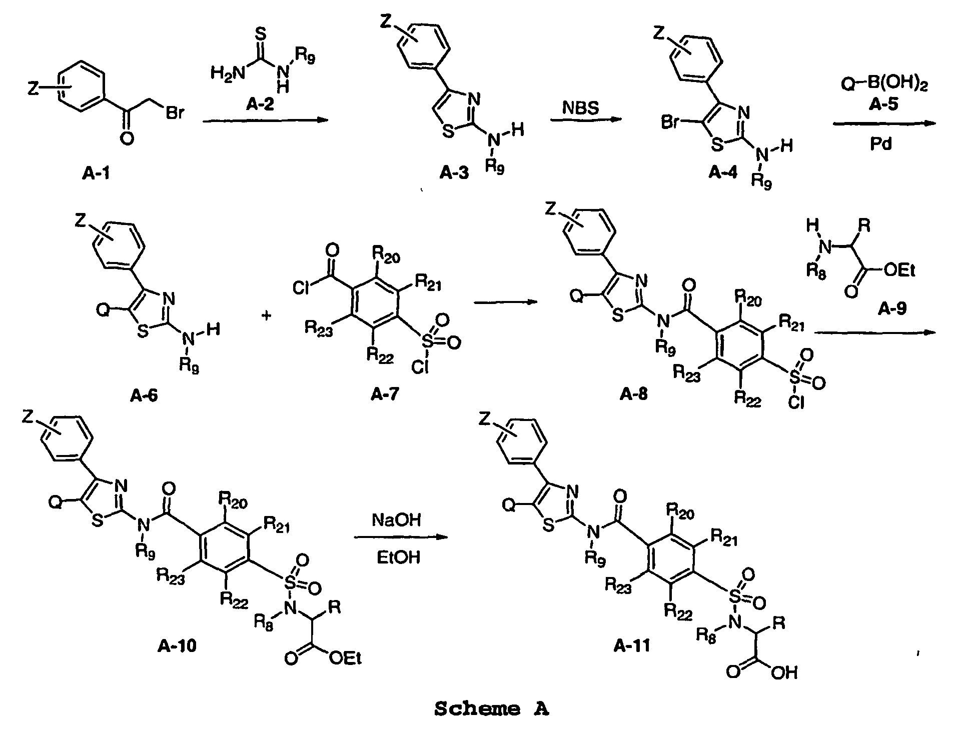

An alternate method for preparing the 4,5-disubstituted-2-amino thiazole intermediate is outlined in scheme B. Here a substituted benzyl chloride or bromide B-1 is treated with magnesium to form the corresponding Grignard reagent B-2. Subsequent treatment with nitrile B-3 followed by an acid hydrolysis gives ketone B-4. Bromination with PTT and subsequent cyclization with thiourea gives the desired aminothiazole intermediate B-6. Alternatively, ketone B-4 can be converted directly to thiazole B-6 by treatment with iodine and thiourea.

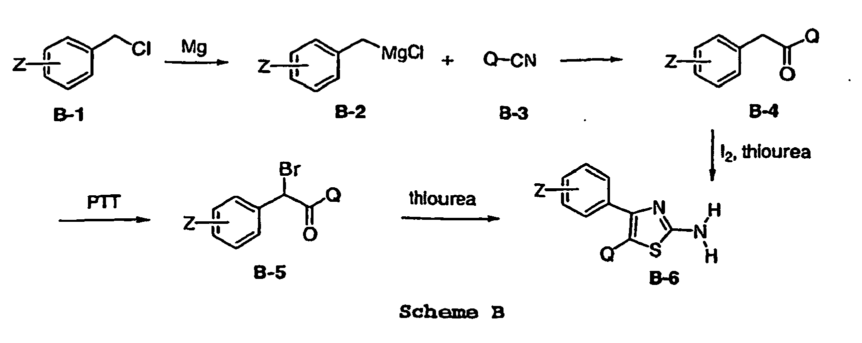

Compounds of the invention where A in Formula II is a thiazole, and L₂ and L₃ are bonds can be prepared from a substituted 2-bromoacetophenone using general Scheme C set forth below.

In this method sulfonyl chloride C-1 is coupled to aminoalkyl acid C-2 to give the sulfonamide. Treatment of methyl ketone C-3 with PTT followed by thiourea give 2-aminothiazole C-5. Bromination at the 5-position with NBS gives bromide C-6, which can be coupled to a variety of Q groups (C-7). Many possible methods can be used for this coupling reaction. Some of the most common methods use an aryl or heteroaryl boronic acid, tin or zinc reagent with a palladium catalyst. The amino group on the thiazole intermediate C-7 can be functionalized using a variety of alkylation or acylation reactions. Subsequent hydrolysis of these intermediates gives target compounds C-8. Alternatively, Intermediate C-7 can be treated with t-BuONO and CuBr₂ to give the corresponding bromide (C-9), which can be modified, as previously described to give target compounds C-10.

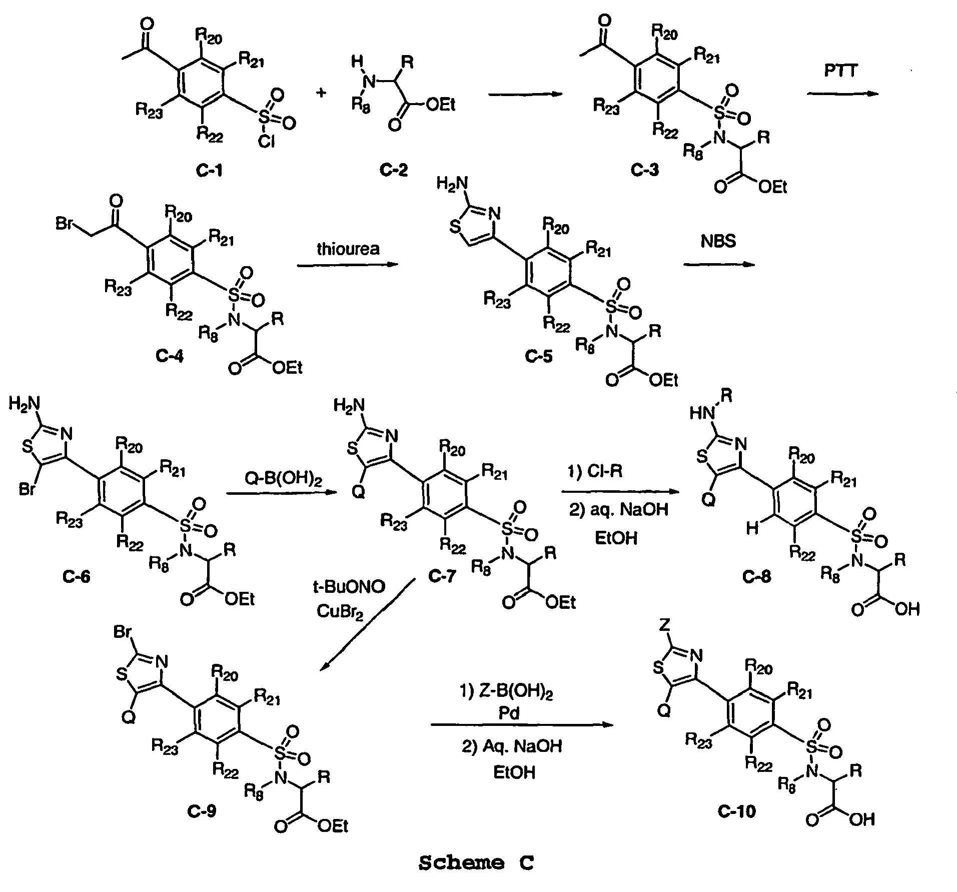

Compounds of the invention where A is a substituted pyrazole or pyrazoline and L₂ is a bond can typically be prepare using the chemistry described in scheme D. Here methyl ketone D-1 and aldehyde D-2 undergo an aldol condensation and form an α, β-unsaturated ketone D-3 when they are treated with a sodium alkoxide in an alcohol solvent. Intermediate D-3 can subsequently be cyclized with an appropriately substituted hydrazine D-4 to give the pyrazoline product D-5. Hydrolysis gives the pyrazoline product D-6. Alternatively, intermediate D-5 may be oxidized with DDQ to give ester intermediate D-7. Hydrolysis gives the desired pyrazole target compound D-8.

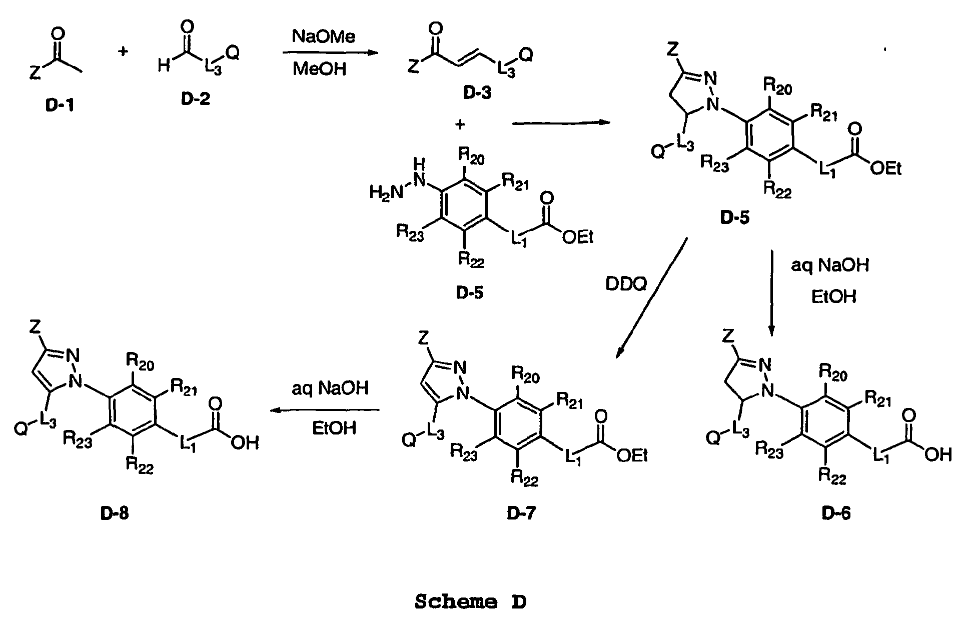

Compounds with a variety of L₁ linkers (Formula I) can be prepared using the chemistry described in general scheme E. Here aryl or heteroaryl bromide E-1 is coupled to intermediate E-2 containing a functional group X that can be modified to provide the desired L₁-CO₂R substituent. The initial coupling reaction between intermediates E-1 and E-2 can often be carried out using a transition metal coupling reaction. Some of the most useful reactions of this type include the Suzuki, Stille and Negishi reactions. Alternatively, for some examples, it may be more convenient to reverse the coupling functional groups such that metal-M is on the E-1 intermediate and the halogen, preferable Br or I, is on the E-2 intermediate. A variety of X substituents may be useful for preparing compounds with a specific L₁-CO₂R group. Some useful X substituents include sulfonamides, acids, esters, aldehydes, ketones, amides, nitro groups, anilino groups, hydroxyl groups, sulfides and halides. Some examples of targets compounds prepared from intermediate E-3 with X equal to aldehyde or ketone are illustrated in scheme E.

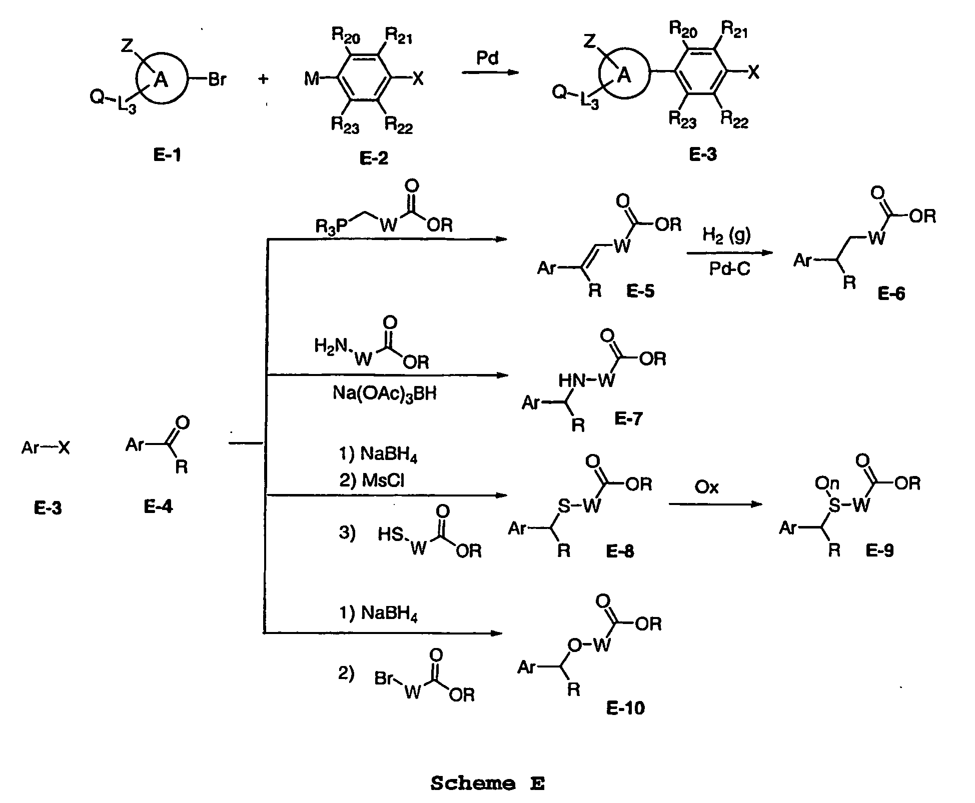

Treatment of carbonyl compound E-4 with a Wittig type reagent provides the unsaturated derivative E-5. If the saturated compound E-6 is required, simple hydrogenation with, for example, palladium on carbon can be used. In some cases the carboxylic acid moiety (R = H) may need to be protected as an ester to facilitate the reactions in the scheme. Carbonyl compound E-4 can also be coupled with an amine derivative using a reducing agent like sodium triacetoxyborohydride in a reductive amination reaction to give the corresponding amine E-7. Reduction of aldehyde or ketone E-4 with sodium borohydride gives the corresponding alcohol. Subsequent conversion of this alcohol to a leaving group such as a mesylate or halide followed be displacement with a nucleophile such as a thiol gives sulfide E-8, which if desired can be oxidized to form the sulfoxide or sulfone. Similarly, the same mesylate or halogen leaving group can be displaced by other nucleophiles like amines or alcohols to give the corresponding amine and ether linkers. The sodium borohydride reduction product can also be coupled directly to an alkyl halide or substituted phenol using simple alkylation or Mitsunobu conditions respectively.

Those having skill in the art will recognize that the starting materials and reaction conditions may be varied, the sequence of the reactions altered, and additional steps employed to produce compounds encompassed by the present invention, as demonstrated by the following examples. In some cases, protection of certain reactive functionalities may be necessary to achieve some of the above transformations. In general, the need for such protecting groups as well as the conditions necessary to attach and remove such groups will be apparent to those skilled in the art of organic synthesis.

The preparation of the compounds of the present invention is illustrated further by the following examples. In all cases, unless otherwise specified, the column chromatography is performed using a silica gel solid phase.

### Example 1

### Preparation of 2-{4-[4-(4-Chloro-phenyl)-5-(4-ethylphenyl)-thiazol-2-ylcarbamoyl]-benzenesulfonylamino}-3-phenyl-propionic acid

### Step 1: Preparation of 1-(4-Chloro-phenyl)-2-(4-ethylphenyl)-ethanone

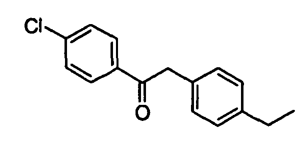

In an oven dried 50 mL round-bottomed flask under an atmosphere of dry nitrogen, a solution of magnesium turnings (2.92 g, 120 mmol) in anhyd THF (10 mL) is treated with 4-ethylbenzylchloride (17.7g, 114 mmol) in anhyd THF (60 mL) and then stirred for 30 min. While stirring, the temperature is maintained between 30 and 38 °C using an ice-bath. The resulting purple solution is stirred at room temperature for an additional 3 h, cooled to -10 °C (NaCl-ice water bath) and then a solution of 4-chlorobenzyl nitrile (13.0 g, 94.6 mmol) in anhyd THF (50 mL) is added over 10 min. During the addition the temperature is maintained between -10 and -15 °C. After the addition is complete, the ice bath is removed and the resulting solution is stirred for an additional 4 h. Once complete, the reaction mixture is cooled to 0 °C degree (ice-bath), and acidified with 5% aq H₂SO₄ to a pH < 7. After stirring an additional 15 min without the ice-bath, the solution is extracted with diethyl ether (2 x 200 mL) and the combined organic extracts are washed sequentially with water (150 mL) and sat'd aq NaCl (100 mL). The resulting solution is dried over MgSO₄, filtered, concentrated under reduced pressure, and then purified by flash column chromatography (0-15% ethyl acetate in heptane) to give 1-(4-Chloro-phenyl)-2-(4-ethyl-phenyl)-ethanone as a yellow oil (13.1 g, 54%).

### Step 2: Preparation of 4-(4-Chloro-phenyl)-5-(4-ethylphenyl)-thiazol-2-ylamine

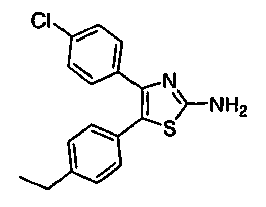

In an oven dried 100 mL round-bottomed flask under an atmosphere of dry nitrogen, 1-(4-chloro-phenyl)-2-(4-ethylphenyl)-ethanone (13.1 g, 50.6 mmol), thiourea (7.71 g, 101 mmol) and iodine (12.8 g, 50.6 mmol) are combined and the neat mixture is heated to 90 °C. After stirring for about 72 h, 50 mL of H₂O is added and the mixture is then heated in a 120 °C oil bath for 30 min. After cooling to room temperature, the reaction mixture is poured into water (300 mL), basified with 1 N aq NaOH to a pH >8 and then extracted with dichloromethane (2 x 350 mL). The combined organic extracts are washed successively with water (200 mL) and sat'd aq NaCl (100 mL), dried over MgSO₄, filtered and concentrated. The concentrate is purified by flash column chromatography (10-30% ethyl acetate in heptane) to afford 4-(4-chloro-phenyl)-5-(4-ethyl-phenyl)-thiazol-2-ylamine (5.32 g, 33%).

### Step 3: Preparation of 2-{4-[4-(4-Chloro-phenyl)-5-(4-ethyl-phenyl)-thiazol-2-ylcarbamoyl]-benzenesulfonylamino}-3-phenyl-propionic acid tert-butyl ester

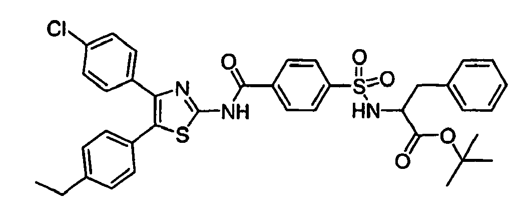

In an oven dried 50 mL round-bottomed flask under an atmosphere of dry nitrogen, a solution of 4-chlorosulfonylbenzoic acid (350 mg, 1.59 mmol) in dry 1,2-dichloroethane (10 mL) is treated with thionyl chloride (0.35 mL, 4.0 mmol), and then heated to reflux in a 100 °C oil bath for 4 h. After cooling to room temperature, the solution is concentrated under reduced pressure. The resulting residue is dissolved in anhyd. dichloromethane (15 mL), cooled to 0 °C, and then a solution of 4-(4-chloro-phenyl)-5-(4-ethyl-phenyl)-thiazol-2-ylamine (500 mg, 1.59 mmol) in dichloromethane (15 mL) is added over a time period of approximately 2 min. After stirring for about 1h at 0 °C, diisopropylethylamine (0.30 mL, 1.6 mmol) is added, and the solution is stirred for an additional 3 h. Next, H-Phe-Ot-Bu.HCl (652 mg, 1.59 mmol) and diisopropylethylamine (0.69 mL, 4.0 mmol) are added, and the solution is stirred for 1 h. The reaction mixture is then allowed to warm to room temperature, stirring is continued for 1 h, and then the reaction mixture is concentrated under reduced pressure. The resulting residue is purified by flash column chromatography (20-30% ethyl acetate in heptane) to give 2-{4-[4-(4-chloro-phenyl)-5-(4-ethyl-phenyl)-thiazol-2-ylcarbamoyl]-benzenesulfonylamino}-3-phenyl-propionic acid tert-butyl ester (610 mg, 55%) as a yellow solid.

### Step 4: Preparation of 2-{4-[4-(4-Chloro-phenyl)-5-(4-ethylphenyl)-thiazol-2-ylcarbamoyl]-benzenesulfonylamino}-3-phenyl-propionic acid

2-{4-[4-(4-Chloro-phenyl)-5-(4-ethyl-phenyl)-thiazol-2-ylcarbamoyl]-benzenesulfonylamino}-3-phenyl-propionic acid tert-butyl ester (1.20 g, 1.71 mmol) is treated with trifluoroacetic acid (6 mL) at room temperature. After stirring for 3 h. the solution is concentrated under reduced pressure and triturated with diethyl ether (3 mL). The yellow precipitate is filtered and washed with diethyl ether (2 x 3 mL), to give 2-{4-[4-(4-chloro-phenyl)-5-(4-ethyl-phenyl)-thiazol-2-ylcarbamoyl]-benzenesulfonylamino}-3-phenyl-propionic acid (750 mg, 68%) as a pale yellow solid.

### Example 2

### Preparation of 2-({4-[3-(4-Chloro-phenyl)-5-(4-ethylphenyl)-pyrazol-1-yl]-benzenesulfonyl}-methyl-amino)-3-phenyl-propionic acid

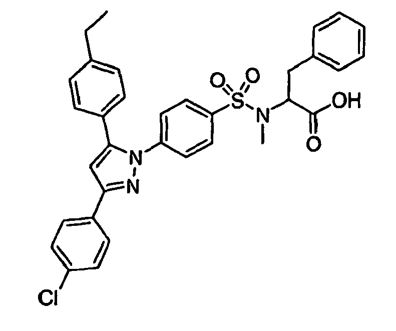

### Step 1: Preparation of 1-(4-Chloro-phenyl)-3-(4-ethyl-phenyl)-propenone

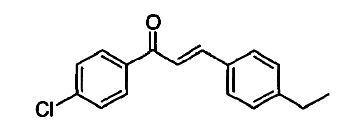

A solution of 4-chloroactophenone (6.18 g, 10 mmol) and 4-ethylbenzaldehyde (5.76 mL, 10.5 mmol) in dry methanol (25 mL) is treated with sodium methoxide (0.568 g, 10.5 mmol) and stirred at room temperature for 16 h. After the reaction is complete, the solution is acidified with 0.5 N HCl (25 mL) and the resulting precipitate is collected by filtration. The crude product is washed with a 50% methanol in water solution (3 X 25 mL) to give 1-(4-chloro-phenyl)-3-(4-ethyl-phenyl)-propenone (10.6 g, 98%) No further purification is required.

Step 2: Preparation of 2-(4-Nitro-benzenesulfonylamino)-3-phenyl-propionic acid ethyl ester

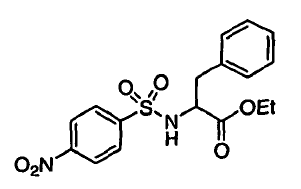

A solution of phenylalanine ethyl ester hydrochloride (3.0 g, 10 mmol) and N,N-diisopropylethylamine (3.8 mL, 22 mmol) in dichloromethane (50 mL) is cooled to 0 °C (ice-water bath) and treated with 4-nitrobenzensulfonyl chloride (2.33 g, 10.5 mmol). After 30 min the solution is warmed to room temperature and stirring is continued for an additional 4 h. After the reaction is complete, the reaction mixture is diluted with water (100 mL), and the organic layer is extracted with dichloromethane (3 x 50 mL). The combined organic extracts are dried over MgSO4, filtered and concentrated. Purification by flash column chromatography (10% ethyl acetate in heptane) gives 2-(4-nitro-benzenesulfonylamino)-3-phenyl-propionic acid ethyl ester (3.56 g, 94%) as a white crystalline solid.

### Step 3: Preparation of 2-[Methyl-(4-nitro-benzenesulfonyl)-amino]-3-phenyl-propionic acid ethyl ester

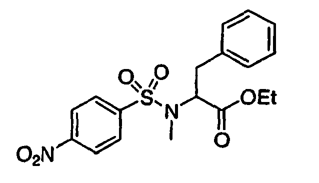

A solution of N-(4-nitrobenznesulfonyl)-phenylalanine ethyl ester (3.8 g, 10 mmol) and cesium carbonate (3.58 g, 11 mmol) in DMF (25 mL) is stirred for 10 min, then treated with methyl iodide (0.75 mL, 12 mmol). After stirring an additional 16 h, the reaction mixture is filtered; the filtrate is diluted with water (50 mL) and extracted with ethyl acetate (3 X 50 mL). The combined organic extracts are dried over MgSO₄, filtered and concentrated. Purification by flash column chromatography (10% ethyl acetate in heptane) gave 2-[methyl-(4-nitrobenzenesulfonyl)-amino]-3-phenyl-propionic acid ethyl ester (3.5 g, 90%) as a white crystalline solid.

### Step 4: Preparation of 2-[(4-Amino-benzenesulfonyl)-methyl-amino]-3-phenyl-propionic acid ethyl ester

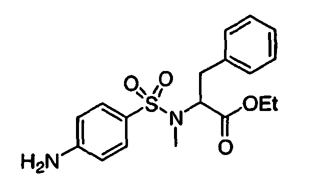

A solution of 2-[methyl-(4-nitro-benzenesulfonyl)-amino]-3-phenyl-propionic acid ethyl ester (3.5 g, 9.0 mmol) and palladium on carbon (0.4 g) in ethyl acetate (25 mL), is treated with hydrogen gas at 20 psi for 4 h at room temperature. The resulting solution is filtered and concentrated. No further purification is necessary.

### Step 5: Preparation of 2-[(4-Hydrazino-benzenesulfonyl)-methyl-amino]-3-phenyl-propionic acid ethyl ester hydrochloride

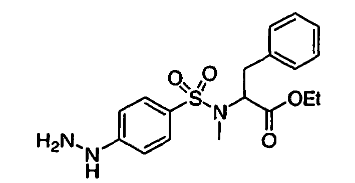

A solution of 2-[(4-amino-benzenesulfonyl)-methyl-amino]-3-phenyl-propionic acid ethyl ester in conc HCl (12 mL) is cooled to 0 °C (ice-bath) and carefully treated with aq NaNO₂ (0.69 g in 5 mL water, 10 mmol) at a rate such that the temperature does not rise above 5 °C. Stirring is continued for 1.5 h. Once complete, the solution is transferred to a separate ice-cooled solution of SnCl₂ (7.11 g, 37.5 mmol) in conc HCl (12 mL) and slowly warmed to room temperature with continued stirring for 2 h. The resulting precipitate is collected by filtration and washed with benzene (3 x 100 mL) to give 2-[(4-hydrazino-benzenesulfonyl)-methyl-amino]-3-phenyl-propionic acid ethyl ester hydrochloride (xx g, 98%) as a white solid.

### Step 6: Preparation of 2-({4-[3-(4-Chloro-phenyl)-5-(4-ethyl-phenyl)-4,5-dihydro-pyrazol-1-yl]-benzenesultonyl}-methyl-amino)-3-phenyl-propionic acid ethyl ester

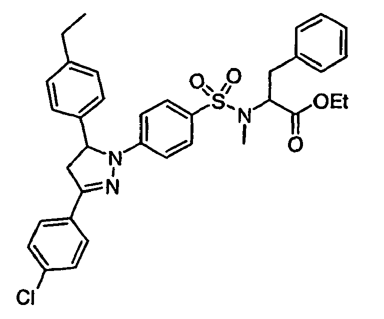

A solution of 1-(4-Chloro-phenyl)-3-(4-ethyl-phenyl)-propenone (0.270 g, 1 mmol) and 2-[(4-hydrazino-benzenesulfonyl)-methyl-amino]-3-phenyl-propionic acid ethyl ester hydrochloride (0.452 g, 1.1 mmol) in ethanol (50 mL) is heated to reflux for 4 h. After cooling to room temperature, the solution is concentrated, diluted with water (50 mL) and extracted with ethyl acetate (3 X 50 mL). The combined organic extracts are dried over MgSO₄, filtered and concentrated. Purification by flash column chromatography (10% ethyl acetate in heptane) provides 2-({4-[3-(4-chloro-phenyl)-5-(4-ethylphenyl)-4,5-dihydro-pyrazol-1-yl]-benzenesulfonyl}-methyl-amino)-3-phenyl-propionic acid ethyl ester (0.49 g, 78%)

### Step 7: Preparation of 2-({4-[3-(4-Chloro-phenyl)-5-(4-ethyl-phenyl)-pyrazol-1-yl]-benzenesulfonyl}-methyl-amino)-3-phenyl-propionic acid ethyl ester

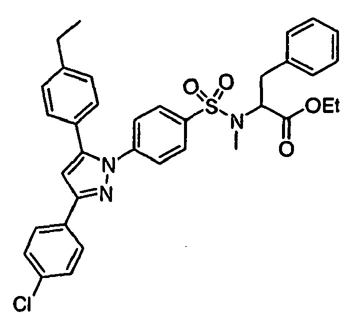

A solution of 2-({4-[3-(4-chloro-phenyl)-5-(4-ethylphenyl)-4,5-dihydro-pyrazol-l-yl]-benzenesulfonyl}-methyl-amino)-3-phenyl-propionic acid ethyl ester (0.25 g, 0.4 mmol) in benzene (20 mL) is treated with 2,3-dichloro-5,6-dicyano-1,4-benzoquinone (DDQ) (0.136 g, 0.6 mmol) and refluxed for 6 h. After cooling to room temperature, the resulting solution is concentrated and purified by flash column chromatography (10% ethyl acetate in heptane) to give 2-({4-[3-(4-chloro-phenyl)-5-(4-ethyl-phenyl)-pyrazol-1-yl]-benzenesulfonyl}-methyl-amino)-3-phenyl-propionic acid ethyl ester (0.232 g, 95%).

### Step 8: Preparation of 2-({4-[3-(4-Chloro-phenyl)-5-(4-ethyl-phenyl)-pyrazol-1-yl]-benzenesulfonyl}-methyl-amino)-3-phenyl-propionic acid

A solution of 2-({4-[3-(4-chloro-phenyl)-5-(4-ethylphenyl)-pyrazol-1-yl]-benzenesulfonyl}-methyl-amino)-3-phenyl-propionic acid ethyl ester in anhyd THF (2 mL) and methanol (6 mL) is treated with 10% v/v aq KOH (1 mL, 1 mmol). After stirring for 2 h, the solution is acidified to pH 2-3 with 0.5 N HCl, extracted with ethyl acetate (3 x 15 mL) and concentrated. Purification by flash column chromatography (50% ethyl acetate in heptane) gives 2-({4-[3-(4-chloro-phenyl)-5-(4-ethyl-phenyl)-pyrazol-1-yl]-benzenesulfonyl}-methyl-amino)-3-phenyl-propionic acid (0.160 g, 85%) as a white solid.

### Example 3 (not according to the invention)

### 4-{4-[(-Chlorobenzyl)-(4-trifluoromethoxybenzenesulfonyl)-amino]-phenyl}-4-oxo-2-pyridin-3-ylmethyl-butyric acid.

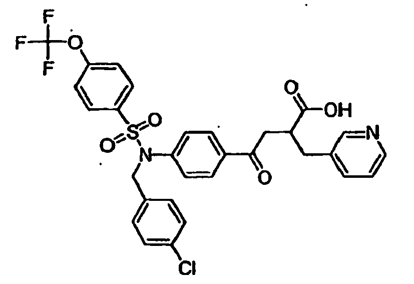

### Step 1: N-[4-(2-Bromoacetyl)-phenyl]-4-trifluoromethoxybenzenesulfonamide

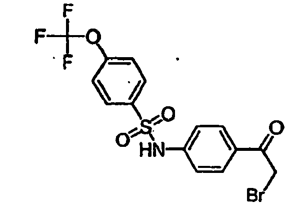

4-Trifluoromethoxybenzenesulfonyl chloride (3.18 g, 2.07 mL, 1.22 mmol) was added to a solution of 4'-aminoacetophenone (1.5 g, 1.11 mmol) and triethylamine (3.1 mL, 2.22 mmol) in anhydrous methylene chloride (50 mL). The reaction was stirred for 16 hours and then poured into water (50 mL), and extracted with diethyl ether (3 x 30 mL). The combined extract was washed with 0.5 N hydrochloric acid (2 x 10 mL), water and finally brine. The ethereal solution was dried over anhydrous MgSO₄, filtered and concentrated *in vacuo.* The product methyl ketone was used in the subsequent bromination step without further purification.

Phenyltrimethylammonium tribromide (4.68 g, 1.22 mmol) was added to a solution of the methyl ketone (prepared in the previous step) in anhydrous dioxan (50 mL). The reaction eas stirred at room temperature for 3 hours and then poured into water (50 mL), and extracted with diethyl ether (3 x 30 mL). The combined extract was washed with 0 water and brine. The ethereal solution was dried over anhydrous MgSO₄, filtered and concentrated *in vacuo.* Purification of the product by flash column chromatography, using 20 % ethyl acetate/heptane as eluent, afforded the title compound has a white solid (4.36 g, 89%); 1H NMR (CDCl₃, 300 MHz): δ 7.92 (4H, d, J = 8 Hz, Ar-H), 7.38 (2H, d, J = 8 Hz, Ar-H), 7.20 (3H, m, Ar-H, NH), 4.38 (2H, s, CH₂Br).

### Step 2: 2-Pyridin-3-ylmethyl-malonic acid diallyl ester

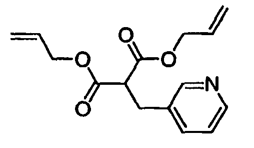

A solution of diallyl malonate (3.0 g, 16.3 mmol) in anhydrous THF (30 mL) was added cautiously to a stirred suspension of sodium hydride (95%, 900 mg, 36 mmol) in anhydrous THF (25 mL). The resulting solution was stirred at room temperature for 1 hr. A solution of 3-(iodomethyl)pyridine hydroiodide (6.24 g, 18 mmol) in anhydrous THF (25 mL) was added dropwise, and the resultant solution was stirred at room temperature for 16-24 hrs (TLC control). The reaction mixture was poured into water (50 mL), and extracted with ethyl acetate (3 x 50 mL). The combined extract was washed with water, brine, dried over anhydrous MgSO₄, filtered and concentrated *in vacuo.* Trituration and filtration from MeOH afforded the title compound as a white solid (4.03g, 90%); 1H NMR (CDCl₃, 300 MHz): δ 8.48 (2H, m), 7.38 (1H, td, J = 8, 2 Hz, Ar-H), 7.20 (2H, dd, J = 8, 5 Hz), 5.82 (2H, m), 5.26 (4H, m), 4.60 (4H, m), 3.88 (1H, t, J = 7 Hz), 3.21 (2H, d, J = 7 Hz).

### Step 3: 2-{2-oxo-2-[4-(4-trifluoromethoxybenzenesulfonylamino]-ethyl}-2-pyridin-3-ylmethyl-malonic acid diallyl ester

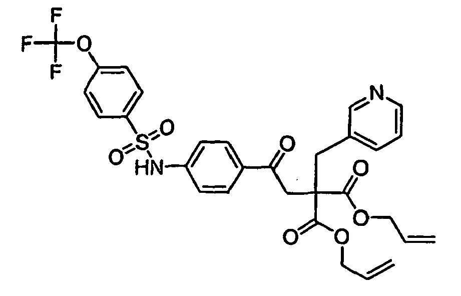

A solution of 2-pyridin-3-ylmethyl-malonic acid diallyl ester (1.15g, 4.18 mmol) in anhydrous THF (30 mL) was added to a stirred suspension of sodium hydride (95%, 232 mg, 9.2 mmol) in anhydrous THF (25 mL). The resulting solution was stirred at room temperature for 1 hr. A solution of N-[4-(2-Bromoacetyl)-phenyl]-4-trifluoromethoxy-benzenesulfonamide (2.01 g, 4.6 mmol) in anhydrous THF (25 mL) was added dropwise, and the resultant solution was stirred at 50°C for 5 hrs (TLC control). The reaction mixture was poured into water (50 mL), and extracted with ethyl acetate (3 x 50 mL). The combined extract was washed with water, brine, dried over anhydrous MgSO₄, filtered and concentrated *in vacuo.* Purification of the product by flash column chromatography, using 20 % ethyl acetate/heptane as eluent, afforded the title compound has a white solid (4.36 g, 89%); 1H NMR (CDCl₃, 300 MHz): δ 8.48 (1H, d, J = 3 Hz), 8.20 (1H, s), 7.90 (2H, d, J = 8 Hz), 7.78 (2H, d, J = 8 Hz), 7.42 (1H, d, J = 7 Hz), 7.32 (3H, m), 7.20 (1H, m), 7.12 (2H, d, J = 8 Hz), 5.88 (2H, m), 5.29 (4H, m), 4.62 (4H, s), 3.58 (2H, s), 3.50 (2H, s); ESI-LCMS e/z calcd for C₃₀H₂₇F₃N₂O₈S: 632.610, found 633 (M+H)⁺.

### Step 4: 4-{4-[(-Chlorobenzyl)-(4-trifluoromethoxybenzenesulfonyl)-amino]-phenyl}-4-oxo-2-pyridin-3-ylmethyl-butyric acid.

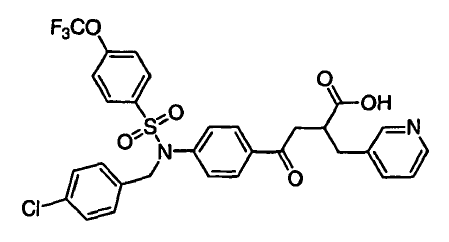

A solution of 2-{2-oxo-2-[4-(4-trifluoromethoxybenzenesulfonylamino]-ethyl}-2-pyridin-3-ylmethyl-malonic acid diallyl ester (1.06g, 1.67 mmol) in anhydrous THF (15 mL) was added to a stirred suspension of sodium hydride (95%, 47 mg, 1.84 mmol) in anhydrous THF (10 mL). The resulting solution was stirred at room temperature for 1 hr. A solution of 4-chlorobenzyl chloride (0.3 g, 1.84 mmol) in anhydrous THF (25 mL) was added dropwise, and the resultant solution was stirred at 50°C for 5 hrs (TLC control). The reaction mixture was poured into water (50 mL), and extracted with ethyl acetate (3 x 50 mL). The combined extract was washed with water, brine, dried over anhydrous MgSO₄, filtered and concentrated *in vacuo* affording the N-alkylated diallyl ester.

The diallyl ester was redissolved in dioxan (15 mL). *Tetrakis*-(Triphenylphospine)-palladium(0) (5 mg) and triethylamine (0.1 mL) was added to the stirred solution, and then the reaction was heated to 100°C for 30 mins, cooled to room temperature and concentrated *in vacuo.* Purification of the product by flash column chromatography, using 20 % ethyl acetate/heptane as eluent, afforded the title compound has a white solid (846 mg, 80%); Rf 0.30 (10% methanol in dichloromethane) 1H NMR (MeOH-d4, 300 MHz): δ 8.42 (1H, s), 8.36 (1H, d, J = 3 Hz), 7.84 (2H, d, J = 8 Hz), 7.76 (3H, m), 7.42 (2H, d, J = 8 Hz), 7.35 (1H, dd, J = 8, 3 Hz), 7.20 (5H, m), 4.82 (2H, s), 3.42 (1H, m), 3.20 (1H, m), 3.02 (2H, m), 2.92 (2H, m) ; ESI-LCMS e/z calcd for C₃₀H₂₄ClF₃N₂O₆S: 633.041, found 633 [M+H(³⁵Cl)]⁺, 635 [M+H(³⁷Cl)]⁺.

### Example 5 (not according to the invention)

### 4-{4-[(4-tert-Butylbenzyl)-(3,4-dichlorobenzenesulfonyl)-amino]-phenyl}-4-oxo-2-(3-trifluoromethylbenzyl)-butyric acid.

### Step 1: 2-(3-Trifluoromethylbenzyl)-malonic acid diallyl ester

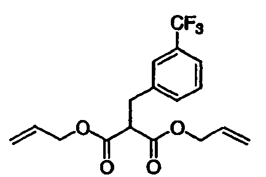

2-(3-Trifluoromethylbenzyl)-malonic acid diallyl ester was prepared in analogous fashion to 2-Pyridin-3-ylmethyl-malonic acid diallyl ester, using malonic acid diallyl ester (4.5 g, 24.5 mmol), sodium hydride (95%, 680 mg, 27 mmol) and 3-trifluoromethylbenzyl bromide (6.45 g, 27 mmol), to yield the title compound as a colorless oil (6.87 g, 82%), 1H NMR (CDCl₃, 300 MHz): δ 7.56 (2H, d, J = 8 Hz), 7.32 (2H, d, J = 8 Hz), 5.82 (2H, m), 5.24 (4H, m), 4.59 (4H, m), 3.72 (1H, t, J = 7 Hz), 3.31 (2H, d, J = 7 Hz).

### Step 2: 2-(2-{4-[(4-tert-Butylbenzyl)-(3,4-dichlobenzenesulfonyl)-aimno]phenyl}-2-oxoethyl)-2-(3-trifluoromethylbenzyl)-malonic acid diallyl ester

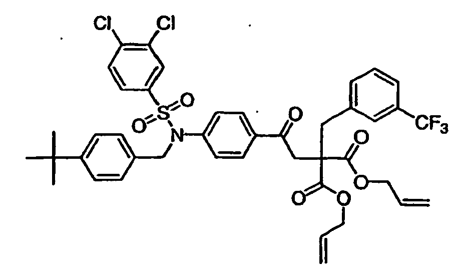

2-(2-{4-[(4-*tert*-Butylbenzyl)-(3,4-dichlobenzenesulfonyl)-amino]phenyl}-2-oxoethyl)-2-(3-trifluoromethylbenzyl)-malonic acid diallyl ester was synthesized in similar fashion to that reported previously using *N*-{4-(2-bromoacetyl)phenyl]-3,4-dichlorobenzene-sulfonamide as the second step alkylating reagent, to afford the *N*-alkylated product 2-{2-[4-(3,4-dichlorobenzenesulfonylamino)-phenyl]-2-oxoethyl}-2-(3-trifluoromethyl-benzyl}-malonic acid diallyl ester.

N-Alkylation of this intermediate with 4-*tert*-butylbenzyl bromide, under the conditions reported previously, afforded the N,N-dialkylated product. 1H NMR (CDCl₃, 300 MHz): δ 7.73 (1H, d, J = 8 Hz), 7.69 (1H, d, J = 2 Hz), 7.56 (2H, m), 7.47 (1H, m), 7.39 (1H, m), 7.32 (2H, m), 7.26 (4H, m), 7.10 (3H, m), 5.88 (2H, m), 5.26 (4H, m), 4.72 (2H, s), 4.64 (4H, m), 3.58 (2H, s), 3.42 (2H, s), 1.26 (9H, s).

### Step 3: 4-{4-[(4-tert-Butylbenzyl)-(3,4-dichlorobenzenesulfonyl)-amino]-phenyl}-4-oxo-2-(3-trifluoromethylbenzyl)-butyric acid.

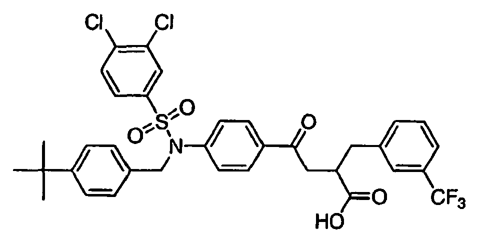

The title compound was prepared by saponification and decarboxylation of 2-(2-{4-[(4-tert-butylbenzyl)-(3,4-dichlobenzenesulfonyl)-amino]phenyl}-2-oxoethyl)-2-(3-trifluoromethylbenzyl)-malonic acid diallyl ester. Purification of the product by flash column chromatography, using 5 % methanol in dichloromethane as eluent, afforded the title compound has a beige solid; Rf 0.62 (10% methanol in dichloromethane): 1H NMR (CDCl₃, 300 MHz): δ 7.77 (1H, d, J = 8 Hz), 7.66 (2H, m), 7.28 - 7.54 (8H, m), 7.24 (2H, d, J = 9 Hz), 7.10 (2H, d, J = 9 Hz), 4.71 (2H, s), 3.16 - 3.38 (3H, m), 2.93 (2H, m), 1.25 (9H, s); ESI-LCMS e/z calcd for C₃₅H₃₂Cl₂F₃NO₅S: 706.606, found 706 (M+H, ³⁵Cl, ³⁵Cl)⁺.

### Example 6 (not according to the invention)

### 4-{4-[(3,4-dichlorobenzenesulfonyl)-(4-isopropylbenzyl)-amino]-phenyl}-4-oxo-2-(3-trifluoromethylbenzyl)-butyric acid.

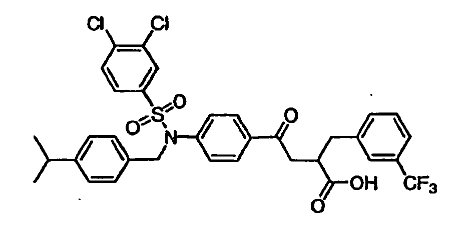

### Step 1: 2-(2-{4-[(3,4-Dichlorobenzenesulfonyl-(4-isopropylbenzyl)-amino]-phenyl-2-oxoethyl)-2-(3-trifluoromethylbenzyl)-malonic acid diallyl ester

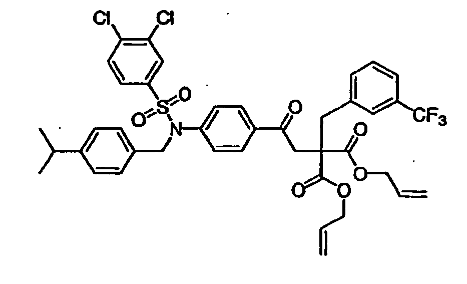

2-(2-(4-[(3,4-Dichlorobenzenesulfonyl-(4-isopropylbenzyl)-amino]-phenyl-2-oxoethyl)-2-(3-trifluoromethylbenzyl)-malonic acid diallyl ester was synthesized i via alkylation of 2-(3-trifluoromethylbenzyl)-malonic acid diallyl ester with *N*-{4-(2-bromoacetyl)-phenyl]-3,4-dichlorobenzene-sulfonamide, with subsequent *N*-alkylation of this intermediate with 4-isopropylbenzyl bromide to afford the N,N-dialkylated product. 1H NMR (CDCl₃, 300 MHz): δ 7.73 (1H, d, J = 8 Hz), 7.69 (1H, s), 7.56 (2H, m), 7.47 (1H, m), 7.36 (3H, m), 7.26 (4H, m), 7.10 (3H, m), 5.89 (2H, m), 5.30 (4H, m), 4.71 (2H, s), 4.66 (4H, m), 3.56 (2H, s), 3.43 (2H, s), 2.83 (1H, sept, J = 7 Hz), 1.20 (3H, s), 1.18 (3H, s).

### Step 2: 4-{4-[(3,4-dichlorobenzenesulfonyl)-(4-isopropylbenzyl)-amino]-phenyl}-4-oxo-2-(3-trifluoromethylbenzyl)-butyric acid.

The title compound prepared by saponification and decarboxylation of 2-(2-{4-[(3,4-dichlorobenzenesulfonyl-(4-isopropylbenzyl)-amino]-phenyl-2-oxoethyl)-2-(3-trifluoromethylbenzyl)-malonic acid diallyl ester. Purification of the product by flash column chromatography, using 5 % methanol in dichloromethane as eluent, afforded the title compound has a cream solid; Rf 0.60 (10% methanol in dichloromethane): 1H NMR (CDCl₃, 300 MHz): 8 7.76 (1H, d, J = 8 Hz), 7.70 (2H, m), 7.28 - 7.62 (8H, m), 7.24 (2H, m), 7.10 (2H, m), 4.70 (2H, s), 3.34 (1H, m), 3.20 (2H, m), 2.89 (2H, m), 2.81 (1H, sept, J = 7Hz), 1.19 (3H, s), 1.17 (3H, s); ESI-LCMS e/z calcd for C₃₄H₃₀Cl₂F₃NO₅S: 692.579, found 692 (M+H, ³⁵Cl, ³⁵Cl)⁺.

### Example 7 (compound indicated with "*" are not covered by the scope of the present invention)

The following compounds were prepared essentially according to the methods and procedures described above in the schemes and examples 1 to 6.

Still other compounds of the invention are the following, which were named using ChemDraw v. 6.02, which is sold by Cambridgesoft.com in Cambridge, MA.

| Cmpnd. No. | Name |
|---|---|
| 1 | N-{[4-({[4-(4-chlorophenyl)-5-(4-methylphenyl)-1,3-thiazol-2-yl]amino}carbonyl)phenyl]sulfonyl}phenylalanine; |
| 2 | N-({4-[3-(4-methoxyphenyl)-5-(4-pentylphenyl)-4,5-dihydro-1H-pyrazol-1-yl]phenyl}sulfonyl)-N-methylphenylalanine; |
| 3 | N-{[4-({[4-(4-chlorophenyl)-5-(4-methoxyphenyl)-1,3-thiazol-2-yl]amino)carbonyl)phenyl]sulfonyl}phenylalanine; |
| 4 | N-methyl-N-[(4-(5-(4-pentylphenyl)-3-[4-(trifluoromethoxy)phenyl]-4,5-dihydro-1H-pyrazol-1-yl)phenyl)sulfonyl]phenylalanine; |
| 5 | N-({4-[3-(4-methoxyphenyl)-5-(4-pentylphenyl)-1H-pyrazol-1-yl]phenyl}sulfonyl)-N-methylphenylalanine; |
| 6 | N-methyl-N-[(4-{5-(4-pentylphenyl)-3-[4-(trifluoromethoxy)phenyl]-1H-pyrazol-1-yl}phenyl)sulfonyl]phenylalanine; |
| 7 | N-({4-[5-(4-butoxyphenyl)-3-(4-methoxyphenyl)-1H-pyrazol-1-yl]phenyl}sulfonyl)-N-methylphenylalanine; |
| 8* | 2-benzyl-4-oxo-4-[3-({[4-(trifluoromethoxy)phenyl]sulfonyl}amino)phenyl]butanoic acid; |
| 9 | N-{[4-({[4-(3-chlorophenyl)-5-(4-methylphenyl)-1,3-thiazol-2-yl]amino}carbonyl)phenyl]sulfonyl}phenylalanine; |
| 10 | N-({4-[5-(4-isopropylphenyl)-3-(4-methoxyphenyl)-1H-pyrazol-1-yl]phenyl}sulfonyl)-N-methylphenylalanine: |
| 11 | N-{[4-({[4-(3-chloro-4-methylphenyl)-5-(4-methylphenyl)-1,3-thiazol-2-yl]amino}carbonyl)phenyl]sulfonyl}phenylalanine; |
| 12 | N-([4-({[4-(4-chlorophenyl)-5-(4-methylphenyl)-1,3-thiazol-2-yl]amino}carbonyl)phenyl]sulfonyl}-N-methylphenylalanine: |
| 13* | methyl (2S)-2-[4-((biphenyl-4-ylmethyl){[3-(trifluoromethyl)phenyl]sulfonyl}amino)phenoxy]-3-phenylpropanoate; |
| 14 | N-{[4-({[4-(4-bromophenyl)-5-(4-methylphenyl)-1,3-thiazol-2-yl]amino}carbonyl)phenyl]sulfonyl}phenylalanine: |
| 15 | N-{[4-({[4-(4-chlorophenyl)-5-(4-ethylphenyl)-1,3-thiazol-2-yl]amino}carbonyl)phenyl]sulfonyl}phenylalanine; |
| 16* | (2S)-2-[4-((biphenyl-4-ylmethyl){[3-(trifluoromethyl)phenyl]sulfonyl}amino)phehoxy]-3-phenylpropanoic acid; |
| 17 | N-[(4-{[4,6-bis(4-methoxyphenyl)pyrimidin-2-yl]amino}phenyl)sulfonyl]-N-methyl-L-phenylalanine; |
| 18* | N-methyl-N-({4-[5-(4-pentylphenyl)-3-(trifluoromethyl)-1H-pyrazol-1-yl]phenyl}sulfonyl)phenylalanine; |
| 19* | 2-benzyl-4-[4-({[2-nitro-4-(trifluoromethyl)phenyl]sulfonyl}amino)phenyl]-4-oxobutanoic acid; |
| 20* | 2-[3-[(4-butylphenyl)aminol-4-({[4-(trifluoromethoxy)phenyl]sulfonyl}amino)phenoxy]-3-phenylpropanoic acid; |
| 21* | 2-[3-[(4-butylphenyl)amino]-4-({[3-(trifluoromethyl)phenyl]sulfonyl}amino)phenoxy]-3-phenylpropanoic acid; |
| 22* | (2S)-2-[3-((biphenyl-4-ylmethyl){[4-(trifluoromethoxy)phenyl]sulfonyl}amino)phenoxy]-3-phenylpropanoic acid; |
| 23* | 2-{4-{[(4-bromophenyl)sulfonyl]amino}-3-[{4-butylphenyl)amino]phenoxy}-3-phenylpropanoic acid; |
| 24 | N-({4-[2-[(4-chlorobenzoyl)amino]-5-(4-ethylphenyl)-1,3-thiazol-4-yl]phenyl}sulfonyl)-N-methylphenylalanine |
| 25* | (2S)-2-[4-((2-naphthylmethyl){[3-(trifluoromethyl)phenyl]sulfonyl}amino)phenoxy]-3-phenylpropanoic acid; |
| 26 | N-[(4-{4-bromo-3-(4-methoxyphenyl)-5-[4-(trifluoromethyl)phenyl]-1H-pyrazol-1-yl}phenyl)sulfonyl]-N-methylphenylalanine; |
| 27 | N-({4-[5-(4-bromophenyl)-3-(4-methoxyphenyl)-1H-pyrazol-1-yl]phenyl]sulfonyl)-N-methylphenylalanine; |
| 28* | 2-{4-[(4-bromobenzoyl)amino]-3-[(4-butylphenyl)amino]phenoxy}-3-phenylpropanoic acid; |
| 29* | N-({4-[(6-bromo-4-phenylquinazolin-2-yl) amino] phenyl} sulfonyl) -N-methylphenylalanine; |
| 30 | N-({4-[2-[(cyclopentylacetyl)amino]-5-(4-ethylphenyl) -1,3-thiazol-4-yl]phenyl}sulfonyl)-N-methyl-L-phenylalanine; |
| 31 | N-({4-[2-(4-chlorophenyl)-5-(4-ethylphenyl)-1,3-thiazol-4-yl]phenyl}sulfonyl)-N-methyl-L-phenylalanine; |
| 32 | N-({4-[5-(4-ethylphenyl)-2-(6-methoxypyridin-3-yl)-1,3-thiazol-4-yl]phenyl}sulfonyl)-N-methyl-L-phenylalanine; |
| 33* | 2-(3-[(4-butylphenyl)amino]-4-{[(4-chloro-3-nitrophenyl)sulfonyl]amino}phenoxy)-3-phenylpropanoic acid; |
| 34 | N-[(4-{[4-(4-chlorophenyl)-5-(4-methylphenyl)-1,3-thiazol-2-yl]amino}phenyl)sulfonyl]-N-methyl-L-phenylalanine; |
| 35* | 2-[3-[(4-butylphenyl)amino]-4-({[5-(dimethylamino)-1-naphthyl]sulfonyl}amino)phenoxy]-3-phenylpropanoic acid; |
| 36* | 2-(3-[(4-butylphenyl)amino]-4-{[(5-chloro-3-methyl-1-benzothien-2-yl)sulfonyl]amino}phenoxy)-3-phenylpropanoic acid; |
| 37* | 2-benzyl-4-[3-((2-naphthylmethyl){[4-(trifluoromethoxy)phenyl]sulfonyl}amino)phenyl]-4-oxobutanoic acid; |
| 38 | N-[(4-{3-(4-chlorophenyl)-5-[4-(trifluoromethyl)phenyl]-1H-pyrazol-1-yl}phenyl)sulfonyl]-N-methylphenylalanine; |
| 39 | N-({4-[3-(4-chlorophenyl)-5-(4-ethylphenyl)-1H-pyrazol-1-yl]phenyl}sulfonyl)-N-methylphenylalanine; |
| 40 | N-[(4-{4-bromo-3-(4-chlorophenyl)-5-[4-(trifluoromethyl)phenyl]-1H-pyrazol-1-yl}phenyl)sulfonyl]-N-methylphenylalanine; |
| 41 | N-((4-(4-bronto-3-(4-chlorophenyl)-5-(4-ethylphenyl)-1H-pyrazol-1-yl]phenyl)sulfonyl)-N-methylphenylalanine; |
| 42 | N-({4-[5-(4-bromophenyl)-3-(4-chlorophenyl)-1H-pyrazol-1-yl]phenyl}sulfonyl)-N-methylphenylalanine; |
| 43 | N-({4-[3-(4-chlorophenyl)-5-(4-pentylphenyl)-1H-pyrazol-1-yl]phenyl}sulfonyl)-N-methylphenylalanine; |
| 44 | N-({4-[4-bromo-3-(4-chlorophenyl)-5-(4-pentylphenyl)-1H-pyrazol-1-yl]phenyl}sulfonyl)-N-methylphenylalanine; |
| 45* | 2-{4-{[(4-bromo-3-fluorophenyl)sulfonyl]amino}-3-[(4-butylphenyl)amino]phenoxy}-3-phenylpropanoic acid; |
| 46* | 2-{4-({[4-bromo-3-(trifluoromethyl)phenyl]sulfonyl}amino)-3-[(4-butylphenyl)amino]phenoxy}-3-phenylpropanoic acid; |
| 47* | 2-benzyl-4-[3-((biphenyl-4-ylmethyl){[4-(trifluoromethoxy)phenyl]sulfonyl}amino)phenyl]-4-oxobutanoic acid; |
| 48* | 2-{4-({[4-bromo-2-(trifluoromethoxy)phenyl]sulfonyl}amino)-3-[(4-butylphenyl)amino]phenoxy}-3-phenylpropanoic acid; |
| 49* | 2-(3-[(4-butylphenyl)amino]-4-{[(3,4-dichlorophenyl) sulfonyl] amino}phenoxy)-3-phenylpropanoic acid; |
| 50* | diallyl {2-oxo-2-[4-({[4-(trifluoromethoxy)phenyl]sulfonyl}amino)phenyl]ethyl} [4-(trifluoromethyl)benzyl]malonate; |
| 51* | N-({4-[(6-isopropyl-4-phenylquinazolin-2-yl)amino]phenyl}sulfonyl)-N-methylphenylalanine; |
| 52 | N-({4-[5-(4-chlorophenyl)-2-(4-ethylphenyl)-1,3-thiazol-4-yl]phenyl}sulfonyl)-N-ethyl-L-phenylalanine; |
| 53 | N-({4-[5-(4-chlorophenyl)-2-(4-ethylphenyl)-1,3-thiazol-4-yl]phenyl}sulfonyl)phenylalanine; |
| 54 | N-({4-[2,5-bis(4-ethylphenyl)-1,3-thiazol-4-yl]phenyl}sulfonyl)phenylalanine; |
| 55* | 2-(3-[(4-butylphenyl)amino]-4-{[(3,4-dibromophenyl)sulfonyl]amino}phenoxy)-3-phenylpropanoic acid; |
| 56* | 2-benzyl-4-(4-{[4-chloro-3-(trifluoromethyl)benzyl][(3,4-dichlorophenyl)sulfonyl]amino}phenyl)-4-oxobutanoic acid; |
| 57* | methyl2-benzyl-4-(3-{(biphenyl-4-ylmethyl)[(3,4-dichlorophenyl)sulfonyl]amino}phenyl)-4-oxobutanoate; |
| 58* | methyl 2-benzyl-4-(3-{(3,4-dichlorobenzyl)[(3,4-dichlorophenyl)sulfonyl]amino}phenyl)-4-oxobutanoate; |
| 59* | methyl 2-benzyl-4-{3-[[4-chloro-3-(trifluoromethyl)benzyl] (2-naphthylsulfonyl)amino]phenyl)-4-oxobutanoate; |
| 60* | methyl 2-benzyl-4-{3-[(biphenyl-4-ylmethyl) (2-naphthylsulfonyl)amino]phenyl}-4-oxobutanoate; |
| 61* | 2-benzyl-4-{3-[(biphenyl-4-ylmethyl) (2-naphthylsulfonyl)amino]phenyl}-4-oxobutanoic acid; |
| 62* | 2-(3-[(4-bromophenyl)amino]-4-{[(4-butylphenyl)sulfonyl]amino}phenoxy)-3-phenylpropanoic acid; |
| 63* | methyl 2-benzyl-4-{3-[(2-naphthylmethyl) (2-naphthylsulfonyl)amino]phenyl}-4-oxobutanoate; |
| 64* | 2-benzyl-4-{3-[(2-naphthylmethyl) (2-naphthylsulfonyl)amino]phenyl}-4-oxobutanoic acid; |
| 65* | 4-{3-[(2-anthrylsulfonyl)(2-naphthylmethyl)amino]phenyl}-2-benzyl-4-oxobutanoic acid; |
| 66* | methyl 2-benzyl-4-{3-[{[4-(dimethylamino)-3-fluorophenyl]sulfonyl}(2-naphthylmethyl)amino]phenyl}-4-oxobutanoate; |
| 67* | methyl 2-benzyl-4-[3-([4-chloro-3-(trifluoromethyl)benzyl]{[4-(dimethylamino)-3-(trifluoromethyl)phanyl]sulfonyl}amino)phenyl]-4-oxobutanoate; |
| 68* | methyl 2-benzyl-4-{3-[{[4-(dimethylamino)-3-(trifluoromethyl)phenyl]sulfonyl} (2-naphthylmethyl)amino]phenyl}-4-oxobutanoate; |
| 69* | 2-benzyl-4-[3-([4-chloro-3-(trifluoromethyl)benzyl]{[4-(dimethylamino)-3-(trifluoromethyl)phenyl]sulfonyl}amino)phenyl]-4-oxobutanoic acid; |
| 70* | methyl 2-benzyl-4-(3-{[4-chloro-3-(trifluoromethyl)benzyl] [(3,4-difluorophenyl)sulfonyl]amino}phenyl)-4-oxobutanoate; or |
| 71* | methyl 2-benzyl-4-[3-([4-chloro-3-(trifluoromethyl)benzyl]{[4-(dimethylamino)-3-fluorophenyl]sulfonyl}amino)phenyl]-4-oxobutanoate. |

Yet still other compounds of the invention are the following, which were named using ChemDraw v. 6.02, which is sold by Cambridgesoft.com in Cambridge, MA.

| Ex. No. | Name |
|---|---|
| 72* | (2S)-2-[4-([4-(methoxycarbonyl)benzyl]{[4-(trifluoromethoxy)phenyl]sulfonyl}amino)phenoxy]-3-phenylpropanoic acid; |
| 73* | 2-benzyl-4-oxo-4-[4-({[4-(trifluoromethoxy)phenyl]sulfonyl}amino)phenyl] butanoic acid; |
| 74 * | 2-[3-[(4-butylphenyl)amino]-4-({[2-nitro-4-(trifluoromethyl)phenyl]sulfonyl}amino)phenoxy]-3-phenylpropanoic acid; |
| 75* | N-{[4-[(4-butylphenyl)amino]-3-({[3-(trifluoromethyl)phenyl]sulfonyl}amino)phenyl] sulfonyl}-N-methyl-L-phenylalanine; |
| 76* | benzyl (2S)-2-[4-([(5-nitro-2-furyl)methyl]{[3-(trifluoromethyl)phenyl]sulfonyl}amino)phenoxy]-3-phenylpropanoate; |
| 77* | (2R)-2-[4-({[4-chloro-2-(trifluoromethyl)quinolin-5-yl]methyl}{[3-(trifluoromethyl)phenyl]sulfonyl}amino)phenoxy]-3-phenylpropanoic acid; |
| 78* | 2-(4-[(4-butylphenyl)amino3-{[4-(trifluoromethoxy)benzoyl]amino}phenoxy)-3-phenylpropanoic acid; |
| 79* | 2-(3-[(4-butylphenyl)amino]-4-{[(4-chlorophenyl)sulfonyl]amino}phenoxy)-3-phenylpropanoic acid; |
| 80* | N-({4-[(6-bromo-4-phenylquinazolin-2-yl) (carboxymethyl)amino]phenyl}sulfonyl)-N-methylphenylalanine; |
| 81* | 2- (3-[(4-butylphenyl)amino]-4-{[(3-cyano-4-fluorophenyl)sulfonyl]amino}phenoxy)-3-phenylpropanoic acid; |
| 82* | 4-[4-((4-chlorobenzyl){[4-(trifluoromethoxy)phenyl]sulfonyl}amino)phenyl]-4-oxo-2-(pyridin-3-ylmethyl)butanoic acid; |
| 83* | 2-benzyl-4-[4-((biphenyl-4-ylmethyl){[4-(trifluoromethoxy)phenyl]sulfonyl}amino)phenyl]-4-oxobutanoic acid; |
| 84* | 2-benzyl-4-{4-[([4-methoxy-3-(trifluoromethyl)phenyl]sulfonyl}(1-naphthylmethyl)amino]phenyl}-4-oxobutanoic acid; |
| 85* | 2-benzyl-4-(4-{[(3,4-dichlorophenyl)sulfonyl][4-(trifluoromethoxy)benzyl]amino}phenyl)-4-oxobutanoic acid; |
| 86* | 2-benzyl-4-(4-{[4-chloro-3-(trifluoromethyl)benzyl][(3-fluoro-4-methoxyphenyl)sulfonyl]amino}phenyl)-4-oxobutanoic acid; |
| 87* | methyl 2-benzyl-4-{3-[[(3,4-dichlorophenyl)sulfonyl](2-naphthylmethyl)amino]phenyl}-4-oxobutanoate; |
| 88* | methyl 2-benzyl-4-(3-{[4-chloro-3-(trifluoromethyl)benzyl][(3,4-dichlorophenyl)sulfonyl]amino)phenyl)-4-oxobutanoate; |
| 89* | 2-benzyl-4-(3-{[4-chloro-3-(trifluoromethyl)benzyl][(3,4-dichlorophenyl)sulfonyl]amino}phenyl)-4-oxobutanoic acid; |
| 90* | 2-benzyl-4-(3-{(biphenyl-4-ylmethyl)[(3,4-dichlorophenyl)sulfonyl]amino}phenyl)-4-oxobutanoic acid; |
| 91* | methyl 4-(3-{(4-benzoylbenzyl)[(3,4-dichlorophenyl)sulfonyl]amino}phenyl)-2-benzyl-4-oxobutanoate; |
| 92* | 2-benzyl-4-{3-[[(3,4-dichlorophenyl)sulfonyl](4-isopropylbenzyl)amino]phenyl}-4-oxobutanoic acid; |
| 93* | 4-(4-dibenzo[b,d]furan-4-ylphenyl)-4-oxo-2-[3-(trifluoromethyl)benzyl]butanoic acid; |
| 94* | 2-benzyl-4-{3-[{[4-methoxy-3-(trifluoromethyl)phenyl]sulfonyl}(2-naphthylmethyl)amino]phenyl}-4-oxobutanoic acid; |
| 95* | methyl 2-benzyl-4-{3-[[(3,4-difluorophenyl)sulfonyl](2-naphthylmethyl)amino]phenyl}-4-oxobutanoate; |
| 96* | N-{[4-(2-bromo-5-dibenzo[b,d]furan-4-yl-1,3-thiazol-4-yl)phenyl]sulfonyl}phenylalanine; |
| 97* | N-([4-(5-bromo-2-dibenzo[b,d]furan-4-yl-1,3-thiazol-4-yl)phenyl]sulfonyl)phenylalanine; |
| 98* | 2-[4-(S-Bromo-2-dibenzofuran-4-yl-thiazol-4-yl)-benzenesulfonylamino]-3-phenyl-propionic acid |
| 99* | 2-[4-(2-Dibenzofuran-4-yl-thiazol-4-yl)-benzenesulfonylamino]-3-phenyl-propionic acid |
| 100* | (4-{*2-[(8-Chloro-dibenzofuran-4-carbonyl)-amino]-5-ethyl-thiazol-4-yl}-phenoxy)-phenyl-acetic acid |
| 101* | [4-(2-Benzo[b]thiophen-3-yl-5-ethyl-thiazol-4-yl)-phenoxy]-phenyl-acetic acid |
| 102* | [4-(2-Dibenzofuran-4-yl-5-ethyl-thiazol-4-yl)-phenoxy]-phenyl-acetic acid. |

Still other compounds of the invention are

| | |
|---|---|
| 2-{4-[4-(4-Chloro-phenyl)-5-p-tolyl-thiazol-2-ylcarbamoyl]-benzenesulfonylamino} -3-phenyl-propionic acid;* | R_{f} 0.66 (20% methanol in dichloromethane) ¹H NMR (CD₃OD, 300 MHz) δ 8.03 (d, J = 8.4 Hz, 2 H), 7.79 (d, J = 8.4 Hz, 2 H), 7.49 (d, J = 8.4 Hz, 2 H), 7.26 (d, J = 8.4 Hz, 2 H), 7.24-7.15 (m, 9 H), 4.13-4.05 (m, 1 H), 3.12-3.06 (m, 1 H), 2.90-2.82 (m, 1 H), 2.38 (s, 3 H) ; ESI-LCMS *m*/*z* calcd for C₃₂H₂₆ClN₃O₅S₃: 631; found 630 (M - 1)⁺. |
| 2-{4-[4-(3-Chloro-phenyl)-5-p-tolyl-thiazol-2-ylcarbamoyl]-benzenesulfonylamino} -3-phenyl-propionic acid;*. | R_{f} 0.63 (20% methanol in dichloromethane), ¹H NMR (CD₃OD, 300 MHz) δ 8.03 (d, J = 8.4 Hz, 2 H), 7.77 (d, J = 8.4 Hz, 2 H), 7.58 (br s, 1 H), 7.40-7.37 (m, 1 H), 7.27-7.12 (m, 11 H), 4.11 (dd, J1 = 9.0 Hz, J2 = 5.4 Hz, 1 H), 3.09 (dd (J1 = 13.8 Hz, J2 = 5.4 Hz, 1 H), 2.85 (dd, J1 = 13.8 Hz, J2 = 9.9 Hz, 1 H), 2.39 (s, 3 H); ESI-LCMS *m*/*z* calcd for C₃₂H₂₆ClN₃O₅S₃ : 631; found 630 (M - 1)⁺. |
| 2-{4-[4-(2-Chloro-phenyl)-5-p-tolyl-thiazol-2-ylcarbamoyl]-benzenesulfonylamino} -3-phenyl-propionic acid;* | R_{f} 0.64 (20% methanol in dichloromethane), ¹H NMR (CD₃OD, 300 MHz) δ 8.03 (d (J = 8.4 Hz, 2 H), 7.76 (d, J = 8.4 Hz, 2 H), 7.47-7.23 (m, 2 H), 7.18-7.04 (m, 11 H), 4.11 (dd, J1 = 8.7 Hz, J2 = 5.1 Hz, 1 H), 3.09 (dd, J1 = 13.8 Hz, J2 = 4.8 Hz, 1 H), 2.85 (dd, J1 = 13.8 Hz, J2 = 9.3 Hz, 1 H), 2.30 (s, 3 H); ESI-LCMS *m*/*z* calcd for C₃₂H₂₆ClN₃O₅S₃: 631; found 630 (M - 1)⁺. |
| 2-((4-[4-(4-Chloro-phenyl)-5-p-tolyl-thiazol-2-ylcarbamoyl]-benzenesulfonyl)-methyl-amino)-3-phenyl-propionic acid;* | R_{f} 0.67 (20% methanol in dichloromethane), ¹H NMR (CD₃OD, 300 MHz) δ 8.02 (d, J = 8.4 Hz, 2 H), 7.63 (d, J = 8.7 Hz, 2 H), 7.49 (d J = 8.4 Hz, 2 H), 7.26 (d, J = 8.4 Hz, 2 H), 7.23-7.19 (m, 9 H), 4.94 (dd, J1 = 10.5 Hz, J2 = 5.4 Hz, 1 H), 3.30-3.23 (m, 1 H), 2.95 (dd, J1 = 14.4 Hz, J2 = 10.5 Hz, 1 H), 2.93 (s, 3 H), 2.38 (s, 3 H); ESI-LCMS *m*/*z* calcd for C₃₃H₂₈ClN₃O₅S₃: 645; found 646 (M + 1)*. |
| 2-({4-[2-(2-Cyclopentyl-acetylamino)-5-(4-ethyl-phenyl)-thiazol-4-yl]-benzenesulfonyl}-methyl-amino)-3-phenyl-propionic acid; | R_{f} 0.82 (20% methanol in dichloromethane), ¹H NMR (CD₃OD, 300 MHz) δ 7.5 (d, J = 8.4 Hz, 2 H), 7.26-7.17 (m, 11 H), 3.32-3.25 (m, 1 H), 2.85 (dd, J1 = 14.4 Hz, J2 = 10.5 Hz, 1 H), 2.83 (s, 3 H), 2.71 (q, J = 7.5 Hz, 2 H), 2.50 (d, J = 7.5 Hz, 2 H), 2.37-2.76 (m, 1 H), 1.90-1.83 (m, 3 H), 1.74-1.57 (m, 6 H), 1.30-1.26 (m, 3 H); ESI-LCMS *m*/*z* calcd for C₃₄H₃₇ClN₃O₅S₂: 631; found 632 (M + 1)⁺. |
| 2-({4-[2-(4-Chloro-benzoylamino)-5-(4-ethyl-phenyl)-thiazol-4-yl]-benzenesulfonyl}-methyl-amino)-3-phenyl-propionic acid; | R_{f} 0.60 (20% methanol in ethyl acetate), ¹H NMR (DMSO-d₆, 300 MHz) δ 8.02 (d, J = 8.7 Hz, 2 H), 7.55 (dd, J1 = 8.1 Hz, J2 = 6.6 Hz, 4 H), 7.293-7.10 (m, 11 H), 3.66 (s, 1 H), 3.30-3.25 (m, 1H), 2.93-2.84 (m, 1 H), 2.72 (q, J = 7.5 Hz, 2 H), 1.28 (t, J = 7.5 Hz, 3 H); ESI-LCMS *m*/*z* calcd for C₃₄H₃₀ClN₃O₅S₂: 659; found 660 (M + 1)⁺. |
| 2-({4-[4-(4-Chloro-phenyl)-5-p-tolyl-thiazol-2-ylamino]-benzenesulfonyl}-methyl-amino)-3-phenyl-propionic acid;* | R_{f} 0.67 (20% methanol in ethyl acetate), ¹H NMR (CDCl₃, 300 MHz) δ 7.28 (d, J = 9.3 Hz, 2 H), 7.51 (d, J = 9.3 Hz, 2 H), 7.35 (d, J = 8.7 Hz, 2 H), 7.31-7.15 (m, 11 H), 3.29-3.25 (m, 1 H), 2.95-2.85 (m, 2 H), 2.85 (s, 3 H), 2.36 (s, 3 H); ESI-LCMS m/z calcd for C₃₂H₂₈ClN₃O₄S₂: 617; found 618 (M + 1)⁺. |
| 2-({4-[5-(4-Chloro-phenyl)-2-(4-ethyl-phenyl)-thiazol-4-yl]-benzenesulfonyl}-ethyl-amino)-3-phenyl-propionic acid; | R_{f} 0.79 (20% methanol in ethyl acetate), ¹H NMR (CDCl₃, 300 MHz) δ 7.90-7.86 (m, 2 H), 7.63-7.53 (m, 6 H), 7.35-7.12 (m, 9 H), 4.79 (dd, J1 = 8.4 Hz, J2 = 6.6 Hz, 1 H), 3.41-3.28 (m, 3 H), 2.91 (dd, J1 = 14.4 Hz, J2 = 8.7 Hz, 2 H), 2.74-2.64 (m, 2 H), 1.29-1.21 (m, 6 H); ESI-LCMS *m*/*z* calcd for C₃₄H₃₁ClN₂O₄S₂: 630; found 631 (M + 1)⁺. |
| 2-{4-[5-(4-Chloro-phenyl)-2-(4-ethyl-phenyl)-thiazol-4-yl]-benzenesulfonylamino} -3-phenyl-propionic acid; or | R_{f} 0.89 (20% methanol in ethyl acetate), ¹H NMR (CDCl₃, 300 MHz) δ 7.93 (d, J = 8.4 Hz, 2 H), 7.64-7.56 (m, 4 H), 7.41-7.33 (m, 4 H), 7.27-7.14 (m, 7 H), 4.06 (dd, J1 = 8.3 Hz, J2 = 5.4 Hz, 1 H), 3.06 (dd, J1 = 13.5 Hz, J2 = 5.4 Hz, 1 H), 2.86 (dd, J1 = 13.5 Hz, J2 = 8.3 Hz, 1 H), 2.73 (q, J = 7.4 Hz, 2 H), 1.29 (t, J = 7.4 Hz, 3 H); ESI-LCMS *m*/*z* calcd for C₃₂H₂₇ClN₂O₄S₂: 602; found 603 (M + 1)⁺. |
| 2-({4-[2-(4-Chloro-phenyl)-5-(6-methoxy-pyridin-3-yl)-thiazol-4-yl]-benzenesulfonyl}-ethyl-amino)-3-phenyl-propionic acid; | R_{f} 0.86 (20% methanol in dichloromethane) |

or pharmaceutically acceptable salts thereof.

### Example 8

### Method for measuring PTP-1B activity

The test compounds are evaluated for their in vitro inhibitory activity against recombinant human PTP1B with phosphotyrosyl dodecapeptide TRDI(P)YETD(P)Y(P)YRK [SEQ ID NO:1]. This corresponds to the 1142-1153 insulin receptor kinase regulatory domain, phosphorylated on the 1146, 1150 and 1151 tyrosine residues; IR-triphosphopeptide as a source of substrate. Enzyme reaction progression is monitored via the release of inorganic phosphate as detected by the malachite green - ammonium molybdate method for the phosphopeptide.

Preferred compounds of the invention exhibit IC₅₀ values of less than 10 µM; more preferred compounds of the invention exhibit IC₅₀ values of less than 1 µM. Particularly preferred compounds exhibit IC₅₀ values of less than 300 nM.

The invention and the manner and process of making and using it, are now described in such full, clear, concise and exact terms as to enable any person skilled in the art to which it pertains, to make and use the same. It is to be understood that the foregoing describes preferred embodiments of the invention.

To particularly point out and distinctly claim the subject matter regarded as invention, the following claims conclude this specification.

### Example 9

Male Wistar rats were fed a High Fat Diet for at least 4 weeks. Jugular vein and carotid artery cannulations were performed one week prior to the clamp experiment. Test compound (Compound 15) is administered p.o. 4 hrs before the clamp and labeled 3-3H-glucose is infused 1 hr prior to calculated endogenous glucose production (EGP). Insulin is infused at a rate of 0.75U/kg/hr raising plasma insulin levels to -200 mU/ml. To maintain euglycemia (80 mg/dl), unlabeled glucose is infused at a variable rate and adjusted every 10 minutes.

Treatment with Compound 15 (30mg/kg) significantly increased the glucose infusion rate (GIR). This effect reflects enhanced suppression of endogenous glucose production (EGP) and augmented stimulation of glucose utilization (GU).

The results of this study indicate that the test compound improves insulin action in insulin resistant rats *in vivo.* This improvement affects both hepatic glucose production as well as peripheral glucose utilization. The results demonstrate that compounds of the invention are useful as insulin potentiators for the treatment of insulin resistance in diabetes mellitus.

| Effects of a single oral dose of Compound 15 on insulin action in insulin resistant rats | | | | |
|---|---|---|---|---|
| Dose | N | N GIR | EGP | GU |
| 0 mg/kg | 6 | 19.4±2.5 | 4.0±1.3 | 15.5±3.6 |
| 10 mg/kg | 3 | 29.6±5.0 | 1.4±1.8 | 28.3±6.6 |
| 30 mg/kg | 5 | 30.8±2.2** | 0.16±0.5* | 30.7±2.00** |
| 60 mg/kg | 4 | 38.0±2.2** | 0.77±0.4 | 37.3±2.3** |

| | | | | |
|---|---|---|---|---|
| *P<0.05 ** P<0.01 vs. control; GIR, EGP and GU are mean ± sem in mg/kg/min | | | | |

The invention and the manner and process of making and using it, are now described in such full, clear, concise and exact terms as to enable any person skilled in the art to which it pertains, to make and use the same. It is to be understood that the foregoing describes preferred embodiments of the invention. To particularly point out and distinctly claim the subject matter regarded as invention, the following claims conclude this specification.

## Claims

1. A compound of the formula 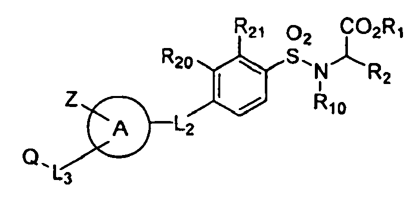 wherein,
L₂ is a bond or -C(O)NR₉-, -N(R₉)C(O)-, -(C₁-C₄)alkyl-C(O)NR₉-,-(C₁-C₄)alkyl-N(R₉)C(O)-, -C(O)N(R₉)-(C₁-C₄)alkyl-, -N(R₉)C(O) -(C₁-C₄)alkyl-, -N(R₉)SO₂-, -SO₂N(R₉)-, -N(R9)-, -N (R₉) - (C₁-C₄)alkyl-, -O-(C₁-C₄)alkyl-, -(C₁-C₄)alkyl-O-, or -(C₁-C₉)alkyl-N(R₉)-,
R₉ is H, C₁-C₆ alkyl, -SO₂ phenyl, phenyl(C₁-C₄)alkyl, wherein the phenyl group is optionally substituted with 1, 2, 3, or 4 groups that are independently C₁-C₄ alkyl, C₁-C₄ alkoxy, halogen, OH, NO₂, NH₂, NH (C₁-C₆) alkyl, N(C₁-C₆)alkyl(C₁-C₆)alkyl, C₁-C₂ haloalkyl, or C₁-C₂ haloalkoxy;
L₃ is a bond, -(C₁-C₄)alkyl-O-, -O-(C₁-C₄)alkyl, -(C₁-C₄) alkyl-, -C (O) -;
the A ring is thiazolyl, pyrazolyl, dihydropyrazolyl, benzofuranyl, imidazolyl, isothiazolyl, pyrrolyl, oxazolyl, pyrimidyl; or triazolyl, each of which is optionally substituted with 1, or 2 groups that are independently, halogen, C₁-C₆ alkyl, C₁-C₄ alkoxy, haloalkyl, haloalkoxy, NO₂, NH₂, NH(C₁-C₆)alkyl, N(C₁-C₆)alkyl(C₁-C₆)alkyl;
Q is phenyl, naphthyl, -phenyl-carbonyl-phenyl, -phenyl -(C₁-C₄)alkyl- phenyl, -phenyl-pyridyl, -phenyl-pyrimidyl, -phenyl-pyrrolyl, -phenyl-piperidinyl, -phenyl-pyrrolidinyl, -phenyl-piperazinyl, -phenyl-, pyridyl, pyrimidyl, furanyl, thienyl, pyrrolyl, imidazolyl,-pyridyl-(C₁-C₄)alkyl-phenyl, imidazolidinyl, dibenzofuranyl, tetrahydrofuranyl, tetrahydrothienyl, piperidinyl, pyrrolidinyl, piperazinyl, C₁-C₆ alkyl, halogen, C₁-C₄ haloalkoxy, C₁-C₄ haloalkyl, or C₁-C₆ alkoxycarbonyl,
wherein the aforementioned cyclic groups are optionally substituted with 1, 2, 3, 4, or 5 groups that are independently alkoxycarbonyl, C₁-C₆ alkyl, C₁-C₆ alkoxy, halogen, C₁-C₄ haloalkyl, C₁-C₄ haloalkoxy, NR₆R₇, or phenyl; wherein
R₆ and R₇ are independently H, C₁-C₆ alkyl, phenyl(C₁-C₆)alkyl, C₂-C₆ alkanoyl, phenyl(C₁-C₆)alkanoyl, C₁-C₆ alkoxycarbonyl, phenyl(C₁-C₆)alkoxycarbonyl, pyridylcarbonyl, or -SO₂-phenyl, wherein the cyclic groups are optionally substituted with 1, 2, 3, or 4 groups that are independently halogen, C₁-C₄ alkyl, C₁-C₄ alkoxy, NO₂ OH, NH₂, NH(C₁-C₆)alkyl,N(C₁-C₆)alkyl(C₁-C₆)alkyl, C₁-C₂ haloalkyl or C₁-C₂ haloalkoxy, and
Z is -NHC (O) phenyl, -NHC(O)naphthyl, -N(C₁-C₄alkyl)C(O)phenyl,-N(C₁-C₄alkyl)C(O)naphthyl, naphthyl, or phenyl, wherein the phenyl groups are optionally substituted with 1, 2, 3, 4, or 5 groups that are independently C₁-C₆ alkyl, C₁-C₆ alkoxy, halogen, C₁-C₂ haloalkyl, C₁-C₂ haloalkoxy, or NO₂ or
Z is -NHC(O)-(C₁-C₄)alkyl-(C₃-C₇)cycloalkyl, or -N(C₁-C₄)alkylC(O)-(C₁-C₄)alkyl-(C₃-C₇)cycloalkyl;
R₁ is H, C₁-C₄ alkyl, or benzyl;
R₂ is phenyl, phenyl(C₁-C₄) alkyl, -(C₁-C₄) alkyl-piperidinyl,-(C₁-C₄) alkyl-pyrrolidinyl, wherein the heterocycloalkyl group is optionally fused to a phenyl ring and wherein the heterocycloalkyl portion, the phenyl portion, or both are optionally substituted with a total of 1, 2, 3, or 4 groups that are independently halogen, C₁-C₄ alkyl, C₁-C₄ alkoxy, -SO₂-(C₁-C₄) alkyl, C₁-C₂ haloalkyl, or C₁-C₂ haloalkoxy;
R₁₀ is H, C₁-C₆ alkyl, wherein the alkyl group is optionally substituted with phenyl, which is optionally substituted with 1, 2, 3, or 4 groups that are independently C₁-C₆ alkyl, C₁-C₆ alkoxy, halogen, OH, NO₂ C₁-C₂ haloalkyl, or C₁-C₂ haloalkoxy; and
R₂₀, and R₂₁, are independently selected from H, benzyloxy, benzyl, halogen, C₁-C₄ alkyl, OH, C₁-C₄ alkoxy, NO₂, NH₂, NH(C₁-C₆)alkyl, N(C₁-C₆)alkyl(C₁-C₆)alkyl, NH-phenyl, N(C₁-C₄)alkyl-phenyl, NHbenzyl, or -N(C₁-C₆)alkylbenzyl, wherein the phenyl groups are optionally substituted with 1, 2, 3, or 4 groups that are independently C₁-C₆ alkyl, C₁-C₆ alkoxy, halogen, OH, NO₂, C₁-C₂ haloalkyl, or C₁-C₂ haloalkoxy.

2. The compound according to claim 1, wherein
L₂ is a bond or -C(O)NR₉-, -N(R₉)C(O)-, -(C₁-C₄)alkyl-C(O)NR₉-, -(C₁-C₄)alkyl-N(R₉)C(O)-, -N(R₉)SO₂-, -SO₂N(R₉)-,-N(R₉)-, -N(R₉)-(C₁-C₄)alkyl-, or -(C₁-C₄)alkyl-N(R₉)-,
R₉ is H, C₁-C₆ alkyl, -SO₂pheny benzyl, phenethyl, naphthyl-CH₂-, anthracenyl-CH₂-, wherein the phenyl group is optionally substituted with 1, 2, 3, or 4 groups that are independently C₁-C₄ alkyl, C₁-C₄ alkoxy, halogen, OH, NO₂, NH₂, NH(C₁-C₆)alkyl, N(C₁-C₆)alkyl(C₁-C₆)alkyl, C₁-C₂ haloalkyl, or C₁-C₂ haloalkoxy;
the A ring is thiazolyl, pyrazolyl, dihydropyrazolyl, benzofuranyl, imidazolyl, isothiazolyl, pyrrolyl, pyrimidyl, or oxazolyl, each of which is optionally substituted with 1, or 2 groups that are independently, halogen, C₁-C₆ alkyl, C₁-C₄ alkoxy, haloalkyl, haloalkoxy, NO₂, NH₂, NH(C₁-C₆)alkyl, N(C₁-C₆)alkyl(C₁-C₆)alkyl; and
Q is phenyl, naphthyl, -phenyl-carbonyl-phenyl, -phenyl-pyridyl, -phenyl-piperidinyl, -phenyl-pyrrolidinyl, pyridyl, pyrimidyl, furanyl, thienyl, piperidinyl, dibenzofuranyl, pyrrolidinyl, piperazinyl, C₁-C₆ alkyl, halogen, C₁-C₄ haloalkoxy, C₁-C₄ haloalkyl, or C₁-C₆ alkoxycarbonyl, wherein the aforementioned cyclic groups are optionally substituted with 1, 2, 3, 4, or 5 groups that are independently alkoxycarbonyl, C₁-C₆ alkyl, C₁-C₆ alkoxy, halogen, C₁-C₄ haloalkyl, C₁-C₄ haloalkoxy, or NR₆R₇ wherein
R₆ and R₇ are independently H, C₁-C₆ alkyl, phenyl(C₁-C₄)alkyl, C₂-C₆ alkanoyl, phenyl(C₁-C₄)alkanoyl, C₁-C₆ alkoxycarbonyl, phenyl(C₁-C₄)alkoxycarbonyl, pyridylcarbonyl, or -SO₂-phenyl, wherein the cyclic groups are optionally substituted with 1, 2, 3, or 4 groups that are independently halogen, C₁-C₄ alkyl, C₁ C₄ alkoxy, NO₂, OH, NH₂, NH(C₁-C₆)alkyl, N(C₁-C₆)alkyl(C₁-C₆)alkyl, CF₃, or OCF₃.

3. The compound according to claim 2, wherein
R₂ is phenyl or phenyl(C₁-C₄) alkyl wherein the phenyl portion, or both are optionally substituted with a total of 1, 2, 3, or 4 groups that are independently halogen, C₁-C₄ alkyl, C₁-C₄ alkoxy, -SO₂-(C₁-C₄) alkyl, CF₃, or OCF₃;
R₁₀ is H, C₁-C₄ alkyl, wherein the alkyl group is optionally substituted with phenyl, which is optionally substituted with 1, 2, 3, or 4 groups that are independently C₁-C₆ alkyl, C₁-C₆ alkoxy, halogen, OH, NO₂, C₁-C₂ haloalkyl, or C₁-C₂ haloalkoxy; and
R₂₀, and R₂₁, are independently selected from H, halogen, C₁-C₄ alkyl, OH, C₁-C₄ alkoxy, NO₂, NH₂, NH(C₁-C₆)alkyl, or N(C₁-C₆)alkyl(C₁-C₆)alkyl,
L₂ is a bond or -C(O)NR₉-, -N(R₉)C(O)-, -(C₁-C₄)alkyl-C(O)NR₉-,-(C₁-C₄)alkyl-N(R₉)C(O)-, -N(R₉)SO₂-, -SO₂N(R₉)-, -N(R₉)-, -N(R₉)-(C₁-C₉)alkyl-, or-(C₁-C₄)alkyl-N(R₉)-,
R₉ is H, C₁-C₆ alkyl, -SO₂phenyl, benzyl, phenethyl, wherein the phenyl group is optionally substituted with 1, 2, 3, or 4 groups that are independently C₁-C₄ alkyl, C₁-C₄ alkoxy, halogen, OH, NO₂, NH₂, NH(C₁-C₆)alkyl, N(C₁-C₆)alkyl(C₁-C₆)alkyl, CF₃, or OCF₃;
Q is phenyl, naphthyl, pyridyl, pyrimidyl, furanyl, thienyl, piperidinyl, pyrrolidinyl, piperazinyl, C₁-C₆ alkyl, halogen, C₁-C₂ haloalkoxy, C₁-C₂ haloalkyl, or C₁-C₆ alkoxycarbonyl, wherein the aforementioned cyclic groups are optionally substituted with 1, 2, 3, 4, or 5 groups that are independently alkoxycarbonyl, C₁-C₆ alkyl, C₁-C₆ alkoxy, halogen, C₁-C₄ haloalkyl, C₁-C₄ haloalkoxy, or NR₆R₇; wherein
R₆ and R₇ are independently H, C₁-C₆ alkyl, phenyl(C₁-C₄)alkyl, C₂-C₆ alkanoyl, phenyl(C₁-C₄)alkanoyl, wherein the phenyl groups are optionally substituted with 1, 2, 3, or 4 groups that are independently halogen, C₁-C₄ alkyl, C₁-C₄ alkoxy, NO₂, OH, NH₂, NH(C₁-C₆)alkyl, N(C₁-C₆)alkyl(C₁-C₆)alkyl, CF₃, or OCF₃, and
Z is -NHC(O)phenyl, -N(C₁-C₄ alkyl)C(O)phenyl, or phenyl, wherein the phenyl groups are optionally substituted with 1, 2, 3, 4, or 5 groups that are independently C₁-C₆ alkyl, C₁-C₆ alkoxy, halogen, C₁-C₂ haloalkyl, C₁-C₂ haloalkoxy, or NO₂, or
Z is -NHC(O)-(C₁-C₄)alkyl-(C₃-C₇)cycloalkyl, or -N(C₁-C₄)alkylc(O)-(C₁-C₄)alkyl-(C₃-C₇)cycloalkyl.

4. The compound according to claim 3, wherein
R₁ is H, or C₁-C₄ alkyl;
R₂ is phenyl, phenyl(C₁-C₉) alkyl, wherein the phenyl portion, or both are optionally substituted with a total of 1, 2, 3, or 4 groups that are independently halogen, C₁-C₄ alkyl, C₁-C₄ alkoxy, or -SO₂-(C₁-C₄) alkyl;
R₁₀ is H, C₁-C₄ alkyl, wherein the alkyl group is optionally substituted with phenyl, which is optionally substituted with 1, 2, 3, or 4 groups that are independently C₁-C₆ alkyl, C₁-C₆ alkoxy, halogen, OH, NO₂, CF₃, or OCF₃; and
at least one of R₂₀ and R₂₁, is H, while the other is H, halogen, C₁-C₄ alkyl, OH, C₁-C₄ alkoxy, NO₂, NH₂, NH(C₁-C₆)alkyl, or N(C₁-C₆)alkyl(C₁-C₆)alkyl;
L₂ is a bond or -C(O)NR₉-, -N(R₉)C(O)-, -N(R₉)SO₂-, -SO₂N(R₉)-,-N(R₉)-, -N(R₉)-(C₁-C₄)alkyl-, or -(C₁-C₄)alkyl-N(R₉)-; and
Q is phenyl, naphthyl, pyridyl, pyrimidyl, furanyl, thienyl, piperidinyl, pyrrolidinyl, or piperazinyl each of which is optionally substituted with 1, 2, 3, 4, or 5 groups that are independently alkoxycarbonyl, C₁-C₆ alkyl, C₁-C₆ alkoxy, halogen, CF₃, OCF₃, or NR₆R₇.

5. The compound according to claim 4, wherein
L₂ is a bond or -NR₉-; wherein
R₉ is H, C₁-C₆ alkyl, or benzyl;
R₂ is phenyl, benzyl, or phenethyl, wherein the phenyl portion is optionally substituted with a total of 1, 2, 3, or 4 groups that are independently halogen, C₁-C₄ alkyl, C₁-C₄ alkoxy, or -SO₂-(C₁-C₄) alkyl;
Q is phenyl, or pyridyl, each of which is optionally substituted with 1, 2, 3, 4, or 5 groups that are independently C₁-C₆ alkoxycarbonyl, C₁-C₆ alkyl, C₁-C₆ alkoxy, halogen, CF₃, OCF₃, or NR₆R₇; and
Z is phenyl, which is optionally substituted with 1, 2, 3, 4, or 5 groups that are independently C₁-C₆ alkyl, C₁-C₆ alkoxy, halogen, C₁-C₂ haloalkyl, C₁-C₂ haloalkoxy, or NO₂.

6. The compound according to claim 5, wherein
the A ring is pyrazolyl, dihydropyrazolyl, thiazolyl, or pyrimidyl each of which is optionally substituted with 1, or 2 groups that are independently, halogen, C₁-C₆ alkyl, C₁-C₄ alkoxy, haloalkyl, haloalkoxy, NO₂, NH₂, NH (C₁-C₆) alkyl, N(C₁-C₆)alkyl(C₁-C₆)alkyl.

7. The compound according to claim 6, wherein R₁₀ is H or C₁-C₄ alkyl; and L₃ is a bond or -(C₁-C₄) alkyl-.

8. The compound according to claim 6 or 7, wherein the A ring is pyrazolyl, dihydropyrazolyl, thiazolyl, or pyrimidyl.

9. The compound according to claim 6, 7, or 8, wherein R₁ is H.

10. The compound according to claim 6, 7, 8, or 9 wherein L₃ is a bond, and L₂ is a bond.

11. The compound according to claim 9 or 10, wherein the A ring is pyrazolyl or thiazolyl.

12. The compound according to any one of claim 5 - 11, wherein Z is phenyl which is optionally substituted with 1, 2, or 3 groups that are independently C₁-C₆ alkyl, C₁-C₆ alkoxy, halogen, C₁-C₂ haloalkyl, C₁-C₂ haloalkoxy, or NO₂.

13. The compound according to claim 12, Z is phenyl which is substituted with 1 group that is C₁-C₆ alkyl, C₁-C₆ alkoxy, halogen, C₁-C₂ haloalkyl, C₁-C₂ haloalkoxy, or NO₂.

14. The compound according to claim 13, wherein Z is phenyl.

15. The compound according to any one of claims 5 - 12, wherein Q is phenyl, which is optionally substituted with 1, 2, or 3, groups that are independently C₁-C₆ alkoxycarbonyl, C₁-C₆ alkyl, C₁-C₆ alkoxy, halogen, CF₃, or OCF₃.

16. A compound according to claim 1 that is
N-{[4-({[4-(4-chlorophenyl)-5-(4-methylphenyl)-1,3-thiazol-2-yl]amino}carbonyl)phenyl]sulfonyl}phenylalanine;
N-({4-[3-(4-methoxyphenyl)-5-(4-pentylphenyl)-4,5-dihydro-1H-pyrazol-1-yl]phenyl}sulfonyl)-N-methylphenylalanine;
N-{[4-({[4-(4-chlorophenyl)-5-(4-methoxyphenyl)-1,3-thiazol-2-yl]amino}carbonyl)phenyl]sulfonyl}phenylalanine;
N-methyl-N-[(4-{5-(4-pentylphenyl)-3-[4-(trifluoromethoxy)phenyl]-4,5-dihydro-1H-pyrazol-1-yl}phenyl)sulfonyl]phenylalanine:
N-({4-[3-(4-methoxyphenyl)-5-(4-pentylphenyl)-1H-pyrazol-1-yl]phenyl}sulfonyl)-N-methylphenylalanine;
N-methyl-N-[(4-{5-(4-pentylphenyl)-3-[4-(trifluoromethoxy)phenyl]-1H-pyrazol-1-yl}phenyl)sulfonyl]phenylalanine;
N-({4-[5-(4-butoxyphenyl)-3-(4-methoxyphenyl)-1H-pyrazol-1-yl]phenyl}sulfonyl)-N-methylphenylalanine;
N-{[4-({[4-(3-chlorophenyl)-5-(4-methylphenyl)-1,3-thiazol-2-yl]amino}carbonyl)phenyl)sulfonyl}phenylalaninei;
N-({4-[5-(4-isopropylphenyl)-3-(4-methoxyphenyl)-1H-pyrazol-1-yl]phenyl}sulfonyl)-N-methylphenylalanine;
N-{[4-({[4-(3-chloro-4-methylphenyl)-5-(4-methylphenyl)-1,3-thiazol-2-yl]amino}carbonyl)phenyl]sulfonyl}phenylalanine;
N-([4-(([4-(4-chlorophenyl)-5-(4-methylphenyl)-1,3-thiazol-2-yl]amino)carbonyl)phenyl]sulfonyl)-N-methylphenylalanine;
N-{[4-({[4-(4-bromophenyl)-5-(4-methylphenyl)-1,3-thiazol-2-yl]amino}carbonyl)phenyl]sulfonyl}phenylalanine;
N-{[4-({[4-(4-chlorophenyl)-5-(4-ethylphenyl)-1,3-thiazol-2-yl]amino}carbonyl)phenyl]sulfonyl}phenylalanine;
N-[(4-{[4,6-bis(4-methoxyphenyl)pyrimidin-2-yl]amino}phenyl)sulfonyl]-N-methyl-L-phenylalanine;
N-({4-[2-[(4-chlorobenzoyl)amino]-5-(4-ethylphenyl)-1,3-thiazol-4-yl]phenyl}sulfonyl)-N-methylphenylalanine
N-[(4-{4-bromo-3-(4-methoxyphenyl)-5-[4-(trifluoromethyl)phenyl]-1H-pyrazol-1-yl}phenyl)sulfonyl]-N-methylphenylalanine;
N-({4-[5-(4-bromophenyl)-3-(4-methoxyphenyl)-1H-pyrazol-1-yl]phenyl}sulfonyl)-N-methylphenylalanine;
N-({4-[2-[(cyclopentylacetyl)amino]-5-(4-ethylphenyl)-1,3-thiazol-4-yl]phenyl}sulfonyl)-N-methyl-L-phenylalanine;
N-({4-[2-(4-chlorophenyl)-5-(4-ethylphenyl)-1,3-thiazol-4-yl]phenyl}sulfonyl)-N-methyl-L-phenylalanine;
N-({4-[5-(4-ethylphenyl)-2-(6-methoxypyridin-3-yl)-1,3-thiazol-4-yl]phenyl}sulfonyl)-N-methyl-L-phenylalanine;
N-[(4-{[4-(4-chlorophenyl)-5-(4-methylphenyl)-1,3-thiazol-2-yl]amino}phenyl)sulfonyl]-N-methyl-L-phenylalanine;
N-[(4-{3-(4-chlorophenyl)-5-[4-(trifluoromethyl)phenyl]-1H-pyrazol-1-yl}phenyl)sulfonyl]-N-methylphenylalanine;
N-({4-[3-(4-chlorophenyl)-5-(4-ethylphenyl)-1H-pyrazol-1-yl]phenyl}sulfonyl)-N-methylphenylalanine;
N-[(4-{4-bromo-3-(4-chlorophenyl)-5-[4-(trifluoromethyl)phenyl]-1H-pyrazol-1-yl}phenyl)sulfonyl]-N-methylphenylalanine;
N-({4-[4-bromo-3-(4-chlorophenyl)-5-(4-ethylphenyl)-1H-pyrazol-1-yl]phenyl}sulfonyl)-N-methylphenylalanine;
N-({4-[5-(4-bromophenyl)-3-(4-chlorophenyl)-1H-pyrazol-1-yl]phenyl}sulfonyl)-N-methylphenylalanine;
N-({4-[3-(4-chlorophenyl)-5-(4-pentylphenyl)-1H-pyrazol-1-yl]phenyl}sulfonyl)-N-methylphenylalanine;
N-({4-[4-bromo-3-(4-chlorophenyl)-5-(4-pentylphenyl)-1H-pyrazol-1-yl]phenyl}sulfonyl)-N-methylphenylalanine;
N- ({4-[5-(4-chlorophenyl)-2-(4-ethylphenyl)-1,3-thiazol-4-yl]phenyl}sulfonyl)-N-ethyl-L-phenylalanine;
N-({4-[5-(4-chlorophenyl)-2-(4-ethylphenyl)-1,3-thiazol-4-yl]phenyl}sulfonyl)phenylalanine;
N-({4-[2,5-bis(4-ethylphenyl)-1,3-thiazol-4-yl]phenyl}sulfonyl)phenylalanine;
2-({4-[2-(2-Cyclopentyl-acetylamino)-5-(4-ethyl-phenyl)-thiazol-4-yl]-benzenesulfonyl}-methyl-amino)-3-phenyl-propionic acid;
2-({4-[2-(4-Chloro-benzoylamino)-5-(4-ethyl-phenyl)-thiazol-4-yl]-benzenesulfonyl}-methyl-amino)-3-phenyl-propionic acid;
2-({4-[5-(4-Chloro-phenyl)-2-(4-ethyl-phenyl)-thiazol-4-yl]-benzenesulfonyl}-ethyl-amino)-3-phenyl-propionic acid;
2-{4-[5-(4-Chloro-phenyl)-2-(4-ethyl-phenyl)-thiazol-4-yl]-benzenesulfonylamino}-3-phenyl-propionic acid;
2-({4-[2-(4-Chloro-phenyl)-5-(6-methoxy-pyridin-3-yl)-thiazol-4-yl]-benzenesulfonyl}-ethyl-amino)-3-phenyl-propionic acid;
or pharmaceutically acceptable salts thereof.

17. Use of a compound of any preceding claim in the preparation of a medicament for the treatment of diabetes.

18. A pharmaceutical composition comprising a compound according to claim 1 and at least one pharmaceutically acceptable carrier, solvent, adjuvant or excipient.

## Patentansprüche

1. Verbindung der Formel 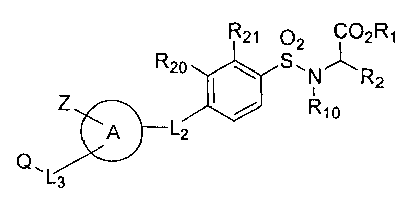 worin
L₂ eine Bindung oder -C(O)NR₉-, -N(R₉)C(O)-, -(C₁-C₄)Alkyl-C(O)NR₉-, -(C₁-C₄)Alkyl-N(R₉)C(O)-, -C(O)N(R₉)-(C₁-C₄)Alkyl-, -N(R₉)C(O)-(C₁-C₄)Alkyl-, -N(R₉)SO₂-, -SO₂N(R₉)-, -N(R9)-, -N(R₉)-(C₁-C₄)Alkyl-, -O-(C₁-C₄)Alkyl-, -(C₁-C₄)Alkyl-O-, oder -(C₁-C₄)Alkyl-N(R₉)- ist,
R₉ H, C₁-C₆ Alkyl, -SO₂Phenyl, Phenyl(C₁-C₄)alkyl ist, wobei die Phenylgruppe gegebenenfalls substituiert ist mit 1, 2, 3 oder 4 Gruppen, unabhängig voneinander ausgewählt aus C₁-C₄-Alkyl, C₁-C₄-Alkoxy, Halogen, OH, NO₂, NH₂, NH(C₁-C₆)Alkyl, N(C₁-C₆)Alkyl(C₁-C₆)alkyl, C₁-C₂-Halogenalkyl, oder C₁-C₂-Halogenalkoxy;
L₃ eine Bindung, - (C₁-C₄) Alkyl-O-, -O-(C₁-C₄)Alkyl, -(C₁-C₄)Alkyl-, -C (O) - ist;
der A-Ring Thiazolyl, Pyrazolyl, Dihydropyrazolyl, Benzofuranyl, Imidazolyl, Isothiazolyl, Pyrrolyl, Oxazolyl, Pyrimidyl oder Triazolyl ist, wobei jeder davon gegebenenfalls substituiert ist mit 1 oder 2 Gruppen, unabhängig voneinander ausgewählt aus Halogen, C₁-C₆-Alkyl, C₁-C₄-Alkoxy, Halogenalkyl, Halogenalkoxy, NO₂, NH₂, NH(C₁-C₆)Alkyl, N(C₁-C₆)Alkyl(C₁-C₆)alkyl;
Q Phenyl, Naphthyl, -Phenyl-carbonyl-phenyl, -Phenyl-(C₁-C₄)alkyl-Phenyl, -Phenyl-pyridyl, -Phenyl-pyrimidyl, -Phenyl-pyrrolyl, -Phenyl-piperidinyl, -Phenyl-pyrrolidinyl, -Phenyl-piperazinyl, -Phenyl-, Pyridyl, Pyrimidyl, Furanyl, Thienyl, Pyrrolyl, Imidazolyl, -Pyridyl-(C₁-C₄)alkyl-phenyl, Imidazolidinyl, Dibenzofuranyl, Tetrahydrofuranyl, Tetrahydrothienyl, Piperidinyl, Pyrrolidinyl, Piperazinyl, C₁-C₆ Alkyl, Halogen, C₁-C₄-Halogenalkoxy, C₁-C₄-Halogenalkyl, oder C₁-C₆-Alkoxycarbonyl ist, wobei die vorstehend genannten cyclischen Gruppen gegebenenfalls substituiert sind mit 1,2,3,4 oder 5 Gruppen, unabhängig voneinander ausgewählt aus Alkoxycarbonyl, C₁-C₆-Alkyl, C₁-C₆-Alkoxy, Halogen, C₁-C₄-Halogenalkyl, C₁-C₄-Halogenalkoxy, NR₆R₇ oder Phenyl;
wobei
R₆ und R₇ unabhängig voneinander H, C₁-C₆-Alkyl, Phenyl(C₁-C₆)alkyl, C₂-C₆-Alkanoyl, Phenyl(C₁-C₆)alkanoyl, C₁-C₆-Alkoxycarbonyl, Phenyl(C₁-C₆)alkoxycarbonyl, Pyridylcarbonyl oder -SO₂-Phenyl sind, wobei die cyclischen Gruppen gegebenenfalls substituiert sind mit 1, 2, 3 oder 4 Gruppen, unabhängig voneinander ausgewählt aus Halogen, C₁-C₄-Alkyl, C₁-C₄-Alkoxy, NO₂, OH, NH₂, NH(C₁-C₆)Alkyl, N(C₁-C₆)Alkyl(C₁-C₆)Alkyl, C₁-C₂-Halogenalkyl oder C₁-C₂-Halogenalkoxy, und
Z -NHC(O)Phenyl, -NHC(O)Naphthyl, -N(C₁-C₄-Alkyl)C(O)phenyl, -N(C₁-C₄-Alkyl)C(O)naphthyl, Naphthyl oder Phenyl ist, wobei die Phenylgruppen gegebenenfalls substituiert sind mit 1, 2, 3, 4 oder 5 Gruppen, unabhängig voneinander ausgewählt aus C₁-C₆-Alkyl, C₁-C₆-Alkoxy, Halogen, C₁-C₂-Halogenalkyl, C₁-C₂-Halogenalkoxy oder NO₂, oder
Z -NHC(O)-(C₁-C₄)Alkyl-(C₃-C₇)cycloalkyl oder -N(C₁-C₄)Alkyl-C(O)-(C₁-C₄)alkyl-(C₃-C₇)cycloalkyl ist;
R₁ H, C₁-C₄-Alkyl oder Benzyl ist;
R₂ Phenyl, Phenyl(C₁-C₄)Alkyl, -(C₁-C₄)Alkyl-piperidinyl, -(C₁-C₄)Alkyl-pyrrolidinyl ist, wobei die Heterocycloalkylgruppe gegebenenfalls annelliert ist an einen Phenylring und wobei der Heterocycloalkylteil, der Phenylteil oder beide gegebenenfalls substituiert sind mit insgesamt 1, 2, 3 oder 4 Gruppen, unabhängig voneinander ausgewählt aus Halogen, C₁-C₄-Alkyl, C₁-C₄-Alkoxy, -SO₂-(C₁-C₄)Alkyl, C₁-C₂-Halogenalkyl oder C₁-C₂-Halogenalkoxy;
R₁₀ H, C₁-C₆ Alkyl ist, wobei die Alkylgruppe gegebenenfalls mit Phenyl substituiert ist, welches gegebenenfalls substituiert ist mit 1, 2, 3 oder 4 Gruppen, unabhängig voneinander ausgewählt aus C₁-C₆-Alkyl, C₁-C₆-Alkoxy, Halogen, OH, NO₂, C₁-C₂-Halogenalkyl oder C₁-C₂-Halogenalkoxy; und
R₂₀ und R₂₁ unabhängig voneinander ausgewählt sind aus H, Benzyloxy, Benzyl, Halogen, C₁-C₄-Alkyl, OH, C₁-C₄-Alkoxy, NO₂, NH₂, NH(C₁-C₆)Alkyl, N(C₁-C₆)Alkyl(C₁-C₆)Alkyl, NH-Phenyl, N(C₁-C₄)Alkyl-phenyl, NHBenzyl oder -N(C₁-C₆)Alkylbenzyl, wobei die Phenylgruppen gegebenenfalls substituiert sind mit 1, 2, 3 oder 4 Gruppen, unabhängig voneinander ausgewählt aus C₁-C₆-Alkyl, C₁-C₆-Alkoxy, Halogen, OH, NO₂, C₁-C₂-Halogenalkyl oder C₁-C₂-Halogenalkoxy.

2. Verbindung nach Anspruch 1, wobei
L₂ eine Bindung oder -C(O)NR₉-, -N(R₉)C(O)-, -(C₁-C₄)Alkyl-C(O)NR₉-, -(C₁-C₄)Alkyl-N(R₉)C(O)-, -N(R₉)SO₂-, -SO₂N(R₉)-, -N(R₉)-, -N(R₉)-(C₁-C₄)Alkyl- oder -(C₁-C₄)alkyl-N(R₉)- ist,
R₉ ist H, C₁-C₆-Alkyl, -SO₂Phenyl, Benzyl, Phenethyl, Naphthyl-CH₂-, Anthracenyl-CH₂-, wobei die Phenylgruppe gegebenenfalls substituiert ist mit 1, 2, 3 oder 4 Gruppen, unabhängig voneinander ausgewählt aus C₁-C₄-Alkyl, C₁-C₄-Alkoxy, Halogen, OH, NO₂, NH₂, NH(C₁-C₆)Alkyl, N(C₁-C₆)Alkyl(C₁-C₆)Alkyl, C₁-C₂-Halogenalkyl oder C₁-C₂-Halogenalkoxy;
der A-Ring Thiazolyl, Pyrazolyl, Dihydropyrazolyl, Benzofuranyl, Imidazolyl, Isothiazolyl, Pyrrolyl, Pyrimidyl oder Oxazolyl ist, wobei jeder davon gegebenenfalls substituiert ist mit 1 oder 2 Gruppen, unabhängig voneinander ausgewählt aus Halogen, C₁-C₆-Alkyl, C₁-C₄-Alkoxy, Halogenalkyl, Halogenalkoxy, NO₂, NH₂, NH(C₁-C₆)Alkyl, N(C₁-C₆)Alkyl(C₁-C₆)alkyl; und
Q Phenyl, Naphthyl, -Phenyl-carbonyl-phenyl, -Phenyl-pyridyl, -Phenyl-piperidinyl, -Phenyl-pyrrolidinyl, Pyridyl, Pyrimidyl, Furanyl, Thienyl, Piperidinyl, Dibenzofuranyl, Pyrrolidinyl, Piperazinyl, C₁-C₆-Alkyl, Halogen, C₁-C₄-Halogenalkoxy, C₁-C₄-Halogenalkyl oder C₁-C₆-Alkoxycarbonyl ist, wobei die vorstehend genannten cyclischen Gruppen gegebenenfalls substituiert sind mit 1, 2, 3, 4 oder 5 Gruppen, unabhängig voneinander ausgewählt aus Alkoxycarbonyl, C₁-C₆-Alkyl, C₁-C₆-Alkoxy, Halogen, C₁-C₄-Halogenalkyl, C₁-C₄-Halogenalkoxy oder NR₆R₇;
wobei
R₆ und R₇ unabhängig voneinander H, C₁-C₆-Alkyl, Phenyl(C₁-C₄)alkyl, C₂-C₆-Alkanoyl, Phenyl(C₁-C₄)alkanoyl, C₁-C₆-Alkoxycarbonyl, Phenyl(C₁-C₄)alkoxycarbonyl, Pyridylcarbonyl oder -SO₂-Phenyl sind, wobei die cyclischen Gruppen gegebenenfalls substituiert sind mit 1, 2, 3 oder 4 Gruppen, unabhängig voneinander ausgewählt aus Halogen, C₁-C₄-Alkyl, C₁-C₄-Alkoxy, NO₂, OH, NH₂, NH(C₁-C₆)Alkyl, N(C₁-C₆)Alkyl(C₁-C₆)alkyl, CF₃ oder OCF₃.

3. Verbindung nach Anspruch 2, wobei
R₂ Phenyl oder Phenyl(C₁-C₄) Alkyl ist, wobei der Phenylteil oder beide gegebenenfalls substituiert sind mit insgesamt 1, 2, 3 oder 4 Gruppen, unabhängig voneinander ausgewählt aus Halogen, C₁-C₄-Alkyl, C₁-C₄-Alkoxy, -SO₂-(C₁-C₄)Alkyl, CF₃ oder OCF₃;
R₁₀ H, C₁-C₄-Alkyl ist, wobei die Alkylgruppe gegebenenfalls substituiert ist mit Phenyl, welches gegebenenfalls substituiert ist mit 1, 2, 3 oder 4 Gruppen, unabhängig voneinander ausgewählt aus C₁-C₆-Alkyl, C₁-C₆-Alkoxy, Halogen, OH, NO₂, C₁-C₂-Halogenalkyl oder C₁-C₂-Halogenalkoxy; und
R₂₀ und R₂₁ unabhängig voneinander ausgewählt sind aus H, Halogen, C₁-C₄-Alkyl, OH, C₁-C₄-Alkoxy, NO₂, NH₂, NH(C₁-C₆)Alkyl oder N(C₁-C₆)Alkyl(C₁-C₆)alkyl,
L₂ eine Bindung oder -C (O) NR₉-, -N(R₉)C(O)-, -(C₁-C₄)Alkyl-C(O)NR₉-, -(C₁-C₄)Alkyl-N(R₉)C(O)-, -N(R₉)SO₂-, -SO₂N(R₉)-, -N(R₉)-, -N(R₉)-(C₁-C₄)Alkyl- oder -(C₁-C₄)Alkyl-N(R₉)- ist,
R₉ H, C₁-C₆-Alkyl, -SO₂Phenyl, Benzyl, Phenethyl ist, wobei die Phenylgruppe gegebenenfalls substituiert ist mit 1, 2, 3 oder 4 Gruppen, unabhängig voneinander ausgewählt aus C₁-C₄-Alkyl, C₁-C₄-Alkoxy, Halogen, OH, NO₂, NH₂, NH(C₁-C₆)Alkyl, N(C₁-C₆)Alkyl(C₁-C₆)alkyl, CF₃ oder OCF₃;
Q Phenyl, Naphthyl, Pyridyl, Pyrimidyl, Furanyl, Thienyl, Piperidinyl, Pyrrolidinyl, Piperazinyl, C₁-C₆-Alkyl, Halogen, C₁-C₂-Halogenalkoxy, C₁-C₂-Halogenalkyl oder C₁-C₆-Alkoxycarbonyl ist, wobei die vorstehend angegebenen cyclischen Gruppen gegebenenfalls substituiert sind mit 1, 2, 3, 4 oder 5 Gruppen, unabhängig voneinander ausgewählt aus Alkoxycarbonyl, C₁-C₆-Alkyl, C₁-C₆-Alkoxy, Halogen, C₁-C₄-Halogenalkyl, C₁-C₄-Halogenalkoxy oder NR₆R₇; wobei
R₆ und R₇ unabhängig voneinander H, C₁-C₆-Alkyl, Phenyl(C₁-C₄)alkyl, C₂-C₆-Alkanoyl, Phenyl(C₁-C₄)alkanoyl sind, wobei die Phenylgruppen gegebenenfalls substituiert sind mit 1, 2, 3 oder 4 Gruppen, unabhängig voneinander ausgewählt aus Halogen, C₁-C₄-Alkyl, C₁-C₄-Alkoxy, NO₂, OH, NH₂, NH(C₁-C₆)Alkyl, N(C₁-C₆)Alkyl(C₁-C₆)alkyl, CF₃ oder OCF₃ und
Z -NHC(O)Phenyl, -N(C₁-C₄-Alkyl)C(O)phenyl oder Phenyl ist, wobei die Phenylgruppen gegebenenfalls substituiert sind mit 1, 2, 3, 4 oder 5 Gruppen, unabhängig voneinander ausgewählt aus C₁-C₆-Alkyl, C₁-C₆-Alkoxy, Halogen, C₁-C₂-Halogenalkyl, C₁-C₂-Halogenalkoxy oder NO₂ oder
Z -NHC(O)-(C₁-C₄)Alkyl-(C₃-C₇)cycloalkyl oder -N(C₁-C₄)alkyl-C(O)-(C₁-C₄)alkyl-(C₃-C₇)cycloalkyl ist.

4. Verbindung nach Anspruch 3, wobei
R₁ H oder C₁-C₄-Alkyl ist;
R₂ Phenyl, Phenyl(C₁-C₄)alkyl ist, wobei der Phenylteil oder beide gegebenenfalls substituiert sind insgesamt 1, 2, 3 oder 4 Gruppen, unabhängig voneinander ausgewählt aus Halogen, C₁-C₄-Alkyl, C₁-C₄-Alkoxy oder -SO₂-(C₁-C₄)Alkyl;
R₁₀ H, C₁-C₄-Alkyl ist, wobei die Alkylgruppe gegebenenfalls substituiert ist mit Phenyl, welches gegebenenfalls substituiert ist mit 1, 2, 3 oder 4 Gruppen, unabhängig voneinander ausgewählt aus C₁-C₆-Alkyl, C₁-C₆-Alkoxy, Halogen, OH, NO₂, CF₃ oder OCF₃; und
mindestens einer von R₂₀ und R₂₁ H ist, wobei der andere H, Halogen, C₁-C₄-Alkyl, OH, C₁-C₄-Alkoxy, NO₂, NH₂, NH(C₁-C₆)Alkyl oder N(C₁-C₆)Alkyl(C₁-C₆)alkyl ist;
L₂ eine Bindung oder -C(O)NR₉-, -N(R₉)C(O)-, -N(R₉)SO₂-, -SO₂N(R₉)-, -N(R₉)-, -N(R₉)-(C₁-C₄)Alkyl- oder -(C₁-C₄)Alkyl-N(R₉)- ist; und
Q Phenyl, Naphthyl, Pyridyl, Pyrimidyl, Furanyl, Thienyl, Piperidinyl, Pyrrolidinyl oder Piperazinyl ist, wobei jeder davon gegebenenfalls substituiert ist mit 1, 2, 3, 4 oder 5 Gruppen, unabhängig voneinander ausgewählt aus Alkoxycarbonyl, C₁-C₆-Alkyl, C₁-C₆-Alkoxy, Halogen, CF₃, OCF₃ oder NR₆R₇.

5. Verbindung nach Anspruch 4, wobei
L₂ eine Bindung oder -NR₉- ist; wobei R₉ H, C₁-C₆-Alkyl oder Benzyl ist;
R₂ Phenyl, Benzyl oder Phenethyl ist, wobei der Phenylteil gegebenenfalls substituiert ist mit insgesamt 1, 2, 3 oder 4 Gruppen, unabhängig voneinander ausgewählt aus Halogen, C₁-C₄-Alkyl, C₁-C₄-Alkoxy oder -SO₂-(C₁-C₄)Alkyl;
Q Phenyl oder Pyridyl ist, wobei jeder davon gegebenenfalls substituiert ist mit 1, 2, 3, 4 oder 5 Gruppen, unabhängig voneinander ausgewählt aus C₁-C₆-Alkoxycarbonyl, C₁-C₆-Alkyl, C₁-C₆-Alkoxy, Halogen, CF₃, OCF₃ oder NR₆R₇; und
Z Phenyl ist, gegebenenfalls substituiert mit 1, 2, 3, 4 oder 5 Gruppen, unabhängig voneinander ausgewählt aus C₁-C₆-Alkyl, C₁-C₆-Alkoxy, Halogen, C₁-C₂-Halogenalkyl, C₁-C₂-Halogenalkoxy oder NO₂.

6. Verbindung nach Anspruch 5, wobei der A-Ring Pyrazolyl, Dihydropyrazolyl, Thiazolyl oder Pyrimidyl ist, wobei jeder davon gegebenenfalls substituiert ist mit 1 oder 2 Gruppen, unabhängig voneinander ausgewählt aus Halogen, C₁-C₆-Alkyl, C₁-C₄-Alkoxy, Halogenalkyl, Halogenalkoxy, NO₂, NH₂, NH(C₁-C₆)Alkyl, N(C₁-C₆)Alkyl(C₁-C₆)alkyl,

7. Verbindung nach Anspruch 6, wobei R₁₀ H oder C₁-C₄-Alkyl ist; und L₃ eine Bindung oder -(C₁-C₄)Alkyl- ist.

8. Verbindung nach Anspruch 6 oder 7, wobei der A-Ring Pyrazolyl, Dihydropyrazolyl, Thiazolyl oder Pyrimidyl ist.

9. Verbindung nach Anspruch 6, 7 oder 8, wobei R₁ H ist.

10. Verbindung nach Anspruch 6, 7, 8 oder 9, wobei L₃ eine Bindung ist, und L₂ eine Bindung ist.

11. Verbindung nach Anspruch 9 oder 10, wobei der A-Ring Pyrazolyl oder Thiazolyl ist.

12. Verbindung nach einem der Ansprüche 5 bis 11, wobei Z Phenyl ist, gegebenenfalls substituiert mit 1, 2 oder 3 Gruppen, unabhängig voneinander ausgewählt aus C₁-C₆-Alkyl, C₁-C₆-Alkoxy, Halogen, C₁-C₂-Halogenalkyl, C₁-C₂-Halogenalkoxy oder NO₂.

13. Verbindung nach Anspruch 12, wobei Z Phenyl ist, gegebenenfalls substituiert mit einer Gruppe, ausgewählt aus C₁-C₆-Alkyl, C₁-C₆-Alkoxy, Halogen, C₁-C₂-Halogenalkyl, C₁-C₂-Halogenalkoxy oder NO₂.

14. Verbindung nach Anspruch 13, wobei Z Phenyl ist.

15. Verbindung nach einem der Ansprüche 5 bis 12, wobei Q Phenyl ist, gegebenenfalls substituiert mit 1, 2 oder 3 Gruppen, unabhängig voneinander ausgewählt aus C₁-C₆-Alkoxycarbonyl, C₁-C₆-Alkyl, C₁-C₆-Alkoxy, Halogen, CF₃ oder OCF₃.

16. Verbindung nach Anspruch 1, ausgewählt aus
N-{[4-({[4-(4-Chlorphenyl)-5-(4-methylphenyl)-1,3-thiazol-2-yl]amino}carbonyl)phenyl]sulfonyl}phenylalanin;
N-({4-[3-(4-Methoxyphenyl)-5-(4-pentylphenyl)-4,5-dihydro-1H-pyrazol-1-yl]phenyl}sulfonyl)-N-methylphenylalanin;
N-{[4-({[4-(4-Chlorphenyl)-5-(4-methoxyphenyl)-1,3-thiazol-2-yl]amino}carbonyl)phenyl]sulfonyl}phenylalanin;
N-Methyl-N-[(4-{5-(4-pentylphenyl)-3-[4-(trifluormethoxy)phenyl]-4,5-dihydro-1H-pyrazol-1-yl}phenyl)sulfonyl]phenylalanin;
N-({4-[3-(4-Methoxyphenyl)-5-(4-pentylphenyl)-1H-pyrazol-1-yl]phenyl}sulfonyl)-N-methylphenylalanin;
N-Methyl-N-[(4-{5-(4-pentylphenyl)-3-[4-(trifluormethoxy)phenyl]-1H-pyrazol-1-yl}phenyl)sulfonyl]phenylalanin;
N-({4-[5-(4-Butoxyphenyl)-3-(4-methoxyphenyl)-1H-pyrazol-1-yl]phenyl}sulfonyl)-N-methylphenylalanin;
N-{[4-({[4-(3-Chlorphenyl)-5-(4-methylphenyl)-1,3-thiazol-2-yl]amino}carbonyl)phenyl]sulfonyl}phenylalanin;
N-({4-[5-(4-Isopropylphenyl)-3-(4-methoxyphenyl)-1H-pyrazol-1-yl]phenyl}sulfonyl)-N-methylphenylalanin;
N-{[4-({[4-(3-Chlor-4-methylphenyl)-5-(4-methylphenyl)-1,3-thiazol-2-yl]amino}carbonyl)phenyl]sulfonyl}phenylalanin;
N-{[4-({[4-(4-Chlorphenyl)-5-(4-methylphenyl)-1,3-thiazol-2-yl]amino}carbonyl)phenyl]sulfonyl}-N-methylphenylalanin;
N-{[4-({[4-(4-Bromphenyl)-5-(4-methylphenyl)-1,3-thiazol-2-yl]amino}carbonyl)phenyl]sulfonyl}phenylalanin;
N-{[4-({[4-(4-Chlorphenyl)-5-(4-ethylphenyl)-1,3-thiazol-2-yl]amino}carbonyl)phenyl]sulfonyl}phenylalanin;
N-[(4-{[4,6-Bis(4-methoxyphenyl)pyrimidin-2-yl]amino}phenyl)sulfonyl]-N-methyl-L-phenylalanin;
N-({4-[2-[(4-Chlorbenzoyl)amino]-5-(4-ethylphenyl)-1,3-thiazol-4-yl]phenyl}sulfonyl)-N-methylphenylalanin
N-[(4-{4-Brom-3-(4-methoxyphenyl)-5-[4-(trifluormethyl)phenyl]-1H-pyrazol-1-yl}phenyl)sulfonyl]-N-methylphenylalanin;
N-({4-[5-(4-Bromphenyl)-3-(4-methoxyphenyl)-1H-pyrazol-1-yl]phenyl}sulfonyl)-N-methylphenylalanin;
N-({4-[2-[(Cyclopentylacetyl)amino]-5-(4-ethylphenyl)-1,3-thiazol-4-yl]phenyl}sulfonyl)-N-methyl-L-phenylalanin;
N-({4-[2-(4-Chlorphenyl)-5-(4-ethylphenyl)-1,3-thiazol-4-yl]phenyl}sulfonyl)-N-methyl-L-phenylalanin;
N-({4-[5-(4-Ethylphenyl)-2-(6-methoxypyridin-3-yl)-1,3-thiazol-4-yl]phenyl}sulfonyl)-N-methyl-L-phenylalanin;
N-[(4-{[4-(4-Chlorphenyl)-5-(4-methylphenyl)-1,3-thiazol-2-yl]amino}phenyl)sulfonyl]-N-methyl-L-phenylalanin;
N-[(4-{3-(4-Chlorphenyl)-5-[4-(trifluormethyl)phenyl]-1H-pyrazol-1-yl}phenyl)sulfonyl]-N-methylphenylalanin;
N-({4-[3-(4-Chlorphenyl)-5-(4-ethylphenyl)-1H-pyrazol-1-yl]phenyl}sulfonyl)-N-methylphenylalanin;
N-[(4-{4-Brom-3-(4-chlorphenyl)-5-[4-(trifluormethyl)phenyl]-1H-pyrazol-1-yl}phenyl)sulfonyl]-N-methylphenylalanin;
N-({4-[4-Brom-3-(4-chlorphenyl)-5-(4-ethylphenyl)-1H-pyrazol-1-yl]phenyl}sulfonyl)-N-methylphenylalanin;
N-({4-[5-(4-Bromphenyl)-3-(4-chlorphenyl)-1H-pyrazol-1-yl]phenyl}sulfonyl)-N-methylphenylalanin;
N-({4-[3-(4-Chlorphenyl)-5-(4-pentylphenyl)-1H-pyrazol-1-yl]phenyl}sulfonyl)-N-methylphenylalanin;
N-({4-[4-Brom-3-(4-chlorphenyl)-5-(4-pentylphenyl)-1H-pyrazol-1-yl]phenyl}sulfonyl)-N-methylphenylalanin;
N-({4-[5-(4-Chlorphenyl)-2-(4-ethylphenyl)-1,3-thiazol-4-yl]phenyl}sulfonyl)-N-ethyl-L-phenylalanin;
N-({4-[5-(4-Chlorphenyl)-2-(4-ethylphenyl)-1,3-thiazol-4-yl]phenyl}sulfonyl)phenylalanin;
N-({4-[2,5-Bis(4-ethylphenyl)-1,3-thiazol-4-yl]phenyl}sulfonyl)phenylalanin;
2-({4-[2-(2-Cyclopentyl-acetylamino)-5-(4-ethyl-phenyl)-thiazol-4-yl]-benzolsulfonyl}-methyl-amino)-3-phenyl-propionsäure;
2-({4-[2-(4-Chlor-benzoylamino)-5-(4-ethyl-phenyl)-thiazol-4-yl]-benzolsulfonyl}-methyl-amino)-3-phenyl-propionsäure;
2-({4-[5-(4-Chlor-phenyl)-2-(4-ethyl-phenyl)-thiazol-4-yl]-benzolsulfonyl}-ethyl-amino)-3-phenyl-propionsäure;
2-{4-[5-(4-Chlor-phenyl)-2-(4-ethyl-phenyl)-thiazol-4-yl]-benzolsulfonylamino}-3-phenyl-propionsäure;
2-({4-[2-(4-Chlor-phenyl)-5-(6-methoxy-pyridin-3-yl)-thiazol-4-yl]-benzolsulfonyl}-ethyl-amino)-3-phenyl-propionsäure;
oder pharmazeutisch verträglichen Salzen davon.

17. Verwendung einer Verbindung nach einem der vorstehenden Ansprüche zur Herstellung eines Medikaments zur Behandlung von Diabetes.

18. Pharmazeutische Zusammensetzung, umfassend eine Verbindung nach Anspruch 1 und mindestens einen pharmazeutisch verträglichen Trägerstoff, ein Lösungsmittel, ein Adjuvans oder ein Exzipiens.

## Revendications

1. Composé de formule 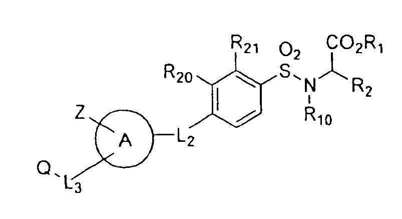 dans laquelle,
L₂ est une liaison ou un groupe -C(O)NR₉-, -N(R₉)C(O)-, -alkyle en C₁₋₄-C(O)NR₉-, -alkyle en C₁₋₄-N(R₉)C(O)-, -C(O)NR₉-alkyle en C₁₋₄-, -N(R₉)C(O)-alkyle en C₁₋₄-, -N(R₉)SO₂-, -SO₂N(R₉)-, -N(R₉)-, -N(R₉)-alkyle en C₁₋₄-, -O-alkyle en C₁₋₄-, -alkyle en C₁₋₄-O-, ou -alkyle en C₁₋₄-N(R₉)-,
R₉ est H, un groupe alkyle en C₁₋₆, -SO₂phényle, phénylalkyle en C₁₋₄, où le groupe phényle est éventuellement substitué par 1, 2, 3 ou 4 groupes qui sont indépendamment un groupe alkyle en C₁₋₄, alcoxy en C₁₋₄, halogène, OH, NO₂, NH₂, NH-alkyle en C₁₋₆, N(alkyle en C₁₋₆) alkyle en C₁₋₆, halogénoalkyle en C₁₋₂ ou halogénoalcoxy en C₁₋₂ ;
L₃ est une liaison, un groupe -alkyle en C₁₋₄-O-, -O-alkyle en C₁₋₄-, -alkyle en C₁₋₄-, -C(O)- ;
le cycle A est un groupe thiazolyle, pyrazolyle, dihydropyrazolyle, benzofuranyle, imidazolyle, isothiazolyle, pyrrolyle, oxazolyle, pyrimidyle ou triazolyle, chacun étant éventuellement substitué par 1 ou 2 groupes qui sont indépendamment un atome d'halogène, un groupe alkyle en C₁₋₆, alcoxy en C₁₋₄, halogénoalkyle, halogénoalcoxy, NO₂, NH₂, NH-alkyle en C₁₋₆, N (alkyle en C₁₋₆)alkyle en C₁₋₆ ;
Q est un groupe phényle, naphtyle, -phényl-carbonylphényle, -phényl-alkyle en C₁₋₄-phényle, phényle, -phényl-pyridyle, -phényl-pyrimidyle, -phényl-pyrrolyle, -phényl-pipéridinyle, -phényl-pyrrolidinyle, -phényl-pipérazinyle, -phényl-, pyridyle, pyrimidyle, furanyle, thiényle, pyrrolyle, imidazolyle, -pyridyl-alkyle en C₁₋₄-phényle, imidazolidinyle, dibenzofuranyle, tétrahydrofuranyle, tétrahydrothiényle, pipéridinyle, pyrrolidinyle, pipérazinyle, alkyle en C₁₋₆, halogène, halogénoalcoxy en C₁₋₄, halogénoalkyle en C₁₋₄ ou (alcoxy en C₁₋₆)carbonyle, où les groupes cycliques susmentionnés sont éventuellement substitués par 1, 2, 3, 4 ou 5 groupes qui sont indépendamment un groupe alcoxycarbonyle, alkyle en C₁₋₆, alcoxy en C₁₋₆, halogène, halogénoalkyle en C₁₋₄, halogénoalcoxy en C₁₋₄, NR₆R₇, ou phényle ;
où
R₆ et R₇ sont indépendamment H, un groupe alkyle en C₁₋₆, phénylalkyle en C₁₋₆, alcanoyle en C₂₋₆, phénylalcanoyle en C₁₋₆, (alcoxy en C₁₋₆) carbonyle, phényl(alcoxy en C₁₋₆)carbonyle, pyridylcarbonyle, ou -SO₂-phényle, où les groupes cycliques sont éventuellement substitués par 1, 2, 3 ou 4 groupes qui sont indépendamment un atome d'halogène, un groupe alkyle en C₁₋₄, alcoxy en C₁₋₄, NO₂, OH, NH₂, NH-alkyle en C₁₋₆, N(alkyle en C₁₋₆) alkyle en C₁₋₆, halogénoalkyle en C₁₋₂ ou halogénoalcoxy en C₁₋₂, et
Z est un groupe -NHC(O)phényle, -NHC(O)naphtyle, -N (alkyle en C₁₋₄)C(O)phényle, -N(alkyle en C₁₋₄)C(O)naphtyle, naphtyle ou phényle, où les groupes phényles sont éventuellement substitués par 1, 2, 3, 4 ou 5 groupes qui sont indépendamment un groupe alkyle en C₁₋₆, alcoxy en C₁₋₆, halogène, halogénoalkyle en C₁₋₂, halogénoalcoxy en C₁₋₂ ou NO₂,
ou
Z est un groupe -NHC(O)-alkyle en C₁₋₄-cycloalkyle en C₃₋₇ ou -N (alkyle en C₁₋₄)C(O)-alkyle en C₁₋₄-cycloalkyle en C₃₋₇;
R₁ est H, un groupe alkyle en C₁₋₄ ou benzyle ;
R₂ est un groupe phényle, phénylalkyle en C₁₋₄, -alkyle en C₁₋₄-pipéridinyle, -alkyle en C₁₋₄-pyrrolidinyle, où le groupe hétérocycloalkyle est éventuellement condensé en un cycle phényle et où la partie hétérocycloalkyle, la partie phényle, ou les deux est/sont substituée(s) par un total de 1, 2, 3 ou 4 groupes qui sont indépendamment un atome d'halogène, un groupe alkyle en C₁₋₄, alcoxy en C₁₋₄, -SO₂-alkyle en C₁₋₄, halogénoalkyle en C₁₋₂ ou halogénoalcoxy en C₁₋₂;
R₁₀ est H, un groupe alkyle en C₁₋₆, où le groupe alkyle est éventuellement substitué par un groupe phényle, qui est éventuellement substitué par 1, 2, 3 ou 4 groupes qui sont indépendamment un groupe alkyle en C₁₋₆, alcoxy en C₁₋₆, halogène, OH, NO₂, halogénoalkyle en C₁₋₂ ou halogénoalcoxy en C₁₋₂ ; et
R₂₀ et R₂₁ sont indépendamment choisis parmi H, un groupe benzyloxy, benzyle, halogène, alkyle en C₁₋₄, OH, alcoxy en C₁₋₄, NO₂, NH₂, NH-alkyle en C₁₋₆, N(alkyle en C₁₋₆)alkyle en C₁₋₆, NH-phényle, N(alkyle en C₁₋₆)phényle, NH-benzyle ou N(alkyle en C₁₋₆)benzyle, où les groupes phényles sont éventuellement substitués par 1, 2, 3 ou 4 groupes qui sont indépendamment un groupe alkyle en C₁₋₆, alcoxy en C₁₋₆, halogène, OH, NO₂, halogénoalkyle en C₁₋₂ ou halogénoalcoxy en C₁₋₂.

2. Composé selon la revendication 1, dans lequel
L₂ est une liaison ou un groupe -C(O)NR₉-, -N(R₉)C(O)-, -alkyle en C₁₋₄-C(O)NR₉-, -alkyle en C₁₋₄-N(R₉)C(O)-, -N(R₉)SO₂-, -SO₂N(R₉)-, -N(R₉)-, -N(R₉)-alkyle en C₁₋₄-, ou -alkyle en C₁₋₄-N(R₉)-,
R₉ est H, un groupe alkyle en C₁₋₆, -SO₂phényle, benzyle, phénéthyle, naphtyl-CH₂-, anthracényl-CH₂-, où le groupe phényle est éventuellement substitué par 1, 2, 3 ou 4 groupes qui sont indépendamment un groupe alkyle en C₁₋₄, alcoxy en C₁₋₄, halogène, OH, NO₂, NH₂, NH-alkyle en C₁₋₆, N(alkyle en C₁₋₆) alkyle en C₁₋₆, halogénoalkyle en C₁₋₂ ou halogénoalcoxy en C₁₋₂ ;
le cycle A est un groupe thiazolyle, pyrazolyle, dihydropyrazolyle, benzofuranyle, imidazolyle, isothiazolyle, pyrrolyle, pyrimidyle ou oxazolyle, chacun étant éventuellement substitué par 1 ou 2 groupes qui sont indépendamment un atome d' halogène, un groupe alkyle en C₁₋₆, alcoxy en C₁₋₄, halogénoalkyle, halogénoalcoxy, NO₂, NH₂, NH-alkyle en C₁₋₆, N(alkyle en C₁₋₆)alkyle en C₁₋₆; et
Q est un groupe phényle, naphtyle, -phényl-carbonylphényle, -phényl-pyridyle, -phényl-pipéridinyle, -phényl-pyrrolidinyle, pyridyle, pyrimidyle, furanyle, thiényle, pipéridinyle, dibenzofuranyle, pyrrolidinyle, pipérazinyle, alkyle en C₁₋₆, halogène, halogénoalcoxy en C₁₋₄, halogénoalkyle en C₁₋₄ ou (alcoxy en C₁₋₆)carbonyle, où les groupes cycliques susmentionnés sont éventuellement substitués par 1, 2, 3, 4 ou 5 groupes qui sont indépendamment un groupe alcoxycarbonyle, alkyle en C₁₋₆, alcoxy en C₁₋₆, halogène, halogénoalkyle en C₁₋₄, halogénoalcoxy en C₁₋₄, ou NR₆R₇ ;
où
R₆ et R₇ sont indépendamment H, un groupe alkyle en C₁₋₆, phénylalkyle en C₁₋₄, alcanoyle en C₂₋₆, phénylalcanoyle en C₁₋₄, (alcoxy en C₁₋₆)carbonyle, phényl(alcoxy en C₁₋₄)carbonyle, pyridylcarbonyle, ou -SO₂-phényle, où les groupes cycliques sont éventuellement substitués par 1, 2, 3 ou 4 groupes qui sont indépendamment un atome d'halogène, un groupe alkyle en C₁₋₄, alcoxy en C₁₋₄, NO₂, OH, NH₂, NH-alkyle en C₁_₆, N (alkyle en C₁₋₆)alkyle en C₁₋₆, CF₃, ou OCF₃.

3. Composé selon la revendication 2, dans lequel.
R₂ est un groupe phényle ou phénylalkyle en C₁₋₄, où la partie phényle ou les deux sont éventuellement substituées par un total de 1, 2, 3 ou 4 groupes qui sont indépendamment un atome d'halogène, un groupe alkyle en C₁₋₄, alcoxy en C₁₋₄, -SO₂-alkyle en C₁₋₄, CF₃, ou OCF₃ ;
R₁₀ est H, un groupe alkyle en C₁₋₄, où le groupe alkyle est éventuellement substitué par un groupe phényle, qui est éventuellement substitué par 1, 2, 3 ou 4 groupes qui sont indépendamment un groupe alkyle en C₁₋₆, alcoxy en C₁₋₆, halogène, OH, NO₂, halogénoalkyle en C₁₋₂ ou halogénoalcoxy en C₁₋₂ ; et
R₂₀ et R₂₁ sont indépendamment choisis parmi H, un atome d' halogène, un groupe alkyle en C₁₋₄, OH, alcoxy en C₁₋₄, NO₂, NH₂, NH-alkyle en C₁₋₆, ou N(alkyle en C₁₋₆) alkyle en C₁₋₆,
L₂ est une liaison ou un groupe -C(O)NR₉-, -N(R₉)C(O)-, -alkyle en C₁₋₄-C(O)NR₉-, -alkyle en C₁₋₄-N(R₉)C(O)-, -N(R₉)SO₂-, -SO₂N(R₉)-, -N(R₉)-, -N(R₉)-alkyle en C₁₋₄-, ou -alkyle en C₁₋₄-N(R₉)-,
R₉ est H, un groupe alkyle en C₁₋₆, -SO₂phényle, benzyle, phénéthyle, où le groupe phényle est éventuellement substitué par 1, 2, 3 ou 4 groupes qui sont indépendamment un groupe alkyle en C₁₋₄, alcoxy en C₁₋₄, halogène, OH, NO₂, NH₂, NH-alkyle en C₁₋₆, N(alkyle en C₁₋₆) alkyle en C₁₋₆, CF₃, ou OCF₃ ;
Q est un groupe phényle, naphtyle, pyridyle, pyrimidyle, furanyle, thiényle, pipéridinyle, pyrrolidinyle, pipérazinyle, alkyle en C₁₋₆, halogène, halogénoalcoxy en C₁₋₂, halogénoalkyle en C₁₋₂ ou (alcoxy en C₁₋₆)carbonyle, où les groupes cycliques susmentionnés sont éventuellement substitués par 1, 2, 3, 4 ou 5 groupes qui sont indépendamment un groupe alcoxycarbonyle, alkyle en C₁₋₆, alcoxy en C₁₋₆, halogène, halogénoalkyle en C₁₋₄, halogénoalcoxy en C₁₋₄, ou NR₆R₇ ;
où
R₆ et R₇ sont indépendamment H, un groupe alkyle en C₁₋₆, phénylalkyle en C₁₋₆, alcanoyle en C₂₋₆, phénylalcanoyle en C₁₋₄, où les groupes phényles sont éventuellement substitués par 1, 2, 3 ou 4 groupes qui sont indépendamment un atome d'halogène, un groupe alkyle en C₁₋₄, alcoxy en C₁₋₄, NO₂, OH, NH₂, NH-alkyle en C₁₋₆, N (alkyle en C₁₋₆) alkyle en C₁₋₆, CF₃, ou OCF₃, et
Z est un groupe -NHC(O)phényle, -N(alkyle en C₁₋₄)C(O)phényle, ou phényle, où les groupes phényles sont éventuellement substitués par 1, 2, 3, 4 ou 5 groupes qui sont indépendamment un groupe alkyle en C₁₋₆, alcoxy en C₁₋₆, halogène, halogénoalkyle en C₁₋₂, halogénoalcoxy en C₁₋₂ ou NO₂,
ou
Z est un groupe -NHC(O)-alkyle en C₁₋₄-cycloalkyle en C₃₋₇ ou -N(alkyle en C₁₋₄)C(O)-alkyle en C₁₋₄-cycloalkyle en C₃₋₇.

4. Composé selon la revendication 3, dans lequel
R₁ est H ou un groupe alkyle en C₁₋₄ ;
R₂ est un groupe phényle ou phénylalkyle en C₁₋₄, où la partie phényle ou les deux sont éventuellement substituées par un total de 1, 2, 3 ou 4 groupes qui sont indépendamment un atome d'halogène, un groupe alkyle en C₁₋₄, alcoxy en C₁₋₄, ou-SO₂-alkyle en C₁₋₄;
R₁₀ est H, un groupe alkyle en C₁₋₄, où le groupe alkyle est éventuellement substitué par un groupe phényle, qui est éventuellement substitué par 1, 2, 3 ou 4 groupes qui sont indépendamment un groupe alkyle en C₁₋₆, alcoxy en C₁₋₆, halogène, OH, NO₂, CF₃, ou OCF₃ ; et
au moins un de R₂₀ et R₂₁ est H, tandis que l'autre est H, un atome d'halogène, un groupe alkyle en C₁₋₄, OH, alcoxy en C₁₋₄, NO₂, NH₂, NH-alkyle en C₁₋₆, ou N(alkyleenC₁₋₆)alkyle en C₁₋₆,
L₂ est une liaison ou un groupe -C(O)NR₉-, -N(R₉)C(O)-, -N(R₉)SO₂-, -SO₂N(R₉)-, -N(R₉)-, -N(R₉)-alkyle en C₁₋₄-, ou -alkyle en C₁₋₄-N(R₉)- ; et
Q est un groupe phényle, naphtyle, pyridyle, pyrimidyle, furanyle, thiényle, pipéridinyle, pyrrolidinyle, ou pipérazinyle, chacun étant éventuellement substitué par 1, 2, 3, 4 ou 5 groupes qui sont indépendamment un groupe alcoxycarbonyle, alkyle en C₁₋₆, alcoxy en C₁₋₆, halogène, CF₃, OCF₃, ou NR₆R₇.

5. Composé selon la revendication 4, dans lequel
L₂ est une liaison ou un groupe -N(R₉)-,où
R₉ est H, un groupe alkyle en C₁₋₆ ou benzyle ;
R₂ est un groupe phényle, benzyle ou phénéthyle, où la partie phényle est éventuellement substituée par un total de 1, 2, 3 ou 4 groupes qui sont indépendamment un atome d' halogène, un groupe alkyle en C₁₋₄, alcoxy en C₁₋₄, ou -SO₂-alkyle en C₁₋₄ ;
Q est un groupe phényle ou pyridyle, chacun étant éventuellement substitué par 1, 2, 3, 4 ou 5 groupes qui sont indépendamment un groupe (alcoxy en C₁₋₆)carbonyle, alkyle en C₁₋₆, alcoxy en C₁₋₆, halogène, CF₃, OCF₃, ou NR₆R₇; et
Z est un groupe phényle, qui est éventuellement substitué par 1, 2, 3, 4 ou 5 groupes qui sont indépendamment un groupe alkyle en C₁₋₆, alcoxy en C₁₋₆, halogène, halogénoalkyle en C₁₋₂, halogénoalcoxy en C₁₋₂ ou NO₂.

6. Composé selon la revendication 5, dans lequel
le cycle A est un groupe pyrazolyle, dihydropyrazolyle, thiazolyle ou pyrimidyle, chacun étant éventuellement substitué par 1 ou 2 groupes qui sont indépendamment un atome d' halogène, un groupe alkyle en C₁₋₆, alcoxy en C₁₋₄, halogénoalkyle, halogénoalcoxy, NO₂, NH₂, NH-alkyle en C₁₋₆, ou N (alkyle en C₁₋₆) alkyle en C₁₋₆.

7. Composé selon la revendication 6, dans lequel R₁₀ est H ou un groupe alkyle en C₁₋₄ ; et L₃ est une liaison ou un groupe alkyle en C₁₋₄.

8. Composé selon la revendication 6 ou 7, dans lequel le cycle A est un groupe pyrazolyle, dihydropyrazolyle, thiazolyle ou pyrimidyle.

9. Composé selon la revendication 6, 7, ou 8, dans lequel R₁ est H.

10. Composé selon la revendication 6, 7, 8 ou 9, dans lequel L₃ est une liaison, et L₂ est une liaison.

11. Composé selon la revendication 9 ou 10, dans lequel le cycle A est un groupe pyrazolyle ou thiazolyle.

12. Composé selon l'une quelconque des revendications 5 à 11, dans lequel Z est un groupe phényle qui est éventuellement substitué par 1, 2 ou 3 groupes qui sont indépendamment un groupe alkyle en C₁₋₆, alcoxy en C₁₋₆, halogène, halogénoalkyle en C₁₋₂, halogénoalcoxy en C₁₋₂ ou NO₂.

13. Composé selon la revendication 12, dans lequel Z est un groupe phényle qui est éventuellement substitué par 1 groupe qui est un groupe alkyle en C₁₋₆, alcoxy en C₁₋₆, halogène, halogénoalkyle en C₁₋₂, halogénoalcoxy en C₁₋₂ ou NO₂.

14. Composé selon la revendication 13, dans lequel Z est un groupe phényle.

15. Composé selon l'une quelconque des revendications 5 à 12, dans lequel Q est un groupe phényle, qui est éventuellement substitué par 1, 2, ou 3 groupes qui sont indépendamment un groupe (alcoxy en C₁₋₆)carbonyle, alkyle en C₁₋₆, alcoxy en C₁₋₆, halogène, CF₃, ou OCF₃.

16. Composé selon la revendication 1 qui est
la N-{[4-({[4-(4-chlorophényl)-5-(4-méthylphényl)-1,3-thiazol-2-yl]amino}carbonyl)phényl]sulfonyl}phénylalanine ;
la N-({4-[3-(4-méthoxyphényl)-5-(4-pentylphényl)-4,5-dihydro-1H-pyrazol-1-yl]phényl}sulfonyl)-N-méthylphénylalanine ;
la N-{[4-({[4-(4-chlorophényl)-5-(4-méthoxyphényl)-1,3-thiazol-2-yl]amino}carbonyl)phényl]sulfonyl}phénylalanine ;
la N-méthyl-N-[(4-{5-(4-pentylphényl)-3-[4-(trifluorométhoxy)phényl-4,5-dihydro-1H-pyrazol-1-yl}phényl)sulfonyl]-phénylalanine ;
la N-({4-[3-(4-méthoxyphényl)-5-(4-pentylphényl)-1H-pyrazol-1-yl]phényl}sulfonyl)-N-méthylphénylalanine ;
la N-méthyl-N-[(4-{5-(4-pentylphényl)-3-[4-(trifluorométhoxy)phényl]-1H-pyrazol-1-yl}phényl)sulfonyl]phénylalanine ;
la N-({4-[5-(4-butoxyphényl)-3-(4-méthoxyphényl)-1H-pyrazol-1-yl]phényl}sulfonyl)-N-méthylphénylalanine ;
la N-{[4-({[4-(3-chlorophényl)-5-(4-méthylphényl)-1,3-thiazol-2-yl]amino}carbonyl)phényl]sulfonyl}phénylalanine ;
la N-({4-[5-(4-isopropylphényl)-3-(4-méthoxyphényl)-1H-pyrazol-1-yl]phényl}sulfonyl)-N-méthylphénylalanine ;
la N-{[4-({[4-(3-chloro-4-méthylphényl)-5-(4-méthylphényl)-1,3-thiazol-2-yl]amino}carbonyl)phényl]sulfonyl}phénylalanine ;
la N-{[4-({[4-(4-chlorophényl)-5-(4-méthylphényl)-1,3-thiazol-2-yl]amino}carbonyl)phényl]sulfonyl}-N-méthylphénylalanine ;
la N-{[4-({[4-(4-bromophényl)-5-(4-méthylphényl)-1,3-thiazol-2-yl]amino}carbonyl)phényl]sulfonyl}phénylalanine ;
la N-{[4-({[4-(4-chlorophényl)-5-(4-éthylphényl)-1,3-thiazol-2-yl]amino}carbonyl)phényl]sulfonyl}phénylalanine;
la N-[(4-{[9,6-bis(4-méthoxyphényl)pyrimidin-2-yl]amino}phényl)sulfonyl]-N-méthyl-L-phénylalanine;
la N-({4-[2-[(4-chlorobenzoyl)amino]-5-(4-éthylphényl)-1,3-thiazol-4-yl]phényl}sulfonyl)-N-méthylphénylalanine;
la N-[(4-{4-bromo-3-(4-méthoxyphényl)-5-[4-(trifluorométhyl)phényl]-1H-pyrazol-1-yl)phényl)sulfonyl]-N-méthylphénylalanine ;
la N-({4-[5-(4-bromophényl)-3-(4-méthoxyphényl)-1H-pyrazol-1-yl]phényl}sulfonyl)-N-méthylphénylalanine ;
la N-({4-[2-[(cyclopentylacétyl)amino]-5-(4-éthylphényl)-1,3-thiazol-4-yl]phényl}sulfonyl)-N-méthyl-L-phénylalanine ;
la N-({4-[2-(4-chlorophényl)-5-(4-éthylphényl)-1,3-thiazol-4-yl]phényl}sulfonyl)-N-méthyl-L-phénylalanine ;
la N-({4-[5-(4-éthylphényl)-2-(6-méthoxypyridin-3-yl)-1,3-thiazol-4-yl]phényl}sulfonyl)-N-méthyl-L-phénylalanine ;
la N-[(4-{[4-(4-chlorophényl)-5-(4-méthylphényl)-1,3-thiazol-2-yl]amino}phényl)sulfonyl]-N-méthyl-L-phénylalanine ;
la N-[(4-{3-(4-chlorophényl)-5-[4-(trifluorométhyl)-phényl]-1H-pyrazol-1-yl}phényl)sulfonyl]-N-méthylphénylalanine ;
la N-({4-[3-(4-chlorophényl)-5-(4-éthylphényl)-1H-pyrazol-1-yl]phényl}sulfonyl)-N-méthylphénylalanine ;
la N-[(4-{4-bromo-3-(4-chlorophényl)-5-[4-(trifluorométhyl)phényl]-1H-pyrazol-1-yl}phényl)sulfonyl]-N-méthylphénylalanine ;
la N-({4-[4-bromo-3-(4-chlorophényl)-5-(4-éthylphényl)-1H-pyrazol-1-yl]phényl}sulfonyl)-N-méthylphénylalanine ;
la N-({4-[5-(4-bromophényl)-3-(4-chlorophényl)-1H-pyrazol-1-yl]phényl}sulfonyl)-N-méthylphénylalanine ;
la N-({4-[3-(1-chlorophényl)-5-(4-pentylphényl)-1H-pyrazol-1-yl]phényl}sulfonyl)-N-méthylphénylalanine ;
la N-({4-[4-bromo-3-(4-chlorophényl)-5-(4-pentylphényl)-1H-pyrazol-1-yl]phényl}sulfonyl)-N-méthylphénylalanine ;
la N-({4-[5-(4-chlorophényl)-2-(4-éthylphényl)-1,3-thiazol-4-yl]phényl}sulfonyl)-N-éthyl-L-phénylalanine ;
la N-({4-[5-(4-chlorophényl)-2-(4-éthylphényl)-1,3-thiazol-4-yl]phényl}sulfonyl)phénylalanine ;
la N-({4-[2,5-bis(4-éthylphényl)-1,3-thiazol-4-yl]-phényl}sulfonyl)phénylalanine ;
l'acide 2-({4-[2-(2-cyclopentyl-acétylamino)-5-(4-éthylphényl)-thiazol-4-yl]-benzènesulfonyl}-méthyl-amino)-3-phénylpropionique ;
l'acide 2-({4-[2-(4-chloro-benzoylamino)-5-(4-éthylphényl)-thiazol-4-yl]-benzènesulfonyl}-méthyl-amino)-3-phénylpropionique ;
l'acide 2-({4-[5-(4-chloro-phényl)-2-(4-éthyl-phényl)-thiazol-4-yl]-benzènesulfonyl}-éthyl-amino)-3-phénylpropionique;
l'acide 2-{4-[5-(4-chloro-phényl)-2-(4-éthyl-phényl)-thiazol-4-yl]-benzènesulfonylamino)-3-phényl-propionique ;
l'acide 2-({4-[2-(4-chloro-phényl)-5-(6-méthoxy-pyridin-3-yl)-thiazol-4-yl]-benzènesulfonyl}-éthyl-amino)-3-phénylpropionique ;
ou des sels pharmaceutiquement acceptables de ceux-ci.

17. Utilisation d'un composé selon l'une quelconque des revendications précédentes pour la préparation d'un médicament pour le traitement du diabète.

18. Composition pharmaceutique comprenant un composé selon la revendication 1 et au moins un support, un solvant, un adjuvant ou un excipient pharmaceutiquement acceptable.
